# EUROPEAN PATENT APPLICATION

(11) **EP 4 640 715 A1**
(43) Date of publication of application: **29.10.2025**
(21) Application number: 23906111.2
(22) Date of filing: 22.12.2023
(51) Int. Cl.: C07K 16/30, C07K 16/28, A61P 35/00

(54) **MULTI-SPECIFIC POLYPEPTIDE COMPLEX TARGETING GPRC5D**

(30) Priority: 23.12.2022 CN 202211667549
(71) Applicant: Chimagen Biosciences, Ltd, Shanghai 201203 (CN)
(72) Inventor: ZHOU, Zhenhao, Shanghai 201203 (CN); ZHANG, Jie, Shanghai 201203 (CN); WANG, Gong, Shanghai 201203 (CN); WANG, Xiaoqing, Shanghai 201203 (CN); ZHANG, Xiaoqin, Shanghai 201203 (CN); WANG, Qi, Shanghai 201203 (CN); SHI, Sainan, Shanghai 201203 (CN)
(74) Representative: Simmons & Simmons LLP (Munich)
(86) International application number: PCT/CN2023/141117
(87) International publication number: WO 2024/131956

(57) **Abstract**

A multi-specific polypeptide complex, an isolated polynucleotide encoding same, a pharmaceutical composition comprising same, and a use thereof.

## Description

### FIELD OF THE INVENTION

The present application generally relates to a multi-specific polypeptide complex targeting GPRC5D and use thereof.

### BACKGROUND

In the field of antibody therapy, bispecific or multi-specific antibodies widely studied at present can recognize two or more different antigens at the same time, neutralize different pathogenic agents, recruit different types of effector cells, and regulate signal pathways, making them superior to monospecific antibodies in many aspects. Therefore, it is of great clinical significance to develop bispecific or multi-specific antibodies for use as therapeutic agents for human diseases. In recent years, the bispecific antibodies have become a widely used form in the diagnosis and treatment.

In the development of bispecific or multi-specific antibodies, G-protein-coupled receptor family C group 5 member D (GPRC5D), a 7-transmembrane protein and an orphan receptor, has become one of the hot targets. It is reported that the overexpression of GPRC5D is observed in patients with multiple myeloma. In particular, its high expression has a significant correlation with the poor results of disease and treatment. In view of the high specific expression of GPRC5D protein on tumor cells, GPRC5D has a high potential to become a next hot candidate target for the treatment of multiple myeloma. When specifically targeting or killing cells that over-express GPRC5D is desired, multi-specific antibodies targeting at least GPRC5D (for example, bispecific antibodies or multi-specific antibodies targeting tumor antigen GPRC5D and immunostimulating antigens (such as CD3, etc.) or other tumor antigens (such as Her2, etc.) are of great significance in research.

Therefore, there is an urgent need in this field to develop a new multi-specific antibody targeting at least GPRC5D.

### SUMMARY OF THE INVENTION

Throughout the present application, the articles "a/an" and "the" are used herein to refer to one or more (i.e., at least one) grammatical object following the article. For example, "an antibody" means one antibody or more than one antibody.

The present invention provides a multi-specific polypeptide complex, an isolated polynucleotide encoding the polypeptide complex, a pharmaceutical composition containing the polypeptide complex, and use thereof.

In one aspect, the present application provides a multi-specific polypeptide complex, which comprises a first antigen binding domain and a second antigen binding domain. At least one of the first antigen binding domain and the second antigen binding domain binds to GPRC5D and comprises a GPRC5D binding domain. The GPRC5D binding domain comprises three heavy chain complementarity determining regions HCDR1, HCDR2, and HCDR3, and three light chain complementarity determining regions LCDR1, LCDR2, and LCDR3. The heavy chain complementarity determining regions are identical to three heavy chain complementarity determining regions comprised in a heavy chain variable region (V_{H}) as shown in SEQ ID NO: 13, and the light chain complementarity determining regions are identical to three light chain complementarity determining regions comprised in a light chain variable region (V_{L}) as shown in SEQ ID NO: 12.

In some embodiments, the GPRC5D binding domain comprises three heavy chain complementarity determining regions HCDR1, HCDR2 and HCDR3, and three light chain complementarity determining regions LCDR1, LCDR2 and LCDR3. a) The HCDR1 comprises an amino acid sequence selected from the group consisting of SEQ ID NO: 4 or a variant thereof having no more than 3, 2 or 1 amino acid substitution, or SEQ ID NO: 9 or a variant thereof having no more than 3, 2 or 1 amino acid substitution. b) The HCDR2 comprises an amino acid sequence selected from the group consisting of SEQ ID NO: 5 or a variant thereof having no more than 3, 2 or 1 amino acid substitution, or SEQ ID NO: 10 or a variant thereof having no more than 3, 2 or 1 amino acid substitution. c) The HCDR3 comprises an amino acid sequence selected from the group consisting of SEQ ID NO: 6 or a variant thereof having no more than 3, 2 or 1 amino acid substitution, or SEQ ID NO: 11 or a variant thereof having no more than 3, 2 or 1 amino acid substitution. d) The LCDR1 comprises an amino acid sequence selected from the group consisting of SEQ ID NO: 1 or a variant thereof having no more than 3, 2 or 1 amino acid substitution, or SEQ ID NO: 7 or a variant thereof having no more than 3, 2 or 1 amino acid substitution. e) The LCDR2 comprises an amino acid sequence selected from the group consisting of SEQ ID NO: 2 or a variant thereof having no more than 3, 2 or 1 amino acid substitution, or SEQ ID NO: 8 or a variant thereof having no more than 3, 2 or 1 amino acid substitution. f) The LCDR3 comprises an amino acid sequence selected from the group consisting of SEQ ID NO: 3 or a variant thereof having no more than 3, 2 or 1 amino acid substitution.

In some embodiments, in the GPRC5D binding domain, a) the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution; or the HCDR1 comprises the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

In some embodiments, in the GPRC5D binding domain, the heavy chain variable region comprises the amino acid sequence as shown in SEQ ID NO: 13 or a variant thereof having no more than 3, 2 or 1 amino acid substitution, and the light chain variable region comprises the amino acid sequence as shown in SEQ ID NO: 12 or a variant thereof having no more than 3, 2 or 1 amino acid substitution.

In some embodiments, the GPRC5D binding domain is humanized.

In some embodiments, the GPRC5D binding domain comprises a heavy chain variable region (V_{H}) and/or a light chain variable region (V_{L}), where a) the heavy chain variable region comprises an amino acid sequence selected from the group consisting of SEQ ID NO: 15, SEQ ID NO: 17, SEQ ID NO: 19, and SEQ ID NO: 21, or a variant thereof having no more than 3, 2 or 1 amino acid substitution; and b) the light chain variable region comprises an amino acid sequence selected from the group consisting of SEQ ID NO: 14, SEQ ID NO: 16, SEQ ID NO: 18, and SEQ ID NO: 20, or a variant thereof having no more than 3, 2 or 1 amino acid substitution.

In some embodiments, the GPRC5D binding domain comprises a heavy chain variable region (V_{H}) and/or a light chain variable region (V_{L}), in which a) the heavy chain variable region comprises the amino acid sequence as shown in SEQ ID NO: 15 or a variant thereof having no more than 3, 2 or 1 amino acid substitution, and the light chain variable region comprises the amino acid sequence as shown in SEQ ID NO: 14 or a variant thereof having no more than 3, 2 or 1 amino acid substitution; b) the heavy chain variable region comprises the amino acid sequence as shown in SEQ ID NO: 17 or a variant thereof having no more than 3, 2 or 1 amino acid substitution, and the light chain variable region comprises the amino acid sequence as shown in SEQ ID NO: 16 or a variant thereof having no more than 3, 2 or 1 amino acid substitution; c) the heavy chain variable region comprises the amino acid sequence as shown in SEQ ID NO: 19 or a variant thereof having no more than 3, 2 or 1 amino acid substitution, and the light chain variable region comprises the amino acid sequence as shown in SEQ ID NO: 18 or a variant thereof having no more than 3, 2 or 1 amino acid substitution; or the heavy chain variable region comprises the amino acid sequence as shown in SEQ ID NO: 21 or a variant thereof having no more than 3, 2 or 1 amino acid substitution, and the light chain variable region comprises the amino acid sequence as shown in SEQ ID NO: 20 or a variant thereof having no more than 3, 2 or 1 amino acid substitution, where the amino acid substitution does not occur in the CDR region.

In some embodiments, the polypeptide complex further comprises an immunoglobulin constant region, optionally a human immunoglobulin constant region, or optionally a human IgG constant region.

In some embodiments, one of the first antigen binding domain and the second antigen binding domain binds to GPRC5D and comprises the GPRC5D binding domain as described in the present application, and the other one binds to an antigen other than GPRC5D. In some embodiments, the antigen other than GPRC5D is an immunostimulatory antigen, where optionally the immunostimulatory antigen is CD3. In some embodiments, one of the first antigen binding domain and the second antigen binding domain comprises the GPRC5D binding domain as described in the present application, and the other one comprises a CD3 binding domain.

In some embodiments, the CD3 binding domain comprises three heavy chain complementarity determining regions HCDR1, HCDR2 and HCDR3, and three light chain complementarity determining regions LCDR1, LCDR2 and LCDR3. The HCDR1 comprises the amino acid sequence as shown in SEQ ID NO: 49 or a variant thereof having no more than 3, 2 or 1 amino acid substitution, the HCDR2 comprises the amino acid sequence as shown in SEQ ID NO: 50 or a variant thereof having no more than 3, 2 or 1 amino acid substitution, the HCDR3 comprises the amino acid sequence as shown in SEQ ID NO: 51 or a variant thereof having no more than 3, 2 or 1 amino acid substitution, the LCDR1 comprises the amino acid sequence as shown in SEQ ID NO: 52 or a variant thereof having no more than 3, 2 or 1 amino acid substitution, the LCDR2 comprises the amino acid sequence as shown in SEQ ID NO: 53 or a variant thereof having no more than 3, 2 or 1 amino acid substitution, and the LCDR3 comprises the amino acid sequence as shown in SEQ ID NO: 54 or a variant thereof having no more than 3, 2 or 1 amino acid substitution. In some embodiments, in the CD3 binding domain, the HCDR1 comprises the amino acid sequence as shown in SEQ ID NO: 92 or a variant thereof having no more than 3, 2 or 1 amino acid substitution, the HCDR2 comprises the amino acid sequence as shown in SEQ ID NO: 93 or a variant thereof having no more than 3, 2 or 1 amino acid substitution, the HCDR3 comprises the amino acid sequence as shown in SEQ ID NO: 94 or a variant thereof having no more than 3, 2 or 1 amino acid substitution, the LCDR1 comprises the amino acid sequence as shown in SEQ ID NO: 95 or a variant thereof having no more than 3, 2 or 1 amino acid substitution, the LCDR2 comprises the amino acid sequence as shown in SEQ ID NO: 96 or a variant thereof having no more than 3, 2 or 1 amino acid substitution, and the LCDR3 comprises the amino acid sequence as shown in SEQ ID NO: 54 or a variant thereof having no more than 3, 2 or 1 amino acid substitution.

In some embodiments, the CD3 binding domain comprises a heavy chain variable region and a light chain variable region. The heavy chain variable region comprises the amino acid sequence as shown in SEQ ID NO: 55 or a variant thereof having no more than 3, 2 or 1 amino acid substitution, and the light chain variable region comprises the amino acid sequence as shown in SEQ ID NO: 56 or a variant thereof having no more than 3, 2 or 1 amino acid substitution.

In some embodiments, the first antigen binding domain and the second antigen binding domain form a DICAD domain. The DICAD domain comprises: (i) a first polypeptide, comprising, in a N-terminal-to-C-terminal direction, a first light chain variable domain VL1 binding to a first antigen and a second heavy chain variable domain VH2 binding to a second antigen, where VL1 and VH2 are linked directly or by a first linker; and (ii) a second polypeptide, comprising, in a N-terminal-to-C-terminal direction, a second light chain variable domain VL2 binding to the second antigen and a first heavy chain variable domain VH1 binding to the first antigen, where VL2 and VH1 are linked directly or by a second linker. VL1 and VH1 associate to form the first antigen binding domain, VL2 and VH2 associate to form the second antigen binding domain, and VL1 and VH1 are covalently linked by a disulfide bond.

In some embodiments, the first linker and/or the second linker each independently comprise(s) 5 to 9 amino acid residues.

In some embodiments, VL1 has a first cysteine substitution in the FR, VH1 has a second cysteine substitution in the FR, and the first cysteine and the second cysteine form a disulfide bond.

In some embodiments, the first cysteine and the second cysteine are selected from the group consisting of 100C in VL1 and 44C in VH1; 43C in VL1 and 105C in VH1; 49C in VL1 and 100bC in VH1; 50C in VL1 and 100C in VH1; and 46C in VL1 and 101C in VH1, wherein the numbering is based on Kabat numbering. In some embodiments, the disulfide bond is formed between 100C in VL1 and 44C in VH1.

In some embodiments, VL1 and VH1 further have an electrostatic interaction between two residues with opposite charges. In some embodiments, the two residues with opposite charges are introduced into VL1 and VH1 to replace residues at positions selected from the group consisting of a) Q38 in VL1 and Q39 in VH1; b) Q40 in VL1 and Q39 in VH1; or c) Q37 in VL1 and Q39 in VH1, where the numbering is based on Kabat numbering.

In some embodiments, VL2 and VH2 further have an electrostatic interaction between two residues with opposite charges. In some embodiments, the two residues with opposite charges are introduced into VL2 and VH2 to replace residues at positions selected from the group consisting of a) Q38 in VL2 and Q39 in VH2; b) Q40 in VL2 and Q39 in VH2; or c) Q37 in VL2 and Q39 in VH2, wherein the numbering is based on Kabat numbering. In some embodiments, the two residues with opposite charges comprise a negatively charged amino acid residue selected from the group consisting of aspartate (D) or glutamate (E), and a positively charged amino acid residue selected from the group consisting of lysine (K) or arginine (R).

In some embodiments, at least one of the residues in the FR of VL1 is replaced by a negatively charged amino acid, and at least one of the residues in the FR of VH1 is replaced by a positively charged amino acid, or at least one of the residues in the FR of VL 1 is replaced by a positively charged amino acid, and at least one of the residues in the FR of VH1 is replaced by a negatively charged amino acid.

In some embodiments, the first antigen binding domain comprises an antigen binding fragment of an antibody binding to GPRC5D as described in the present application, and the second antigen binding domain comprises the CD3 binding domain as defined in the present application. In some embodiments, the amino acid sequence of the first polypeptide comprises an amino acid sequence as shown in SEQ ID NO: 22, and the amino acid sequence of the second polypeptide comprises an amino acid sequence as shown in SEQ ID NO: 23. In some embodiments, the second polypeptide further comprises, in a N-terminal-to-C-terminal direction, a first Fc polypeptide. In some embodiments, the polypeptide complex further comprises a third polypeptide, which comprises, in a N-terminal-to-C-terminal direction, a second Fc polypeptide.

In some embodiments, the polypeptide complex further comprises a third antigen binding domain, and optionally, the third antigen binding domain comprises a Fab domain. In some embodiments, the Fab domain comprises (i) a third polypeptide, comprising, in a N-terminal-to-C-terminal direction, a third heavy chain variable domain VH3 binding to a third antigen and a CH1 domain; and (ii) a fourth polypeptide, comprising, in a N-terminal-to-C-terminal direction, a third light chain variable domain VL3 binding to the third antigen and a CL domain, where VL3 and VH3 associate to form the third antigen binding domain.

In some embodiments, the Fab domain binds to GPRC5D, and comprises the GPRC5D binding domain as described in the present application. In some embodiments, the first polypeptide comprises an amino acid sequence as shown in SEQ ID NO: 29, the second polypeptide comprises an amino acid sequence as shown in SEQ ID NO: 30, the third polypeptide comprises an amino acid sequence as shown in SEQ ID NO: 31, and the fourth polypeptide comprises an amino acid sequence as shown in SEQ ID NO: 32.

In some embodiments, the polypeptide complex further comprises a third antigen binding domain, and optionally the third antigen binding domain comprises a Fab domain. The Fab domain comprises (i) a third polypeptide, comprising, in a N-terminal-to-C-terminal direction, a third heavy chain variable domain VH3 binding to a third antigen and a CH1 domain; and (ii) a fourth polypeptide, comprising, in a N-terminal-to-C-terminal direction, a third light chain variable domain VL3 binding to the third antigen and a CL domain, where VL3 and VH3 associate to form the third antigen binding domain.

In some embodiments, the first antigen, the second antigen and the third antigen are each independently selected from GPRC5D, an immunostimulatory antigen and a tumor antigen; and optionally, the immunostimulatory antigen is CD3, and the tumor antigen is Her2. In some embodiments, the first antigen is Her2, the second antigen is CD3, and the third antigen is GPRC5D.

In some embodiments, the Her2 binding domain comprises three heavy chain complementarity determining regions HCDR1, HCDR2 and HCDR3 in the heavy chain variable region (V_{H}), and three light chain complementarity determining regions LCDR1, LCDR2 and LCDR3 in the light chain variable region (V_{L}). The HCDR1 comprises the amino acid sequence as shown in SEQ ID NO: 57 or a variant thereof having no more than 3, 2 or 1 amino acid substitution, the HCDR2 comprises the amino acid sequence as shown in SEQ ID NO: 58 or a variant thereof having no more than 3, 2 or 1 amino acid substitution, the HCDR3 comprises the amino acid sequence as shown in SEQ ID NO: 59 or a variant thereof having no more than 3, 2 or 1 amino acid substitution, the LCDR1 comprises the amino acid sequence as shown in SEQ ID NO: 60 or a variant thereof having no more than 3, 2 or 1 amino acid substitution, the LCDR2 comprises the amino acid sequence as shown in SEQ ID NO: 61 or a variant thereof having no more than 3, 2 or 1 amino acid substitution, and the LCDR3 comprises the amino acid sequence as shown in SEQ ID NO: 62 or a variant thereof having no more than 3, 2 or 1 amino acid substitution.

In some embodiments, the Her2 binding domain comprises a light chain variable domain having the amino acid sequence as shown in SEQ ID NO: 64, and the VH3 comprises a heavy chain variable domain having the amino acid sequence as shown in SEQ ID NO: 63.

In some embodiments, the first antigen binding domain comprises the Her2 binding domain as defined in the present application, the second antigen binding domain comprises the CD3 binding domain as defined in the present application, and the third antigen binding domain the antigen binding fragment of an antibody binding to GPRC5D as defined in the present application. In some embodiments, the first polypeptide comprises the amino acid sequence as shown in SEQ ID NO: 25, the second polypeptide comprises the amino acid sequence as shown in SEQ ID NO: 26, the third polypeptide comprises the amino acid sequence as shown in SEQ ID NO: 27, and the fourth polypeptide comprises the amino acid sequence as shown in SEQ ID NO: 28.

In some embodiments, the first antigen is GPRC5D, the second antigen is GPRC5D, and the third antigen is CD3; and the first antigen binding domain and the second antigen binding domain comprise the antigen binding fragment of an antibody binding to GPRC5D as described in the present application, and the third antigen binding domain comprises the CD3 binding domain as defined in the present application. In some embodiments, the first polypeptide comprises the amino acid sequence as shown in SEQ ID NO: 33, the second polypeptide comprises the amino acid sequence as shown in SEQ ID NO: 34, the third polypeptide comprises the amino acid sequence as shown in SEQ ID NO: 35, and the fourth polypeptide comprises the amino acid sequence as shown in SEQ ID NO: 36.

In some embodiments, the second polypeptide further comprises, in a N-terminal-to-C-terminal direction, a first Fc polypeptide, and/or the third polypeptide further comprises, in a N-terminal-to-C-terminal direction, a second Fc polypeptide, where the first Fc polypeptide and the second Fc polypeptide are bounded to form a dimer.

In some embodiments, the polypeptide complex comprises the first antigen binding domain and the second antigen binding domain. The first antigen binding domain comprises a first Fab domain, which comprises (i) a first polypeptide, comprising, in a N-terminal-to-C-terminal direction, a first heavy chain variable domain VH1 binding to the first antigen and a first CH1 domain CH1a; and (ii) a second polypeptide, comprising in a N-terminal-to-C-terminal direction, a first light chain variable domain VL1 binding to the first antigen and a first CL domain CLa. The second antigen binding domain comprises a second Fab domain, which comprises (iii) a third polypeptide, comprising, in a N-terminal-to-C-terminal direction, a second heavy chain variable domain VH2 binding to the second antigen and a second CH1 domain CH1b; and (iv) a fourth polypeptide, comprising, in a N-terminal-to-C-terminal direction, a second light chain variable domain VL2 binding to the second antigen and a second CL domain CLb. VL1 and VH1 associate to form the first antigen binding domain; and VL2 and VH2 associate to form the second antigen binding domain. CH1a and CLa are paired, CH1b and CLb are paired, and the pairing of CH1a and CLa and the pairing of CH1b and CLb are configured to avoid mispairing of CH1a and CLb and/or mispairing of CH1b and CLa.

In some embodiments, the first antigen binding domain comprises the antigen binding fragment of an antibody binding to GPRC5D as described in the present application. In some embodiments, the second antigen binding domain comprises the CD3 binding domain as defined in the present application.

In some embodiments, the pairing of CH1b and CLb in the polypeptide complex has at least one non-natural disulfide bond, which hinders mispairing between CH1b and CLa and/or mispairing between CH1a and CLb.

In some embodiments, a first CH1/CL pair and a second CH1/CL pair in the polypeptide complex are respectively selected from CH1b/CLb and CH1a/CLa. The first CH1/CL pair is bound by a first disulfide bond, the first disulfide bond is non-natural, and optionally the originally naturally occurring disulfide bond in the first CH1/CL pair is deleted or destroyed. In some embodiments, the second CH1/CL pair is bound by a second disulfide bond, and the second disulfide bond is located at a different position from the first disulfide bond, and is optionally a naturally occurring disulfide bond.

In some embodiments, the first disulfide bond is formed by two cysteines introduced at positions selected from the group consisting of a) heavy chain EU position 126 and light chain EU position 121 in the first CH1/CL pair; b) heavy chain EU position 173 and light chain EU position 160 in the first CH1/CL pair; and c) heavy chain EU position 128 and light chain EU position 118 in the first CH1/CL pair.

In some embodiments, the naturally occurring disulfide bond is formed between heavy chain EU position 220 and light chain EU position 214. In some embodiments, the first CH1/CL pair comprises CH1 with a mutation to a cysteine residue at EU position 126 and a mutation to a non-cysteine residue at EU position 220; and CL with a mutation to a cysteine residue at EU position 121 and a mutation to a non-cysteine residue at EU position 214.

In some embodiments, the first CH1/CL pair comprises at least one mutation from an uncharged amino acid residue to a charged amino acid residue, and/or at least one mutation from a charged amino acid residue to an oppositely charged amino acid residue, such that the first CH1/CL pair comprises a first pair of oppositely charged residues that favors the pairing of the first CH1/CL pair. In some embodiments, the second CH1/CL pair comprises at least one mutation from an uncharged amino acid residue to a charged amino acid residue, and/or at least one mutation from a charged amino acid residue to an oppositely charged amino acid residue, such that the second CH1/CL pair comprises a second pair of oppositely charged residues that favors the pairing of the second CH1/CL pair, and optionally the first pair of oppositely charged residues and the second pair of oppositely charged residues hinder the pairing of CH1a and CLb or the pairing of CH1b and CLa.

In some embodiments, the first pair of oppositely charged residues and the second pair of oppositely charged residues are configured such that CH1a and CLb are both positively charged or negatively charged, and/or CH1b and CLa are both positively charged or negatively charged.

In some embodiments, the first pair of oppositely charged residues and/or the second pair of oppositely charged residues is introduced at a set of heavy chain-light chain EU positions selected from the group consisting of: a) heavy chain EU position 183 and light chain EU position 176 in the first CH1/CL pair; b) heavy chain EU position 183 and light chain EU position 133 in the first CH1/CL pair; c) heavy chain EU position 147 and light chain EU position 176 in the first CH1/CL pair; d) heavy chain EU position 141 and light chain EU position 116 in the first CH1/CL pair; e) heavy chain EU position 126 and light chain EU position 121 in the first CH1/CL pair; and f) heavy chain EU position 218 and light chain EU position 122 in the first CH1/CL pair.

In some embodiments, the pair of oppositely charged amino acid residues comprises one positively charged amino acid residue and one negatively charged amino acid residue, wherein the positively charged amino acid residue is selected from the group consisting of lysine (K), histidine (H) and arginine (R), and/or the negatively charged amino acid residue is selected from the group consisting of aspartate (D) and glutamate (E).

In some embodiments, the pairing of CH1b and CLb in the polypeptide complex has the first non-natural disulfide bond and the first pair of oppositely charged residues that hinder mispairing between CH1b and CLa and/or mispairing between CH1a and CLb.

In some embodiments, the first polypeptide comprises the amino acid sequence as shown in SEQ ID NO: 39, the second polypeptide comprises the amino acid sequence as shown in SEQ ID NO: 40, the third polypeptide comprises the amino acid sequence as shown in SEQ ID NO: 38, and the fourth polypeptide comprises the amino acid sequence as shown in SEQ ID NO: 37.

In some embodiments, the third polypeptide in the polypeptide complex comprises, in a N-terminal-to-C-terminal direction, a first Fc polypeptide, and the first polypeptide, further comprises, in a N-terminal-to-C-terminal direction, a second Fc polypeptide.

In some embodiments, the polypeptide complex further comprises a third antigen binding domain, and optionally the third antigen binding domain is a Fab domain. In some embodiments, the C terminus of the third antigen binding domain is linked to the N terminus of the second antigen binding domain. In some embodiments, the third antigen binding domain is identical to the first antigen binding domain, and comprises (i) a first fragment, comprising, in a N-terminal-to-C-terminal direction, a first heavy chain variable domain VH1 bonding to the first antigen and a first CH1 domain CH1a; and (ii) a second fragment, comprising, in a N-terminal-to-C-terminal direction, a first light chain variable domain VL1 bonding to the first antigen and a first CL domain CLa, where the C terminus of the first fragment is linked to the N terminus of the fourth polypeptide.

In some embodiments, the polypeptide complex comprises a first polypeptide, a second polypeptide, a third polypeptide, a fourth polypeptide, and a fifth polypeptide. In a N-terminal-to-C-terminal direction, (i) the first polypeptide comprises VH1-CH1a; (ii) the third polypeptide comprises VH2-CH1b; (iii) the fourth polypeptide comprises VH1-CH1a-linker-VL2-CLb; and (iv) the second polypeptide and the fifth polypeptide are identical, and both comprise VL1-CLa.

In some embodiments, the pairing of CH1b and CLb in the polypeptide complex has at least one non-natural disulfide bond, which hinders mispairing between CH1b and CLa and/or mispairing between CH1a and CLb. In some embodiments, the pairing of CH1b and CLb in the polypeptide complex has one or more introduced amino acid mutations and forms at least one introduced charged amino acid residue that hinders mispairing between CH1b and CLa and/or mispairing between CH1a and CLb.

In some embodiments, the first polypeptide comprises the amino acid sequence as shown in SEQ ID NO: 43, the second polypeptide comprises the amino acid sequence as shown in SEQ ID NO: 44, the third polypeptide comprises the amino acid sequence as shown in SEQ ID NO: 42, and the fourth polypeptide comprises the amino acid sequence as shown in SEQ ID NO: 41.

In some embodiments, the first polypeptide comprises the amino acid sequence as shown in SEQ ID NO: 47, the second polypeptide comprises the amino acid sequence as shown in SEQ ID NO: 48, the third polypeptide comprises the amino acid sequence as shown in SEQ ID NO: 46, and the fourth polypeptide comprises the amino acid sequence as shown in SEQ ID NO: 45.

In some embodiments, the third polypeptide in the polypeptide complex further comprises, in a N-terminal-to-C-terminal direction, a first Fc polypeptide, and the first polypeptide comprises in a N-terminal-to-C-terminal direction, a second Fc polypeptide.

In some embodiments, the first Fc polypeptide and/or the second Fc polypeptide in the polypeptide complex is/are derived from IgG1, IgG2, IgG3 or IgG4.

In some embodiments, the first Fc polypeptide and the second Fc polypeptide in the polypeptide complex have different amino acid sequences and are at least configurated to favor the heterodimerization of the first Fc polypeptide and the second Fc polypeptide.

In some embodiments, one of the first Fc polypeptide and the second Fc polypeptide comprises a first Fc mutation, and the other one comprises a second Fc mutation. The first Fc mutation and the second Fc mutation comprise a) a combination of T366W or S354C with Y349C, T366S, L368A or Y407V; b) a combination of D399K or E356K with K392D or K409D; c) a combination of E356K, E357K or D399K with K370E, K409D or K439E; d) a combination of S364H or F405A with Y349T or T394F; e) a combination of S364H or T394F with Y394T or F405A; f) a combination of K370D or K409D with E357K or D399K; or g) a combination of L351D or L368E with L351K or T366K, where the amino acid position is based on EU numbering.

In some embodiments, the first Fc polypeptide comprises the amino acid sequence as shown in SEQ ID NO: 65 or SEQ ID NO: 67, and the second Fc polypeptide comprises the amino acid sequence as shown in SEQ ID NO: 66.

In another aspect, the present application provides a nucleic acid comprising a nucleotide sequence encoding the polypeptide complex as described in the present application.

In another aspect, the present application provides a vector comprising the nucleic acid as described in the present application.

In another aspect, the present application provides a host cell comprising the nucleic acid as described in the present application or the vector as described in the present application.

In another aspect, the present application provides a pharmaceutical composition, comprising the polypeptide complex as described in the present application, the nucleic acid as described in the present application or the vector as described in the present application.

In another aspect, the present application provides a conjugate, comprising the polypeptide complex as described in the present application and a payload conjugated to the polypeptide complex, wherein the payload is selected from the group consisting of a radioactive label, a fluorescent label, an enzyme substrate label, an affinity purification tag, a tracer molecule, an anticancer drug and a cytotoxic molecule.

In another aspect, the present application provides a composition, comprising the polypeptide complex as described in the present application or the conjugate as described in the present application, and a pharmaceutically acceptable carrier.

In another aspect, the present application provides a method for treating or preventing a disease, a disorder, or a condition, comprising administering a therapeutically effective amount of the polypeptide complex as described in the present application, the pharmaceutical composition as described in the present application, the conjugate as described in the present application or the composition as described in the present application to a subject in need thereof.

The disease, the disorder or the condition is selected from the group consisting of cancers, autoimmune diseases, and inflammations.

### BRIEF DESCRIPTION OF ACCOMPANYING DRAWINGS

Fig. 1 shows the binding of an GPRC5D antibody (ch-72C7 and GC5B596) to HEK293 cells expressing hGPRC5D in an antigen binding FACS assay.
Fig. 2 shows the binding of an GPRC5D antibody (ch-72C7 and GC5B596) to CHOS cells expressing hGPRC5D in an antigen binding FACS assay.
Fig. 3 shows the binding of an GPRC5D antibody (ch-72C7 and GC5B596) to natural MM.1R cells expressing GPRC5D in an antigen binding FACS assay.
Fig. 4 shows the binding of an GPRC5D antibody (ch-72C7 and GC5B596) to NCI-H929 cells in an antigen binding FACS assay.
Fig. 5 shows the binding of an GPRC5D antibody (ch-72C7 and GC5B596) to RPMI-8226 cells in an antigen binding FACS assay.
Fig. 6 shows the cytotoxicity of an GPRC5D antibody (ch-72C7 and GC5B596) on natural NCI-H929 cells expressing GPRC5D in an ADCC activity assay.
Fig. 7 shows the cytotoxicity of an GPRC5D antibody (ch-72C7 and GC5B596) on natural MM.1R cells expressing GPRC5D in an ADCC activity assay.
Fig. 8 shows a CD3 × GPRC5D bispecific antibody structure A.
Fig. 9 shows a CD3 × GPRC5D bispecific antibody structure B.
Fig. 10 shows a CD3 × GPRC5D bispecific antibody structure C.
Fig. 11 shows a CD3 × GPRC5D bispecific antibody structure D.
Fig. 12 shows the binding of a bispecific antibody to HEK293T-hGPRC5D (human GPRC5D).
Fig. 13 shows the binding of a bispecific antibody to HEK293T-cynoGPRC5D (Cynomolgus GPRC5D).
Fig. 14 shows the inhibition of bispecific antibody (22A1-5 and 22B1)-mediated PBMC on the proliferation of NCI-H929 cells.
Fig. 15 shows the inhibition of bispecific antibody (22A6 and 10B1)-mediated PBMC on the proliferation of NCI-H929 cells.
Fig. 16 shows the inhibition of bispecific antibody (22A8, 10B1 and 22B1)-mediated PBMC on the proliferation of NCI-H929 cells.
Fig. 17 shows the inhibition of bispecific antibody (22A8 and 22B1)-mediated PBMC on the proliferation of MM1S cells.
Fig. 18 shows the inhibition of bispecific antibody (22A8 and 22B1)-mediated PBMC on the proliferation of RPMI-8226 cells.
Fig. 19 shows the inhibition of bispecific antibody (22A8 and 22B1)-mediated PBMC on the proliferation of KMS-12-BM cells.
Fig. 20 shows the inhibition of a bispecific antibody (22A2 and 22A8) on the tumor in mice developed by subcutaneous transplantation of human myeloma cells NCI-H929 after immune reconstitution with human PBMC.
Fig. 21 shows the inhibition of a bispecific antibody (22B1 and 22A8) on the tumor in mice developed by subcutaneous transplantation of human myeloma cells NCI-H929 after immune reconstitution with human PBMC.
Fig. 22 shows the full-length sequences of various specific antibodies (22A1, 22A2, 22A3, 22A4, 22A5, 22A6, 22A8 and 22B1) in the present application.
Figs. 22(A) to (H) show the amino acid sequences in the polypeptide complex, various mutant sequences of VH and VL in various antigen binding domains targeting GPRC5D, CD3 and Her2, and various mutant sequences of CH1 region and CL region provided in the present application.

### DETAILED DESCRIPTION

Various examples of the present application will be described hereinafter for the purpose of describing the present application. Therefore, particular modifications discussed cannot be construed as limiting on the present application. It will be obvious to those skilled in the art that various equivalents, changes and modifications can be made without departing from the scope of the present application, and it should be understood that these equivalent embodiments will be comprised herein. All references, comprising the publications, patents, and patent applications, cited herein are hereby incorporated by reference in their entirety.

### Definitions

As used herein, the term "antibody" comprises any immunoglobulin, monoclonal antibody, polyclonal antibody, multivalent antibody, divalent antibody, monovalent antibody, multi-specific antibody or bispecific antibody that binds to a specific antigen. Natural intact antibodies comprise two heavy (H) chains and two light (L) chains. Mammalian heavy chains are classified into α, δ, ε, γ and µ chains, and each heavy chain consists of a variable region (V_{H}) and a first, a second, a third, and optionally a fourth constant region (C_{H1}, C_{H2}, C_{H3}, and C_{H4} respectively). Mammalian light chains are classified into λ or κ chain, and each light chain consists of a variable region (V_{L}) and a constant region. The antibody is Y-shaped, in which the stem of Y consists of the second and third constant regions of two heavy chains which are bound together by a disulfide bond. Each arm of Y contains the variable region and the first constant region of a single heavy chain binding to the variable region and the constant region of a single light chain. The variable regions of the light chain and the heavy chain are responsible for antigen binding. The variable regions of the two chains generally contain three highly variable loops, which are called complementarity determining regions (CDRs) (the light-chain CDR comprises LCDR1, LCDR2 and LCDR3, and the heavy chain CDR comprises HCDR1, HCDR2, and HCDR3). CDR boundaries of antibodies and antigen binding fragments disclosed herein can be defined or identified by Kabat, IMGT, Chothia or Al-Lazikani (Al-Lazikani, B., Chothia, C., Lesk, A. M., J. Mol. Biol., 273(4), 927 (1997); Chothia, C. et al., J. Mol. Biol., Dec. 5;186(3):651-63 (1985); Chothia, C. and Lesk, A.M., J. Mol. Biol., 196,901 (1987); Chothia, C. et al., Nature. Dec. 21-28; 342(6252): 877-83 (1989); Kabat E.A. et al., Sequences of Proteins of immunological Interest, Fifth Edition, Public Health Service, National Institutes of Health, Bethesda, Md.) (1991); Marie-Paule Lefranc et al., Developmental and Comparative Immunology, 27: 55-77 (2003); Marie-Paule Lefranc et al., Immunome Research, 1(3), (2005); Marie-Paule Lefranc, Molecular Biology of B cells (second edition), Chapter 26, 481-514, (2015)). The three CDRs are inserted between flanking segments called framework regions (FRs), which are more conservative than CDRs and form a scaffold to support the hypervariable loops. The constant regions of the heavy chain and the light chain are not involved in antigen binding, but show various effector functions. The antibody is classified based on the amino acid sequence of the heavy-chain constant region of the antibody. The five main types or isoforms of antibodies are IgA, IgD, IgE, IgG and IgM, which are characterized by the existence of α, δ, ε, γ and µ heavy chain respectively. Several major antibody categories are classified into subclasses, such as IgG1 (γ1 heavy chain), IgG2 (γ2 heavy chain), IgG3 (γ3 heavy chain), IgG4 (γ4 heavy chain), IgA1 (α1 heavy chain) or IgA2 (α2 heavy chain).

In the present application, the numbering indicating the positions of amino acid residues in the constant region of the antibody is based on the EU numbering, see, for example, Edelman, G.M. et al., Proc. Natl. Acad. USA, 63, 78-85 (1969). The numbering indicating the positions of amino acid residues in the variable region of the antibody is based on the EU numbering, see, for example, Kabat et al., Sequences of Proteins of Immunological Interest, 5th Ed. Public Health Service, National Institutes of Health, Bethesda, Md. (1991). These numbering systems and their corresponding relations therebetween can be found in the IMGT scientific chart obtained from the website of the International ImMunoGeneTics information system.

As used herein, the term "divalent" refers to an antibody or antigen binding fragment having two antigen binding sites. The term "monovalent" refers to an antibody or antigen binding fragment having only a single antigen binding site. The term "multivalent" refers to an antibody or antigen binding fragment having multiple antigen binding sites. In some embodiments, the antibody or an antigen binding fragment thereof is monovalent, divalent or multivalent.

As used herein, the "bispecific" antibody refers to an artificial antibody that has fragments derived from two different monoclonal antibodies and can bind to two different epitopes. The two epitopes can exist on the same antigen, or on two different antigens.

As used herein, the "multi-specific" antibody refers to an artificial antibody that has fragments derived from more than two different monoclonal antibodies and can bind to more than two different epitopes. The more than two different epitopes may exist on the same antigen or on different antigens.

As used herein, the term "antigen binding fragment" refers to an antibody fragment formed by a part of an antibody that comprises one or more CDRs, or any other antibody fragment that binds to an antigen, but does not comprise an intact natural antibody structure. Examples of the antigen binding fragment comprise, but are not limited to, a diabody, Fab, Fab', F(ab')₂, an Fv fragment, a disulfide stabilized Fv fragment (dsFv), (dsFv)₂, a bispecific dsFv(dsFv-dsFv'), a disulfide stabilized diabody (ds diabody), a single-chain antibody molecule (scFv), an scFv dimer (a divalent diabody), a bispecific antibody, a multi-specific antibody, a camelized single-domain antibody, a nanobody, a domain antibody, and a divalent domain antibody. The antigen binding fragment can bind to the same antigen that the parent antibody binds.

The "Fab" with reference to an antibody refers to a part of the antibody consisting of a single light chain (variable region and constant region) bound to the variable region and the first constant region of a single heavy chain by a disulfide bond.

The Fab' refers to a Fab fragment containing a part of the hinge region.

"F(ab')₂" refers to a dimer of Fab'. The Fv with reference to an antibody refers to the smallest antibody fragment with a complete antigen binding site. The Fv fragment consists of the variable region of a single light chain bound to the variable region of a single heavy chain.

The "dsFv" refers to a disulfide stabilized Fv fragment, in which the linkage between the variable region of a single light chain and the variable region of a single heavy chain is a disulfide bond. In some embodiments, "(dsFv)₂" or "(dsFv-dsFv')" comprises three peptide chains, that is, two V_{H} moieties linked by a peptide linker (such as a long flexible linker) and bound to two V_{L} moieties, respectively, by disulfide bridges. In some embodiments, dsfv-dsfv' is bispecific, in which each pair of heavy chain and light chain linked by a disulfide bond has a different antigen specificity.

The "single-chain Fv antibody" or "scFv" refers to an engineered antibody consisting of a light chain variable region and a heavy chain variable region linked to each other directly or by a peptide linker sequence (Huston JS et al., Proc Natl Acad Sci USA, 85:5879(1988)).

The "Fc" of an antibody (such as IgG, IgA or IgD isotype antibody) refers to a part of the antibody consisting of the second and third constant domains of a first heavy chain bound to the second and third constant domains of the second heavy chain by a disulfide bond. The Fc of IgM and IgE isotype antibodies further comprises a fourth constant domain. The Fc part of an antibody is responsible for various effector functions, such as antibody-dependent cell-mediated cytotoxicity (ADCC) and complement-dependent cytotoxicity (CDC), but it does not play a role in antigen binding.

The "single chain Fv-Fc antibody" or "scFv-Fc" refers to an engineered antibody consisting of ScFv linked to the FC region of an antibody.

The "camelized single-domain antibody", "heavy chain antibody", or "HCAb" refer to an antibody containing two V_{H} domains and not containing a light chain (Riechmann L. and Muyldermans S., J Immunol Methods., Dec. 10; 231(1-2): 25-38 (1999); Muyldermans S., J Biotechnol., June; 74(4): 277-302 (2001); WO94/04678; WO94/25591; US Patent No. 6,005,079). The heavy-chain antibody is originally derived from *Camelidae* (camel, dromedary and llama). Despite the lack of light chain, the camelized antibody has an authentic antigen-binding repertoire (Hamers-Casterman C. et al., Nature, June 3; 363(6428): 446-8 (1993); Nguyen VK. et al. Immunogenetics., April; 54(1):39-47 (2002); Nguyen VK. et al. Immunology., May; 109(1): 93-101 (2003)). The variable domain (VHH domain) of the heavy chain antibody represents the known smallest antigen binding unit generated by adaptive immune responses (Koch-Nolte F. et al., FASEB J., Nov.; 21(13): 3490-8. E-version, June 15, 2007 (2007)).

The "nanobody" refers to an antibody fragment consisting of a VHH domain from a heavy chain antibody and two constant domains CH2 and CH3.

The "bifunctional antibody" or "dAb" comprises a small antibody fragment with two antigen binding sites, where the fragment comprises a V_{H} domain linked to a V_{L} domain in the same polypeptide chain (V_{H}-V_{L} or V_{L}-V_{H}) (see, for example, Holliger P. et al., Proc Natl Acad Sci USA, July 15; 90(14): 6444-8 (1993); EP404097; WO93/11161).By using a linker that is too short to cause two domains on the same chain to pair, the domain is forced to pair with a complementary domain on the other chain, thus producing two antigen binding sites. The antigen binding sites can target the same or different antigens (or epitopes). In some embodiments, the "bispecific ds bifunctional antibody" is a bifunctional antibody targeting two different antigen (or epitopes). In some embodiments, the "scFv dimer" is a divalent bifunctional antibody or divalent scFv (BsFv), which comprises a dimer of V_{H}-V_{L} (linked by a peptide linker) with another V_{H}-V_{L} moiety, so that V_{H} in one moiety coordinates with V_{L} in another moiety to form two binding sites that can target the same antigen (or epitope) or different antigens (or epitopes). In other embodiments, the "scFv dimer" is a bispecific bifunctional antibody, which comprises a combination of V_{H1}-V_{L2} (linked by a peptide linker) with V_{L1}-V_{H2} (linked by a peptide linker), such that V_{H1} coordinates with V_{L1} and V_{H2} coordinates with V_{L2}, where each coordinated pair has different antigen specificity.

The "domain antibody" refers to an antibody fragment containing only the variable region of a heavy chain or the variable region of a light chain. In some cases, two or more V_{H} domains are covalently bound by a peptide linker, to produce a bivalent or multivalent domain antibody. The two V_{H} domains of a divalent domain antibody can target the same or different antigens.

As used herein, the term "chimeric" refers to an antibody or an antigen binding fragment in which a part of the heavy chain/or light chain is derived from one species and the rest of the heavy chain/or light chain is derived from a different species. In an illustrative example, the chimeric antibody may comprise a constant region derived from human and a variable region derived from a non-human animal such as mice. In some embodiments, the non-human animal is a mammal, such as mice, rats, rabbits, goats, sheep, guinea pigs or hamsters.

As used herein, the term "humanized" means that an antibody or an antigen binding fragment comprises a CDR derived from a nonhuman animal, a FR region derived from human, and, where applicable, a constant region derived from human.

As used herein, "GPRC5D" refers to G protein-coupled receptor class C group 5 member D derived from primates (such as humans and monkeys). In some embodiments, GPRC5D is human GPRC5D. An exemplary sequence of human GPRC5D comprises human GPRC5D protein (UniProt No. Q9NZD1). An exemplary sequence of monkey GPRC5D comprise, for example, rhesus monkey GPRC5D protein (UniProt No. F6Y5U7) or cynomolgus monkey GPRC5D protein (UniProt No. A0A2K5W6I7). GPRC5D is a relatively new target for immunotherapy of multiple myeloma. It is an orphan G protein-coupled receptor with unknown functions, highly expressed in malignant bone marrow plasma cells and hard keratinized structures comprising hair shaft, nails and the central area of tongue (see Smith EL et al., Sci Transl Med 2019; 11: eaau7746; Pillarisetti K et al., Blood 2020;135:1232-43.; and Inoue S et al., J Invest Dermatol 2004;122:565-73). The high expression of GPRC5D is related to the poor prognosis of multiple myeloma (see Atamaniuk J et al., Eur J Clin Invest 2012;42:953-60.). GPRC5D is used as a target of CART in the treatment of multiple myeloma, with promising results achieved in preclinical studies (see de Larrea CF et al., Blood Cancer Discov 2020;1:146.); and is now a target of the bispecific antibody JNJ-64407564 (talquetamab) in four phase I trials.

As used herein, the term "CD3" refers to cluster of differentiation 3, which is a protein complex and a co-receptor of T cells participating in the activation of cytotoxic T cells (CD8+ naive T cells) and T helper cells (CD4+ naive T cells). CD3 is a complex composed of four different chains. In mammals, the complex contains one CD3γ chain, one CD3δ chain and two CD3ε chains. These chains binds to the T cell receptor (TCR) and CD3ζ chain (ζ-chain) to produce an activation signal in T lymphocytes. TCR, CD3ζ and other CD3 molecules together form a TCR complex. Because CD3 is essential for T cell activation, drugs targeting CD3 (usually monoclonal antibodies) are being studied for use as immunosuppressant therapy for cancers and other autoimmune diseases. Based on CD3, a co-receptor of T cells, new therapies with anti-cancer drugs are being developed, and molecules are designed to change the co-stimulatory signals to facilitate T cells to recognize cancer cells and be fully activated.

As used herein, the term "Her2" refers to human epidermal growth factor receptor 2, also known as receptor tyrosine protein kinase erbB-2, cluster of differentiation 340 or protooncogene neu, which is encoded by the gene *ErbB2. ErbB* is the abbreviation of erythroblastic oncogene B, which belongs to the epidermal growth factor receptor family, and consists of an extracellular domain, a transmembrane domain and a cytoplasmic tyrosine kinase domain. In humans, the *ErbB* family comprises four members: *ErbB1*(Her1), *ErbB2(Her2), ErbB3(Her3),* and *ErbB4*(Her4). Contrary to other members in the *ErbB* family, Her2 does not directly bind to the ligand. When the concentration of Her2 is high (for example, in a cancer environment), its homodimerization or heterodimerization with another *ErbB* member can cause the activation of Her2. The amplification or overexpression of Her2 gene plays an important role in the development and progress of some invasive breast cancers. In recent years, the Her2 protein has become an important biomarker and therapeutic target in about 30% of the patients with breast cancer.

The term "anti-GPRC5D antibody" refers to an antibody that can specifically bind to GPRC5D (for example, human GPRC5D). The term "anti-human GPRC5D antibody" refers to an antibody that can specifically bind to human GPRC5D.

As used herein, the term "specific binding/specifically binding" refers to a non-random binding reaction between two molecules, such as, for example, beween an antibody and an antigen. The specific binding can be characterized by the binding affinity, for example, expressed by K_{D}, the ratio (k_{off}/kₒₙ) of the dissociation rate to the binding rate when the binding between an antigen and an antigen binding molecule reaches equilibrium. K_{D} can be determined by any conventional method known in the art, including, but not limited to, surface plasmon resonance, microscale thermophoresis, HPLC-MS and flow cytometry (such as FACS). An K_{D} of ≤10⁻⁶ M (for example, ≤5 × 10⁻⁷ M, ≤2 × 10⁻⁷ M, ≤10⁻⁷ M, ≤5 × 10⁻⁸ M, ≤2 × 10⁻⁸ M, ≤10⁻⁸ M, ≤5 × 10⁻⁹ M, ≤4 × 10⁻⁹M, ≤3 × 10⁻⁹M, ≤3 × 1⁻⁹ M or ≤10⁻⁹ M) indicates the specific binding between an antibody or an antigen binding fragment thereof and GPRC5D (for example, human GPRC5D).

The conservative substitution of an amino acid sequence refers to the replacement of an amino acid residue by a different amino acid residue with a side chain having similar physiological and chemical characteristics. For example, conservative substitution can be made among amino acid residues with a hydrophobic side chain (for example, Met, Ala, Val, Leu and Ile), amino acid residues with a neutral or hydrophilic side chain (for example, Cys, Ser, Thr, Asn and Gln), amino acid residues with an acidic side chain (for example, Asp, and Glu), amino acid residues with a basic side chain (for example, His, Lys, and Arg), or amino acid residues with an aromatic side chain (for example, Trp, Tyr and Phe). As is known in the art, conservative substitution usually does not cause significant changes in the conformation structure of a protein, and thus the biological activity of the protein can be retained.

As used herein, the term "homologous" means that a nucleic acid sequence (or a complementary strand thereof) or an amino acid sequence has at least 60% (*e.g.,* at least 65%, 70%, 75%, 80%, 85%, 88%, 90%, 91%, 92%, 93%, 94%, 95%, 96%, 97%, 98%, or 99%) sequence identity to another sequence in optimal alignment.

The "percent sequence identity (%)" with reference to an amino acid sequence (or a nucleic acid sequence) is defined as the percent amino acid (or nucleic acid) residues in a candidate sequence that are identical those in a reference sequence after aligning the candidate sequence with the reference sequence and introducing gaps when necessary to maximize the number of identical amino acids (or nucleic acids). Conservative substitution of amino acid residues may or may not be regarded as an identical residue. The alignment for the purpose of determining the percent amino acid (or nucleic acid) sequence identity can carried out using, for example, publicly available tools, such as BLASTN and BLASTp (available on the website of National Center for Biotechnology Information (NCBI), and see also Altschul S.F. et al., J. Mol. Biol., 215:403-410 (1990); Stephen F. et al., Nucleic Acids Res., 25:3389-3402 (1997)), ClustalW2 (available on the website of European Bioinformatics Institute, and also see Higgins D.G. et al., Methods in Enzymology, 266:383-402 (1996); Larkin M.A. et al., Bioinformatics, Oxford, England, 23(21): 2947-8 (2007)) and ALIGN or Megalign (DNASTAR) software. Those skilled in the art can use the default parameters provided by the tool, or customize the parameters suitable for alignment, such as by selecting an appropriate algorithm.

As used herein, the "effector function" refers to the biological activity caused by the binding of the Fc region of an antibody to its effector, such as the C1 complex to the Fc receptor. Exemplary effector functions include: complement-dependent cytotoxicity (CDC) mediated by the interaction of an antibody with C1q on the C1 complex; antibody-dependent cell-mediated cytotoxicity (ADCC) mediated by the binding of the Fc region of an antibody to the Fc receptor on effector cells; and phagocytosis. Various assays (such as Fc receptor binding assay, C1q binding assay and cytolysis assay) can be used to evaluate the effector function.

The "isolated" material has been changed from its natural state by artificial means. If an "isolated" composition or material exists in nature, it has been changed or removed from its original environment, or both. For example, a polynucleotide or polypeptide naturally existing in a living animal is not "isolated", but the polynucleotide or polypeptide is "isolated" if it is sufficiently separated from materials coexisting therewith in nature state and thus exists in a substantially pure state. "An isolated nucleic acid sequence" refers to the sequence of an isolated nucleic acid molecule. In some embodiments, the "isolated antibody or an antigen binding fragment thereof" means an antibody or an antigen binding fragment thereof having a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% as determined by electrophoresis (such as SDS-PAGE, isoelectric focusing, and capillary electrophoresis) or chromatography (such as ion exchange chromatography or reversed-phase HPLC).

As used herein, the term "vector" refers to a vehicle into which a gene element is operably inserted to realize the expression of the gene element, thereby generating a protein, RNA or DNA encoded by the gene element, or replicating the gene element. The vector can be used to transform, transduct or transfect the host cells, so that the gene elements carried can be expressed in the host cells. Examples of vectors include plasmids; phagemids; cosmids; artificial chromosome, such as yeast artificial chromosome (YAC), bacterial artificial chromosome (BAC) or P1-derived artificial chromosome (PAC); phage, such as λ phage or M13 phage; and animal viruses. The vector can contain various elements for controlling expression, including a promoter sequence, a transcription initiation sequence, an enhancer sequence, a selection element and a reporter gene. In addition, the vector can contain the origin of replication. The vector can also contain substances promoting the entry into cells, including, but not limited to, virus particles, liposomes or protein envelopes. The vector can be an expression vector or a cloning vector. The present application provides a vector (such as an expression vector) containing a nucleic acid sequence encoding the polypeptide complex provided herein, at least one promoter (such as SV40, CMV, and EF-1α) operably linked to the nucleic acid sequence, and at least one selectable marker.

As used herein, the phrase "host cell" refers to a cell into which an exogenous polynucleotide and/or vector can be or has been introduced.

As used herein, the "treating" or "treatment" of a condition includes alleviating the condition, slowing down the onset or development rate of the condition, reducing the risk of suffering from the condition, delaying the development of symptoms related to the condition, reducing or eliminating symptoms related to the condition, causing complete or partial regression of the condition, and curing the condition or some combination thereof.

As used herein, the "GPRC5D-related" disease or condition refers to any disease or condition caused or aggravated by, or otherwise related to the increase or decrease of the expression or activity of GPRC5D. In some embodiments, the GPRC5D-related diseases or condition is cancers, such as myeloma. In some embodiments, the GPRC5D-related disease or condition is characterized by over-expression of GPRC5D gene. In one embodiment, the GPRC5D-related disease or condition includes, but is not limited to, GPRC5D positive breast cancer, multiple myeloma, Waldenstörm macroglobulinemia, endometrial cancer, ovarian cancer, lung cancer, gastric cancer, prostate cancer, renal cancer, liver cancer, pancreatic cancer, colorectal cancer, esophageal cancer, bladder cancer, cervical cancer, hematological cancer, lymphoma or malignant melanoma.

The term "pharmaceutically acceptable" indicates that the specified carrier, vehicle, diluent, excipient and/or salt are generally chemically and/or physically compatible with other ingredients constituting the formulation and physiologically compatible with its recipient.

### Multi-specific polypeptide complex

In one aspect, the present application provides a multi-specific polypeptide complex, which comprises a first antigen binding domain and a second antigen binding domain. The first antigen binding domain comprises a first antigen binding domain binding to a first antigen, and the second antigen binding domain comprises a second antigen binding domain binding to a second antigen. In some embodiments, the polypeptide complex further comprises a third antigen binding domain. The third antigen binding domain comprises a third antigen binding domain binding to a third antigen.

### i. Target of polypeptide complex

In one embodiment, at least one of the first antigen binding domain and the second antigen binding domain in the polypeptide complex described in the present application binds to GPRC5D and comprises a GPRC5D binding domain. In some embodiments, at least one of the first antigen binding domain, the second antigen binding domain and the third antigen binding domain in the polypeptide complex described in the present application binds to GPRC5D and comprises a GPRC5D binding domain.

### GPRC5D binding domain

The GPRC5D binding domain in the polypeptide complex can specifically bind to GPRC5D.

In some embodiments, the GPRC5D binding domain specifically binds to human GPRC5D at a K_{D} value of no more than 8 × 10⁻⁸ M, no more than 5 × 10⁻⁸ M, no more than 2 × 10⁻⁸ M, no more than 8 × 10⁻⁹ M, no more than 5 × 10⁻⁹ M, no more than 2 × 10⁻⁹ M, not more than 10⁻⁹ M, no more than 8 × 10⁻¹⁰ M, no more than 7 × 10⁻¹⁰ M or no more than 6 × 10⁻¹⁰ M as shown by Biacore analysis. The Biacore analysis is based on the surface plasmon resonance technology, see, for example, Murphy, M. et al., Current protocols in protein science, 2006.Chapter 19, Unit 19.14, 2006.

The binding of the GPRC5D binding domain to human GPRC5D can also be expressed by the "median effective concentration" (EC₅₀), and EC₅₀ refers to the antibody concentration when 50% of maximum binding is observed. The EC₅₀ value can be measured by binding assays known in the art, such as sandwich assays, such as enzyme-linked immunosorbent assay (ELISA), flow cytometry and other binding assays. In some embodiments, as measured by flow cytometry, the antibody and a fragment thereof provided herein specifically bind to cells expressing human GPRC5D at an EC₅₀ value (i.e. 50% binding concentration) of no more than 1 µg/ml, no more than 2 µg/ml, no more than 3 µg/ml, no more than 4 µg/ml, no more than 5 µg/ml, and no more than 10 µg/ml.

As used herein, the "binding ability" refers to the ability of a molecule (such as an antibody) to bind to another molecule (such as an antigen). The ability can be measured by, for example, the binding activity with an antigen of interest using any suitable binding assay known in the art. For example, the antibody of interest can be labeled to allow for the direct quantification of the binding activity with the antigen. For another example, the binding activity of an antibody of interest (i.e., a primary antibody) to its antigen can also be detected by using a labeled secondary antibody (*e.g.,* an anti-species antibody) that detects, by binding to the primary antibody in the complex, the complex formed by the primary antibody bound to its antigen, and thus indirectly quantifies the binding activity. The labeled antibody can be detected by, for example, enzyme-linked immunosorbent assay (ELISA, for example, where the label is an enzyme), flow cytometry (for example, where the label is fluorescent), Western blotting (for example, the label is a fluorescent or radioactive ligand), colorimetry, and chemiluminescence-based methods, etc.

In some embodiments, the GPRC5D binding domain in the polypeptide complex of the present application is derived from the antigen binding domain of the monoclonal antibody ch-72C7 against GPRC5D.

As used herein, "ch-72C7" refers to monoclonal antibody having a light chain variable region comprising a sequence as shown in SEQ ID NO: 12 and a heavy chain variable region comprising a sequence as shown in SEQ ID NO: 13. ch-72C7 is an antibody from mice, in which the CDR sequence can be divided by methods known in the art, including, but not limited to, IMGT, or based on Kabat numbering.

Table 1 below shows the CDR sequence of antibody ch-72C7 divided based on IMGT numbering. Table 2 below shows the CDR sequence of antibody ch-72C7 divided based on Kabat numbering. Table 3 below shows the amino acid sequences of the heavy chain and light chain variable region of GPRC5D.

**Table 1. Amino acid sequence of CDR of antibody ch-72C7 based on IMGT numbering**

| | | **CDR1** | **CDR2** | **CDR3** |
|---|---|---|---|---|
| **ch-72C7** | **LCDR** | SEQ ID NO: 1 SSVSF | SEQ ID NO: 2 DTT | SEQ ID NO: 3 QQWNSHPLT |
| | **HCDR** | SEQ ID NO: 4 GYPFTNYW | SEQ ID NO: 5 INPSNGRT | SEQ ID NO: 6 ARGFAY |

**Table 2. Amino acid sequence of CDR of antibody ch-72C7 divided based on Kabat numbering**

| | | **CDR1** | **CDR2** | **CDR3** |
|---|---|---|---|---|
| **ch-72C7** | **LCDR** | SEQ ID NO: 7 SASSSVSFMH | SEQ ID NO: 8 DTTKLAS | SEQ ID NO: 3 QQWNSHPLT |
| | **HCDR** | SEQ ID NO: 9 NYWMH | SEQ ID NO: 10 EINPSNGRTNYNEKFKS | SEQ ID NO: 11 GFAY |

**Table 3. Amino acid sequence of variable region of ch-72C7**

| | **VL** | **VH** |
|---|---|---|
| **ch-72C7** | SEQ ID NO: 12 QIVLSQFPAIMSASPGEKVTMT CSASSSVSFMHWYQQKSGTSP KRWIYDTTKLASGVPARFTGSG SGTSYSLTISSMEAEDAATYYC QQWNSHPLTFGPGTKLELK | SEQ ID NO: 13 QVQLQQPGAELVKPGTSVKLSCKAS GYPFTNYWMHWVRQRPGQGLEWI GEINPSNGRTNYNEKFKSLATLTLDK SSSTAYMHLNSLTSEDSAVYYCARG FAYWGQGTLVTVSA |

In some embodiments, the GPRC5D binding domain in the polypeptide complex of the present application comprises one or more (*e.g.* 1, 2, 3, 4, 5 or 6) CDR sequences of antibody ch-72C7.

In some embodiments, the GPRC5D binding domain in the polypeptide complex of the present application comprises three heavy chain complementarity determining regions HCDR1, HCDR2 and HCDR3, and three light chain complementarity determining regions LCDR1, LCDR2, and LCDR3. The three heavy chain complementarity determining regions are identical to three heavy chain complementarity determining regions comprised in a heavy chain variable region (V_{H}) as shown in SEQ ID NO: 13, and the light chain complementarity determining regions are identical to three light chain complementarity determining regions comprised in a light chain variable region (V_{L}) as shown in SEQ ID NO: 12.

In some embodiments, the GPRC5D binding domain in the polypeptide complex of the present application comprises one or more (*e.g.* 1, 2, 3, 4, 5 or 6) CDRs. The one or more CDRs comprise a sequence selected from the group consisting of SSVSF (SEQ ID NO: 1), DTT (SEQ ID NO: 2), QQWNSHPLT (SEQ ID NO: 3), GYPFTNYW (SEQ ID NO: 4), INPSNGRT (SEQ ID NO: 5) and ARGFAY (SEQ ID NO: 6).

In some embodiments, the GPRC5D binding domain in the polypeptide complex of the present application comprises one or more (*e.g.* 1, 2, 3, 4, 5 or 6) CDRs. The one or more CDRs comprise a sequence selected from the group consisting of SASSSVSFMH (SEQ ID NO: 7), DTTKLAS (SEQ ID NO: 8), QQWNSHPLT (SEQ ID NO: 3), NYWMH (SEQ ID NO: 9), EINPSNGRTNYNEKFKS (SEQ ID NO: 10) and GFAY (SEQ ID NO: 11).

In some embodiments, the GPRC5D binding domain in the polypeptide complex of the present application comprises LCDR1 comprising the amino acid sequence as shown in SEQ ID NO: 1, LCDR2 comprising the amino acid sequence as shown in SEQ ID NO: 2, and LCDR3 comprising the amino acid sequence as shown in SEQ ID NO: 3, and/or HCDR1 comprising the amino acid sequence as shown in SEQ ID NO: 4, HCDR2 comprising the amino acid sequence as shown in SEQ ID NO: 5, and HCDR3 comprising the amino acid sequence as shown in SEQ ID NO: 6. In some embodiments, the GPRC5D binding domain in the polypeptide complex of the present application comprises LCDR1 comprising the amino acid sequence as shown in SEQ ID NO: 7, LCDR2 comprising the amino acid sequence as shown in SEQ ID NO: 8, and LCDR3 comprising the amino acid sequence as shown in SEQ ID NO: 3, and/or HCDR1 comprising the amino acid sequence as shown in SEQ ID NO: 9, HCDR2 comprising the amino acid sequence as shown in SEQ ID NO: 10, and HCDR3 comprising the amino acid sequence as shown in SEQ ID NO: 11.

In some embodiments, in the GPRC5D binding domain in the polypeptide complex of the present application, the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

In some embodiments, in the GPRC5D binding domain in the polypeptide complex of the present application, the HCDR1 comprises the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

CDR is known to be responsible for antigen binding. However, it has been found that not all 6 CDRs are essential or unchangeable. In other words, it is possible to replace, change or modify one or more CDRs in the anti-GPRC5D antibody ch-72C7, with the specific binding affinity for GPRC5D being substantially retained.

In some embodiments, the GPRC5D binding domain in the polypeptide complex provided herein comprises a heavy chain CDR3 sequence of antibody ch-72C7. In some embodiments, the GPRC5D binding domain in the polypeptide complex provided herein comprises a heavy chain CDR3 sequence as shown in SEQ ID NO: 6, where the CDR3 is numbered based on IMGT numbering. In some embodiments, the GPRC5D binding domain in the polypeptide complex provided herein comprises a heavy chain CDR3 sequence as shown in SEQ ID NO: 11, where the CDR3 is numbered based on Kabat numbering.

In some embodiments, the GPRC5D binding domain in the polypeptide complex provided herein comprises a suitable framework region (FR) sequence, as long as the GPRC5D binding domain can specifically bind to GPRC5D. The CDR sequence provided in Table 1 or Table 2 above is obtained from a mouse antibody, but it can be transplanted into any suitable FR sequence of any suitable species such as mouse, human, rat, and rabbit, etc. using suitable methods known in the art, such as recombinant technology.

In some embodiments, the GPRC5D binding domain in the polypeptide complex provided herein is humanized. The humanized antibody or antigen binding fragment is desirable in reducing the immunogenicity in human. The humanized antibody is chimeric in its variable region, because the non-human CDR sequence is transplanted into a human or substantially human FR sequence. The humanization of an antibody or an antigen binding fragment can basically be carried out by replacing a non-human (such as mouse) CDR gene with a corresponding human CDR gene in human immunoglobulin gene (see, for example, Jones et al. (1986) Nature, 321:522-525; Riechmann et al. (1988) Nature, 332:323-327; Verhoeyen et al. (1988) Science 239:1534-1536).

Methods known in the art can be used to select suitable human heavy chain and light chain variable domains for this purpose. In an illustrative example, the "best fit" method can be used, in which a variable domain sequence of a non-human (*e.g.,* rodent) antibody is screened or blasted against a database of known human variable domain sequences, and a human sequence closest to a non-human query sequence is identified, and used as a human scaffold for transplanting a non-human CDR sequence (see, for example, Sims et al., (1993) J Immunol. 151: 2296; and Chothia et al. (1987) J. Mol. Biol. 196: 901). Alternatively, the framework derived from the consensus sequence of all human antibodies can be used for the transplantation of non-human CDRs (see, for example, Carter et al. (1992) Proc Natl Acad Sci USA, 89:4285; and Presta et al. (1993) J Immunol.,151:2623).

In some embodiments, the humanized GPRC5D binding domain provided herein Consists essentially of human sequences except that the CDR sequence is non-human. In some embodiments, the variable region FR and the constant region, if present, are completely or substantially derived from the human immunoglobulin sequence. The human FR sequence and the human constant region sequence can be derived from different human immunoglobulin genes. For example, the FR sequence is derived from one human antibody and the constant region is derived from another human antibody. In some embodiments, the humanized antibody or antigen binding fragment comprises human heavy chain HFR1-4 and/or light chain LFR1-4.

In some embodiments, the human-derived FR region may comprise the same amino acid sequence as the human immunoglobulin from which it is derived. In some embodiments, one or more amino acid residues of human FR are substituted with a corresponding residue from a parent non-human antibody. In some embodiments, it may be necessary to closely approximate the structure of a humanized antibody or a fragment thereof to a non-human parent antibody. In some embodiments, the humanized GPRC5D binding domain provided herein comprises no more than 10, 9, 8, 7, 6, 5, 4, 3, 2 or 1 amino acid residue substitution in each human FR sequence, or no more than 10, 9, 8, 7, 6, 5, 4, 3, 2 or 1 amino acid residue substitution in all FRs of the heavy chain or light chain variable domain. In some embodiments, such changes in amino acid residues can exist only in the FR region of the heavy chain, only in the FR region of the light chain, or in both chains.

The present application also provides an exemplary humanized GPRC5D binding domain derived from ch-72C7, comprising:
1) "22Mono5JO4", comprising a heavy chain variable region (22Mono5JO4-VH) with an amino acid sequence as shown in SEQ ID NO: 15 and a light chain variable region (22Mono5JO4-VL) with an amino acid sequence as shown in SEQ ID NO: 14;
2) "22Mono3L7F", comprising a heavy chain variable region (22Mono3L7F-VH) with an amino acid sequence as shown in SEQ ID NO: 17 and a light chain variable region (22Mono3L7F-VL) with an amino acid sequence as shown in SEQ ID NO: 16;
3) "22Mono4DN4", comprising a heavy chain variable region (22Mono4DN4-VH) with an amino acid sequence as shown in SEQ ID NO: 19 and a light chain variable region (22Mono4DN4-VL) with an amino acid sequence as shown in SEQ ID NO: 18; and
4) "22Mono5UQY", comprising a heavy chain variable region (22Mono5UQY-VH) with an amino acid sequence as shown in SEQ ID NO: 21 and a light chain variable region (22Mono5UQY-VL) with an amino acid sequence as shown in SEQ ID NO: 20.

**Table 4. Amino acid sequence of variable region of humanized GPRC5D binding domain**

| | **VL** | **VH** |
|---|---|---|
| 22Mono5JO4 | SEQ ID NO: 14 EIVLTQSPGTLSLSPGERATLSC SASSSVSFMHWYQQKPGQAPR RWIYDTTKLASGIPDRFSGSGS GTDYTLTISRLEPEDFAVYYCQ QWNSHPLTFGQGTTVEIK | SEQ ID NO: 15 QVQLVQSGAEVKKPGSSVKVS CRASGYPFTNYWMHWVRQAP GQGLEWIGEINPSNGRTNYNEK FKSRATLTLDKLTSTAYMELSSL TSEDTALYYCARGFAYWGQGT LVTVSS |
| 22Mono3L7F | SEQ ID NO: 16 EIVLTQSPATLSLSPGERATLSCS ASSSVSFMHWYQQKPGQAPRR WIYDTTKLASGIPARFSGSGSG TDYTLTISSLEPEDFAVYYCQQ WNSHPLTFGQGTKLEIK | SEQ ID NO: 17 EVTLKESGPVLVKPTETLTLTCT ASGYPFTNYWMHWVRQPPGK ALEWIGEINPSNGRTNYNEKFK SRATLSLDKSKSQAVLTMTNM DPVDTATYYCARGFAYWGQGT LVTVSS |
| 22Mono4DN4 | SEQ ID NO: 18 EIVLTQSPATLSLSPGERATLSCS ASSSVSFMHWYQQKPGQAPRR WIYDTTKLASGVPARFSGSGSG TDYTLTISSLEPEDFAVYYCQQ WNSHPLTFGQGTKVEIK | SEQ ID NO: 19 ELVQSGAEVKKPGSSVKVSCK ASGYPFTNYWMHWVRQAPGQ GLEWIGEINPSNGRTNYNEKFK SRATLTLDKSTSTAYMELSSLRS EDTAVYYCARGFAYWGQGTLV TVSS |
| 22Mono5UQY | SEQ ID NO: 20 AIQLTQSPSSLSASVGDRVTITC SASSSVSFMHWYQQKPGKAPK RWIYDTTKLASGVPSRFSGSGS GTDYTFTISSLQPEDIATYYCQQ WNSHPLTFGPGTKVDIK | SEQ ID NO: 21 QVQLQESGPGLVKPSETLSLTC TASGYPFTNYWMHWVRQPPG KGLEWIGEINPSNGRTNYNEKF KSRATLSLDKSKNQASLNLDSV SAADTAIYYCARGFAYWGKGS TVTVSS |

In some embodiments, the present invention further provides a humanized GPRC5D binding domain, which comprises a HFR1, HFR2, HFR3 and/or HFR4 sequence in a heavy chain variable region. The heavy chain variable region is selected from the group consisting of 22Mono5JO4-VH (SEQ ID NO: 15), 22Mono3L7F-VH (SEQ ID NO: 17), 22Mono4DN4-VH (SEQ ID NO: 19) and 22Mono5UQY-VH (SEQ ID NO: 21).

In some embodiments, the present invention further provides a humanized GPRC5D binding domain, which comprises a LFR1, LFR2, LFR3 and/or LFR4 sequence in a light chain variable region. The light chain variable region is selected from the group consisting of 22Mono5JO4-VL (SEQ ID NO: 14), 22Mono3L7F-VL (SEQ ID NO: 16), 22Mono4DN4-VL (SEQ ID NO: 18) and 22Mono5UQY-VL (SEQ ID NO: 20).

In some embodiments, the humanized GPRC5D binding domain provided herein comprises a heavy chain variable domain sequence selected from the group consisting of SEQ ID NO: 15, SEQ ID NO: 17, SEQ ID NO: 19, and SEQ ID NO: 21; and/or a light chain variable domain sequence selected from the group consisting of SEQ ID NO: 14, SEQ ID NO: 16, SEQ ID NO: 18, and SEQ ID NO: 20.

These exemplary humanized GPRC5D binding domains retain the specific binding ability or affinity to GPRC5D, and and are at least comparable to or even superior to the parent mouse antibody ch-72C7 in this aspect. For example, data are provided in Examples 4 and 5.

In some embodiments, the GPRC5D binding domain in the polypeptide complex provided herein comprises all or part of the heavy chain variable domain and/or all or part of the light chain variable domain. In one embodiment, the GPRC5D binding domain in the polypeptide complex provided herein is a single domain antibody composed of all or part of the heavy chain variable domain provided herein. More information about such single domain antibodies is available in the art (see, for example, US Patent No. 6,248,516).

### Binding domain targeting immunostimulatory antigen

In some embodiments, the other one of the first antigen binding domain and the second antigen binding domain in the polypeptide complex described in the present application binds to an antigen other than GPRC5D. In some embodiments, the antigen other than GPRC5D is an immunostimulatory antigen.

Examples of antigen related to immune stimulation include, but are not limited to, CD2, CD3, CD7, CD16, CD27, CD30, CD70, CD83, CD28, CD80(B7-1), CD86(B7-2), CD40, CD40L(CD154), CD47, CD122, CD137, CD137L, OX40(CD134), OX40L(CD252), NKG2C, 4-1BB, LIGHT, PVRIG, SLAMF7, HVEM, BAFFR, ICAM-1, 2B4, LFA-1, GITR, ICOS(CD278), ICOSLG(CD275), LAG3(CD223), A2AR, B7-H3(CD276), B7-H4(VTCN1), BTLA(CD272), BTLA, CD160, CTLA-4(CD152), IDO1, IDO2, TDO, KIR, LAIR-1, NOX2, PD-1, PD-L1, PD-L2, TIM-3, VISTA, SIGLEC-7(CD328), TIGIT, PVR(CD155), TGFβ, SIGLEC9(CD329), or any combination thereof.

In some embodiments, the immunostimulatory antigen is CD3. In some embodiments, one of the first antigen binding domain and the second antigen binding domain binds to GPRC5D, and the other one binds to CD3.

In some embodiments, the antigen binding domain that binds to GPRC5D comprises any anti-GPRC5D antibody or anti-GPRC5D antigen binding fragment provided in the present application.

In some embodiments, the polypeptide complex comprises a CD3 binding domain. In some embodiments, the CD3 binding domain is derived from an antibody having a heavy chain variable region (V_{H}) as shown in SEQ ID NO: 55 and a light chain variable region (V_{L}) as shown in SEQ ID NO: 56.

In some specific embodiments, the CD3 binding domain has a CDR sequence or a VH/VL sequence as shown in Table 5 below.

**Table 6. Exemplary CD3 binding domain sequence**

| **Target** | | **CDR1** | **CDR2** | **CDR3** |
|---|---|---|---|---|
| CD3 | HCDR (Kabat) | SEQ ID NO: 49 TYAMN | SEQ ID NO: 50 RIRSKYNNYATY YADSVKG | SEQ ID NO: 51 HGNFGTSYVSW FAY |
| | HCDR (IMGT) | SEQ ID NO: 92 GFTFSTYA | SEQ ID NO: 93 IRSKYNNYAT | SEQ ID NO: 94 VRHGNFGTSYV SWFAY |
| | VH | SEQ ID NO: 55 EVQLVESGGGLVQPGGSLRLSCAASGFTFSTYAMNWVR QAPGKGLEWVGRIRSKYNNYATYYADSVKGRFTISRDDS KNSLYLQMNSLRAEDTAVYYCVRHGNFGTSYVSWFAY WGQGTLVTVSS | | |
| | LCDR (Kabat) | SEQ ID NO: 52 RSSTGAVTTSNY AN | SEQ ID NO: 53 GTNKRAP | SEQ ID NO: 54 ALWYSNLWV |
| | LCDR (IMGT) | SEQ ID NO: 95 TGAVTTSNY | SEQ ID NO: 96 GTN | SEQ ID NO: 54 ALWYSNLWV |
| | VL | SEQ ID NO: 56 QAVVTQEPSLTVSPGGTVTLTCRSSTGAVTTSNYANWVQ QKPGQAPRGLIGGTNKRAPWTPARFSGSLLGGKAALTLS GAQAEDEADYYCALWYSNLWVFGGGTKLTVL | | |

In some embodiments, the CD3 binding domain comprises three heavy chain complementarity determining regions HCDR1, HCDR2 and HCDR3, and three light chain complementarity determining regions LCDR1, LCDR2, and LCDR3. The three heavy chain complementarity determining regions are identical to heavy chain complementarity determining regions comprised in a heavy chain variable region (V_{H}) as shown in SEQ ID NO: 55, and the light chain complementarity determining regions are identical to light chain complementarity determining regions comprised in a light chain variable region (V_{L}) as shown in SEQ ID NO: 56.

In some embodiments, the CD3 binding domain comprises one or more (*e.g.* 1, 2, 3, 4, 5 or 6) CDRs. The one or more CDRs comprise a sequence selected from the group consisting of HCDR1 comprising an amino acid sequence as shown in SEQ ID NO: 49, HCDR2 comprising an amino acid sequence as shown in SEQ ID NO: 50, and HCDR3 comprising an amino acid sequence as shown in SEQ ID NO: 51, and/or LCDR1 comprising an amino acid sequence as shown in SEQ ID NO: 52, LCDR2 comprising an amino acid sequence as shown in SEQ ID NO: 53, and LCDR3 comprising an amino acid sequence as shown in SEQ ID NO: 54.

In some embodiments, the CD3 binding domain comprises one or more (*e.g.* 1, 2, 3, 4, 5 or 6) CDRs. The one or more CDRs comprise a sequence selected from the group consisting of HCDR1 comprising an amino acid sequence as shown in SEQ ID NO: 92, HCDR2 comprising an amino acid sequence as shown in SEQ ID NO: 93, and HCDR3 comprising an amino acid sequence as shown in SEQ ID NO: 94, and/or LCDR1 comprising an amino acid sequence as shown in SEQ ID NO: 95, LCDR2 comprising an amino acid sequence as shown in SEQ ID NO: 96, and LCDR3 comprising an amino acid sequence as shown in SEQ ID NO: 54.

In some embodiments, the CD3 binding domain comprises HCDR1 comprising an amino acid sequence as shown in SEQ ID NO: 49 or SEQ ID NO: 92, HCDR2 comprising an amino acid sequence as shown in SEQ ID NO: 50 or SEQ ID NO: 93, and HCDR3 comprising an amino acid sequence as shown in SEQ ID NO: 51 or SEQ ID NO: 94, and/or LCDR1 comprising an amino acid sequence as shown in SEQ ID NO: 52 or SEQ ID NO: 95, LCDR2 comprising an amino acid sequence as shown in SEQ ID NO: 53 or SEQ ID NO: 96, and LCDR3 comprising an amino acid sequence as shown in SEQ ID NO: 54.

### Binding domains targeting other tumor-associated antigens

In some embodiments, the polypeptide complex provided in the present application comprises a first antigen binding domain, a second antigen binding domain and a third antigen binding domain, and the third antigen binding domain further binds to a disease-associated antigen. In some embodiments, the disease-associated antigen is a tumor-associated antigen, an immune disease-associated antigen, or an inflammatory disease-associated antigen.

The tumor-associated antigen comprises, but is not limited to, CD19, CD20, CD38, CD30, Her2, CA125, MUC-1, prostate-specific membrane antigen (PSMA), CD44 surface adhesion molecule, mesothelin, carcinoembryonic antigen (CEA), epidermal growth factor receptor (EGFR), EGFRvIII, vascular endothelial growth factor receptor-2 (VEGFR2), high molecular weight melanoma associated antigen (HMW-MAA), MAGE-A1, IL-13R-a2, GD2, 4-1BB, 5T4, adenocarcinoma antigen, alpha-fetoprotein, BAFF, B lymphoma cells, C242 antigen, carbonic anhydrase 9 (CA-IX), C-MET, CCR4, CD152, CD19, CD200, CD22, CD221, CD23 (IgE receptor), CD28, CD30 (TNFRSF8), CD33, CD4, CD40, CD44 v6, CD51, CD52, CD56, CD74, CD80, CNTO888, CTLA-4, DRS, EGFR, EpCAM, CD3, FAP, fibronectin extra domain-B, folate receptor 1, GD2, GD3 ganglioside, glycoprotein 75, GPNMB, HGF, human scatter factor receptor kinase, IGF-1 receptor, IGF-I, IgG1, L1-CAM, IL-13, IL-6, insulin-like growth factor I receptor, integrin α5β1, integrin αvβ3, MORAb-009, MS4A1, MUC1, mucoprotein CanAg, N-hydroxyacetylneuraminic acid, NPC-1C, PDGF-Rα, PDL192, phosphatidylserine, prostate cancer cells, RANKL, RON, ROR1, SCH900105, SDC1, SLAMF7, TAG-72, tenascin-C, TGF β2, TGF-β, TRAIL-R1, TRAIL-R2, tumor antigen CTAA16.88, VEGF-A, VEGFR-1, VEGFR2, and vimentin.

In some embodiments, the tumor-associated antigen is Her2. In some embodiments, the polypeptide complex comprises a Her2 binding domain.

In some embodiments, the Her2 binding domain is derived from an antibody having a heavy chain variable region (V_{H}) as shown in SEQ ID NO: 63 and a a light chain variable region (V_{L}) as shown in SEQ ID NO: 64.

In some specific embodiments, the Her2 binding domain has a CDR sequence or a VH/VL sequence as shown in Table 5 below.

**Table 5. Exemplary Her2 binding domain**

| **Target** | | **CDR1** | **CDR2** | **CDR3** |
|---|---|---|---|---|
| Her2 | HCDR | SEQ ID NO: 57 GFNIKDTY | SEQ ID NO: 58 IYPTNGYT | SEQ ID NO: 59 SRWGGDGFYAM DY |
| | VH | SEQ ID NO: 63  | | |
| | LCDR | SEQ ID NO: 60 QDVNTA | SEQ ID NO: 61 SAS | SEQ ID NO: 62 QQHYTTPPT |
| | VL | SEQ ID NO: 64 DIQMTQSPSSLSASVGDRVTITCRASQDVNTAVAWYQQKP GKAPKLLIYSASFLYSGVPSRFSGSRSGTDFTLTISSLQPED FATYYCQQAYTTPPTFGQGTKVEIK | | |

In some embodiments, the Her2 binding domain comprises three heavy chain complementarity determining regions HCDR1, HCDR2 and HCDR3, and three light chain complementarity determining regions LCDR1, LCDR2, and LCDR3. The three heavy chain complementarity determining regions are identical to heavy chain complementarity determining regions comprised in a heavy chain variable region (V_{H}) as shown in SEQ ID NO: 63, and the light chain complementarity determining regions are identical to light chain complementarity determining regions comprised in a light chain variable region (V_{L}) as shown in SEQ ID NO: 64.

In some embodiments, the Her2 binding domain comprises one or more (*e.g.* 1, 2, 3, 4, 5 or 6) CDRs. The one or more CDRs comprise a sequence selected from the group consisting of HCDR1 comprising an amino acid sequence as shown in SEQ ID NO: 57, HCDR2 comprising an amino acid sequence as shown in SEQ ID NO: 58, and HCDR3 comprising an amino acid sequence as shown in SEQ ID NO: 59, and/or LCDR1 comprising an amino acid sequence as shown in SEQ ID NO: 60, LCDR2 comprising an amino acid sequence as shown in SEQ ID NO: 61, and LCDR3 comprising an amino acid sequence as shown in SEQ ID NO: 62.

### ii. Structure of polypeptide complex

The polypeptide complex provided in the present application can be in various suitable forms. In some embodiments, the polypeptide complex provided in the present application contains a DICAD domain.

### Polypeptide complex structure containing DICAD

In some embodiments, the first antigen domain and the second antigen domain in the polypeptide complex of the present application form a DICAD domain. As used herein, the full name of the term "DICAD" is "disulfide and charge adjusted diabody", which refers to a diabody structure with a covalent bond and electrostatic charge introduced at the VH-VL interface, as disclosed in PCT Application No. WO2019/120245, which is incorporated herein by reference in its entirety. This DICAD structure has the following advantages: (1) maintenance of the affinity, efficacy and other characteristics of each individual targeting domain; (2) high stability and less aggregation compared with other antibodies; and (3) easy expression and purification.

Specifically, the DICAD domain comprises: (i) a first polypeptide, comprising, in a N-terminal-to-C-terminal direction, a first light chain variable domain VL1 binding to a first antigen and a second heavy chain variable domain VH2 binding to a second antigen, where VL1 and VH2 are linked directly or by a first linker; and (ii) a second polypeptide, comprising, in a N-terminal-to-C-terminal direction, a second light chain variable domain VL2 binding to the second antigen and a first heavy chain variable domain VH1 binding to the first antigen, where VL2 and VH1 are linked directly or by a second linker. VL1 and VH1 are bound to form the first antigen binding domain, and VL2 and VH2 associate to form the second antigen binding domain.

In some embodiments, the C terminus of VL1 is linked to the N terminus of VH2, and the C terminus of VL2 is linked to the N terminus of VH1. In some embodiments, the C terminus of VH1 is linked to the N terminus of VL2, and the C terminus of VH2 is linked to the N terminus of VL1.

In some embodiments, VH2 and VL1 and/or VH1 and VL2 are covalently linked directly or indirectly (for example, by a linker, such as a peptide linker). In some embodiments, the linker comprises a peptide linker. As used herein, the term "peptide linker" may be any suitable polypeptide, which can bind to two entities to form a molecule, or maintain the association of two entities in close enough proximity, without substantial interference with their respective biological activities. The linker may consist of amino acid residues linked together by a peptide bond, and may optionally further contain one or more non-natural amino acids. Any suitable polypeptide can be used as a linker. In some embodiments, the linker can be essentially composed of amino acids that are not sterically hindered, such as glycine and alanine. In some embodiments, the linker is polyglycine, polyalanine, a combination of glycine and alanine (such as poly(Gly-Ala)) or a combination of glycine and serine (such as poly(Gly-Ser)). In some embodiments, the peptide linker comprises an amino acid sequence of RTVAA (SEQ ID NO: 74).

In some embodiments, VH1 is linked to VL2 by a first peptide linker, and VL1 is linked to VH2 by a second peptide linker. In some embodiments, the first peptide linker and the second peptide linker each independently comprise 5 to 9 amino acid residues. In some embodiments, the first peptide linker and the second peptide linker each independently comprise an amino acid sequence of RTVAA (SEQ ID NO: 74).

In some implementations, the DICAD may further comprise one or more modifications to promote the binding between the corresponding heavy chain and light chain, for example, the binding between VH1 and VL1 or between VH2 and VL2. In some embodiments, a non-natural covalent bond (such as non-natural disulfide bond) and/or electrostatic interaction can be introduced between the VH1-VL1 interaction interface and/or between the VH2-VL2 interaction interface.

### (a) Introduction of disulfide bond between VH/VL

In some embodiments, one of the first antigen binding domain and the second antigen binding domain in the polypeptide complex contains a first non-natural covalent bond, or alternatively, the other one does not comprise a non-natural covalent bond or comprises a second non-natural covalent bond different from the first non-natural covalent bond. For example, the second non-natural covalent bond is formed between two amino acid residues that are different from two amino acid residues forming the first non-natural covalent bond.

In some embodiments, the first non-natural covalent bond may be a non-natural disulfide bond. In some embodiments, the first non-natural disulfide bond is formed between two introduced cysteine residues. In such an embodiment, at least one of the first antigen binding domain and the second antigen binding domain is a disulfide stabilized Fv. The crystal structure analysis of the antibody shows that cysteine mutations can be introduced into some relatively conservative sequences at the VL-VH interface, thus forming a disulfide bond between VL and VH, which makes them covalently linked. The covalent bond between VL and VH significantly improves the stability of the antibody. The original dsFv (disulfide stabilizedd Fv) is constructed by introducing a disulfide bond at the VH-VL interface through the covalent interaction between cysteine residues in the CDR of each fragment (see Glockshuber, R. et al., A comparison of strategies to stabilize immunoglobulin Fv-fragments, (1990) Biochemistry, 291362-1367). Although the activity of an antibody will not be affected by this method, the "customized" design needs the detailed structural information of the CDR of the original antibody to avoid the interference with the antigen recognition/binding ability of the CDR. This makes this method difficult to be a general solution for constructing various antibodies. To ensure the wide use of this method, amino acids at selected sites in conserved FR is required to participate in the construction of dsFv.

Since 1993, several pairs of sites suitable for forming covalent bonds between VH-VL have been found, comprising VH44-VL100, VH105-VL43, VH100b-VL49, VH100-VL150 and VH101-VL46, etc., wherein the numbering is based on Kabat numbering (see Reiter,Y et al., Stabilization of the Fv fragments in recombinant immunotoxins by disulfide bonds engineered into conserved framework regions, (1994) Biochemistry, 335451-5459; Jung, S.H. et al., Design of interchain disulfide bond in the framework region of Fv fragment of monoclonal antibody B3, (1994) Protein, Structure, Function, and Gene, 19, 35-47; Glockshuber, R. et al., A comparison of strategies to stabilize immunoglobulin Fv-fragments, (1990) Biochemistry, 291362-1367; and Zhu, Z. et al., Remodeling domain interfaces to enhanceheterodimer formation, (1997) Protein Science, 6, 781-788) . VH44-VL100 and VH105-VL43 are better than other sites in many aspects (such as protein expression level, purity, Tm and affinity) to some degrees, and are thus subjected to broader application.

In some embodiments, one of the first antigen binding domain and the second antigen binding domain in the DICAD described in the present application comprises a first non-natural disulfide bond. In such an embodiment, the other one of the first antigen binding domain and the second antigen binding domain does not contain a disulfide bond, or comprises a second non-natural disulfide bond different from the first non-natural disulfide bond.

In some embodiments, the first antigen binding domain in the DICAD comprises a first non-natural disulfide bond, and VL1 and VH1 are covalently linked by a disulfide bond. In some embodiments, VL1 has a first cysteine substitution in the FR, VH1 has a second cysteine substitution in the FR, and the first cysteine and the second cysteine form a disulfide bond. In some embodiments, the first cysteine and the second cysteine are located in the FR2 region of VL1 and the FR4 region of VH1, respectively.

In some embodiments, the first cysteine and the second cysteine are respectively located at position 100 in VL1 and position 44 in VH1, position 43 in VL1 and position 105 in VH1, position 49 in VL1 and position 100 in VH1, position 50 in VL1 and position 100 in VH1, or position 46 in VL1 and position 101 in VH1, where the numbering is based on Kabat numbering. In some embodiments, the first cysteine and the second cysteine are selected from the group consisting of 100C in VL1 and 44C in VH1; 43C in VL1 and 105C in VH1; 49C in VL1 and 100bC in VH1; 50C in VL1 and 100C in VH1; or 46C in VL1 and 101C in VH1, where the numbering is based on Kabat numbering.

In some embodiments, the second antigen binding domain in the DICAD does not comprise any introduced non-natural disulfide bond. In some embodiments, the natural disulfide bond in the second antigen-binding domain in the DICAD is destroyed. In some other embodiments, the second antigen binding domain in the DICAD comprises a second non-natural disulfide bond different from the first non-natural disulfide bond, and VL2 and VH2 are covalently linked by the second non-natural disulfide bond. In some embodiments, the second non-natural disulfide bond is formed between two non-natural cysteine residues respectively in VL2 and VH2. In some embodiments, VL2 has a third cysteine substitution in the FR, VH2 has a fourth cysteine substitution in the FR, and the third cysteine and the fourth cysteine form a disulfide bond. In some embodiments, the third cysteine and the fourth cysteine are located in the FR2 region of VL2 and the FR4 region of VH2, respectively. In some embodiments, the third cysteine and the fourth cysteine are respectively located at position 100 in VL2 and position 44 in VH2, position 43 in VL2 and position 105 in VH2, position 49 in VL2 and position 100 in VH2, position 50 in VL2 and position 100 in VH2, or position 46 in VL2 and position 101 in VH2, where the numbering is based on Kabat numbering. In some embodiments, the third cysteine and the fourth cysteine are selected from the group consisting of 100C in VL2 and 44C in VH2; 43C in VL2 and 105C in VH2; 49C in VL2 and 100bC in VH2; 50C in VL2 and 100C in VH2; or 46C in VL2 and 101C in VH2, wherein the numbering is based on Kabat numbering.

In some specific embodiments, the first cysteine in VL1 is located at 100C in VL1, and the second cysteine in VH1 is located at 44C in VH1. In some embodiments, the disulfide bond is formed between 100C in VL1 and 44C in VH1. In some specific embodiments, the second antigen binding domain in the DICAD does not comprise a non-natural disulfide bond.

### (b) Substitution of charged amino acids in VH/VL

The strategy of enhancing the stable bonding of VH and VL in a bispecific antibody by introducing charged amino acids is well known in the art. Tan *et al.* tried to influence the stability of scFv (single-chain F variant) by adjusting amino acids on VH-VL interface based on its electrostatic properties (see Philip H. Tan et al., Contributions of a highly conserved VH/VL hydrogen bonding interaction to scFv folding stability and refolding efficiency, Biophysical Journal, September 1998; 75(3): 1473-1482). Later, Igawa *et al.* changed this method to improve scDb. Two pairs of Q39-Q38 in 4V fragment were replaced by amino acids with appropriate charges, respectively, to promote or inhibit the production of some isotypes so as improve the uniformity of the product (see Igawa T. et al., VH/VL interface engineering to promote selective expression and inhibit conformational isomerization of thrombopoietin receptor agonist single-chain diabody, Protein Engineering Design & Selection, Aug. 2010; 23(8) :667-77; and WO2006106905A1) .Gunasekaran and others from Amgen further studied this method and applied it to the improvement of the antibody Fab arm. The modifications to adjust the electrostatic interaction at the interface of CH1-CL and at 38-39 of VH-VL promoted the specific interaction between CH1-VH and between CL-VL (see Gunasekaran K et al., Enhancing antibody Fc heterodimer formation through electrostatic steering effects: applications to bispecific molecules and monovalent IgG, J Biol Chem., June 18, 2010, 285(25):19637-46; Liu Z et al., A Novel Antibody Engineering Strategy for Making Monovalent Bispecific Heterodimeric IgG Antibodies by Electrostatic Steering Mechanism, J Biol Chem., March 30, 2015; 290(12):7535-62.). By these methods, each VH of a bispecific antibody can interact with a corresponding VL, thus producing a bispecific antibody that can simultaneously bind to two antigens.

Therefore, in some embodiments, in the DICAD of the polypeptide complex provided herein, besides introducing a non-natural disulfide bond, the electrostatic steering of a specific region is also changed, whereby the undesired non-specific interaction is minimized. The modifications to introduce electrostatic interaction can improve the pharmacokinetic characteristics of the polypeptide complex, to eliminate the obstacles in the downstream development process, and increase the probability of successful development of the polypeptide complex in the present application.

In some embodiments, electrostatic interaction is introduced into the first antigen binding domain and/or the second antigen binding domain in the DICAD in the polypeptide complex provided in the present application, thereby promoting the pairing between VH1/VL1 and/or the pairing between VH2/VL2.

In some embodiments, two oppositely charged amino acid residues are introduced into VL1 and VH1 to promote the electrostatic interaction between VL1 and VH1. In some such embodiments, such oppositely charged amino acid residues are not introduced into VL2 and VH2.

In some other embodiments, two oppositely charged amino acid residues are introduced into VL2 and VH2 to promote the electrostatic interaction between VL2 and VH2. In some such embodiments, such oppositely charged amino acid residues are not introduced into VL1 and VH1.

In some other embodiments, the DICAD described in the present application is modified to introduce a first pair of oppositely charged amino acid residues that promote the electrostatic interaction between VL1 and VH1, and a second pair of oppositely charged amino acid residues that promote the electrostatic interaction between VL2 and VH2, thereby reducing mispairing between VH1 and VL2 and mispairing between VH2 and VL1 (for example, by electrostatic repulsion). For example, the charged residues introduced into VL2 and VH1 are amino acid residues with the same charge, and/or the charged residues introduced into VH2 and VL1 are amino acid residues with the same charge, thus reducing mispairing between VL2 and VH1 and mispairing between VL1 and VH2.

In some embodiments, the oppositely charged residues comprise a positively charged amino acid residue and a negatively charged amino acid residue.

In some embodiments, the first pair of oppositely charged amino acid residues comprises a negatively charged residue in VL1 and a positively charged residue in VH1. In some embodiments, the second pair of oppositely charged amino acid residues comprises a positively charged residue in VL1 and a negatively charged residue in VH1.

In some embodiments, the first pair of oppositely charged amino acid residues comprises a positively charged residue in VL1 and a negatively charged residue in VH1.

In some embodiments, the negatively charged amino acid can be selected from aspartate (D) or glutamate (E). In some embodiments, the positively charged amino acid can be selected from lysine (K), histidine (H) or arginine (R).

In some embodiments, the charged residue introduced in VL1 or VL2 is in FR (for example, FR2). In some embodiments, the charged residue introduced in VH1 or VH2 is in FR (for example, FR2).

In some embodiments, the first pair of oppositely charged amino acid residues is introduced into VL1 and VH1, to replace Q38 in VL1 and Q39 in VH1, Q40 in VL1 and Q39 in VH1, or Q37 in VL1 and Q39 in VH1, respectively, where the numbering is based on Kabat numbering.

In some embodiments, the second pair of oppositely charged amino acid residues is further introduced into VL2 and VH2, to replace Q40 in VL2 and Q39 in VH2, Q40 in VL2 and Q39 in VH2, or Q37 in VL2 and Q39 in VH2, respectively, where the numbering is based on Kabat numbering. In such an embodiment, the charged residues introduced into VL2 and VH1 are amino acid residues with the same charge, and/or the charged residues introduced into VH2 and VL1 are amino acid residues with the same charge, thus reducing mispairing between VL2 and VH1 and mispairing between VL1 and VH2.

In some specific embodiments, the first pair of oppositely charged amino acid residues comprises Q37K in VL1 and Q39D in VH1, respectively, where the numbering is based on Kabat numbering. In some specific embodiments, the second pair of oppositely charged amino acid residue comprises Q39D in VH2 and Q40K in VL2, respectively, where the numbering is based on Kabat numbering.

The introduction of a positively charged or a negatively charged amino acid into an antibody is known in the art.

In some embodiments, both a disulfide bond and charged amino acids are introduced between and into VH1 and VL1. In some embodiments, in the first antigen binding domain, the first cysteine introduced in VL1 and the second cysteine introduced in VH1 are selected from the group consisting of: 100C in VL1 and 44C in VH1; 43C in VL1 and 105C in VH1; 49C in VL1 and 100bC in VH1; 50C in VL1 and 100C in VH1; or 46C in VL1 and 101C in VH1; and the first pair of oppositely charged amino acid residues introduced replaces Q38 in VL1 and Q39 in VH1, Q40 in VL1 and Q39 in VH1, or Q37 in VL1 and Q39 in VH1, respectively, where the numbering is based on Kabat numbering.

### Polypeptide complex of structure A:

In some embodiments, the polypeptide complex according to the present application comprises a first antigen binding domain and a second antigen binding domain, where at least one of the first antigen binding domain and the second antigen binding domain binds to GPRC5D and comprises the GPRC5D binding domain provided in the present application.

In some embodiments, one of the first antigen binding domain and the second antigen binding domain in the present application binds to GPRC5D and comprises the GPRC5D binding domain provided in the present application, and the other one binds to an antigen other than GPRC5D. In some embodiments, the antigen other than GPRC5D is an immunostimulatory antigen, optionally where the immunostimulatory antigen is CD3.

In some embodiments, one of the first antigen binding domain and the second antigen binding domain comprises the GPRC5D binding domain provided in the present application, and the other one comprises a CD3 binding domain.

In some specific embodiments, the first antigen targeted by the first antigen binding domain in the DICAD is GPRC5D. In some embodiments, VL1 in the first antigen binding domain comprises mutations P100C and/or Q37K, and VH1 comprises mutations G44C and/or Q39D, where the numbering is based on Kabat numbering. In some embodiments, VL1 comprises an amino acid sequence as shown in SEQ ID NO: 103. In some embodiments, VH1 comprises an amino acid sequence as shown in SEQ ID NO: 104.

In some such embodiments, the second antigen targeted by the second antigen binding domain in the DICAD is CD3. In some embodiments, VL2 and VH2 in the second antigen binding domain comprise no mutations. In some embodiments, VL2 comprises an amino acid sequence as shown in SEQ ID NO: 56. In some embodiments, VH2 comprises an amino acid sequence as shown in SEQ ID NO: 108. In some specific embodiments, the amino acid sequence of the first polypeptide in the DICAD comprises an amino acid sequence as shown in SEQ ID NO: 22, and the amino acid sequence of the second polypeptide comprises an amino acid sequence as shown in SEQ ID NO: 23. In some embodiments, VL2 in the second antigen binding domain comprises mutation Q40K, and VH2 comprises mutation Q39D, where the numbering is based on Kabat numbering. In some embodiments, VL2 comprises an amino acid sequence as shown in SEQ ID NO: 106, and VH2 comprises an amino acid sequence as shown in SEQ ID NO: 107.

In some embodiments, the polypeptide complex of the present application further comprises an Fc polypeptide, which comprises a first Fc polypeptide and a second Fc polypeptide. In some embodiments, the first Fc polypeptide and the Fc polypeptide are the same or different. In some embodiments, the first Fc polypeptide and the second Fc polypeptide can be bound to form a dimer. In some embodiments, the second polypeptide of the polypeptide complex further comprises a first Fc polypeptide at the N- terminus. In some embodiments, the polypeptide complex further comprises a third polypeptide, and the N terminus of the third polypeptide comprises a second Fc polypeptide.

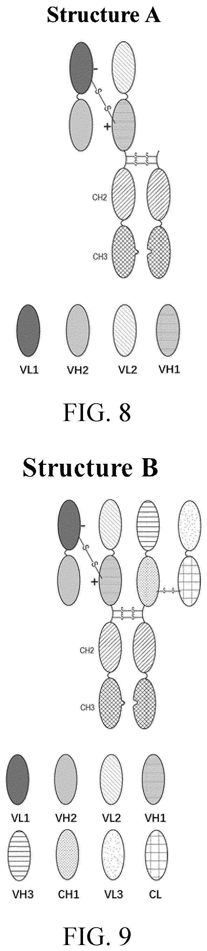

In some specific embodiments, the polypeptide complex has a structure as shown in Fig. 8.

In some embodiments, the first antigen targeted by the polypeptide complex is GPRC5D and the second antigen is CD3. In some specific embodiments, the polypeptide complex comprises a first polypeptide having an amino acid sequence as shown in SEQ ID NO: 22 and a second polypeptide having an amino acid sequence as shown in SEQ ID NO: 23. In some specific embodiments, the polypeptide complex further comprises a first Fc polypeptide and a second Fc polypeptide, and comprises a first polypeptide having an amino acid sequence as shown in SEQ ID NO: 22, a second polypeptide having an amino acid sequence as shown in SEQ ID NO: 75, and a third polypeptide having an amino acid sequence as shown in SEQ ID NO: 24.

### Polypeptide complex of structure B:

In some embodiments, the polypeptide complex comprises a first antigen binding domain and a second antigen binding domain, which together forms a DICAD domain provided in the present application, and further comprises a third antigen binding domain, where optionally, the third antigen binding domain comprises a Fab domain.

In some embodiments, the third antigen binding domain in the polypeptide complex is a Fab domain. The Fab domain comprises a third polypeptide, comprising, in a N-terminal-to-C-terminal direction, a third heavy chain variable domain VH3 binding to a third antigen and a CH1 domain; and a fourth polypeptide, comprising, in a N-terminal-to-C-terminal direction, a third light chain variable domain VL3 binding to the third antigen and a CL domain, where VL3 and VH3 are bonded to form the third antigen binding domain.

In some embodiments, the polypeptide complex of the present application further comprises an Fc polypeptide, which comprises a first Fc polypeptide and a second Fc polypeptide. In some embodiments, the first Fc polypeptide and the Fc polypeptide are the same or different. In some embodiments, the first Fc polypeptide and the second Fc polypeptide can be bound to form a dimer. In some embodiments, the second polypeptide of the polypeptide complex further comprises a first Fc polypeptide at the N- terminus. In some embodiments, the polypeptide complex further comprises a third polypeptide, and the N terminus of the third polypeptide comprises a second Fc polypeptide.

In some specific embodiments, the polypeptide complex has a structure as shown in Fig. 9.

In some embodiments, the first antigen targeted by the polypeptide complex is GPRC5D, the second antigen is GPRC5D, and the third antigen is CD3. In some embodiments, the first antigen targeted by the first antigen binding domain in the DICAD is GPRC5D. In some embodiments, VL1 in the first antigen binding domain comprises mutations P100C and Q37K, and VH1 comprises mutations G44C and Q39D, where the numbering is based on Kabat numbering. In some embodiments, VL1 comprises an amino acid sequence as shown in SEQ ID NO: 103. In some embodiments, VH1 comprises an amino acid sequence as shown in SEQ ID NO: 104.

In some specific embodiments, the second antigen targeted by the second antigen binding domain in the DICAD is also GPRC5D, where VH2 comprises no disulfide bond or charge mutation, and the numbering is based on Kabat numbering. In some embodiments, VL2 comprises an amino acid sequence as shown in SEQ ID NO: 12. In some embodiments, VH2 comprises an amino acid sequence as shown in SEQ ID NO: 105.

In some such embodiments, the third antigen targeted by the third antigen binding domain is CD3, and the third antigen binding domain comprises a Fab domain. In some such embodiments, VL3 in the third antigen binding domain comprises an amino acid sequence as shown in SEQ ID NO: 56. In some embodiments, VH3 comprises an amino acid sequence as shown in SEQ ID NO: 108.

In some such embodiments, the first polypeptide comprises an amino acid sequence as shown in SEQ ID NO: 33, the second polypeptide comprises an amino acid sequence as shown in SEQ ID NO: 34, the third polypeptide comprises an amino acid sequence as shown in SEQ ID NO: 35, and the fourth polypeptide comprises an amino acid sequence as shown in SEQ ID NO: 36. In some specific embodiments, the polypeptide complex further comprises a first Fc polypeptide and a second Fc polypeptide, and comprises an amino acid sequence as shown in SEQ ID NO: 33, SEQ ID NO: 80, SEQ ID NO: 81, and SEQ ID NO: 36.

In some embodiments, the first antigen targeted by the polypeptide complex is Her2, the second antigen is CD3, and the third antigen is GPRC5D. In some embodiments, one of the first antigen binding domain and the second antigen binding domain comprises the Her2 binding domain provided in the present application, and the other one comprises a CD3 binding domain.

In some embodiments, the first antigen targeted by the first antigen binding domain in the DICAD is Her2. In some embodiments, VL1 in the first antigen binding domain comprises mutations Q38D and Q100C, and VH1 comprises mutations Q39K and G44C. In some embodiments, VL1 comprises an amino acid sequence as shown in SEQ ID NO: 109. In some embodiments, VH1 comprises an amino acid sequence as shown in SEQ ID NO: 110.

In some such embodiments, the second antigen targeted by the second antigen binding domain in the DICAD is CD3, where VL2 comprises mutation Q40K, VH2 comprises mutation Q39D, and the numbering is based on Kabat numbering. In some embodiments, VL2 comprises an amino acid sequence as shown in SEQ ID NO: 106. In some embodiments, VH2 comprises an amino acid sequence as shown in SEQ ID NO: 107.

In some such embodiments, the third antigen targeted by the third antigen binding domain is GPRC5D, and the third antigen binding domain comprises a Fab domain. In some such embodiments, VL3 in the third antigen binding domain comprises an amino acid sequence as shown in SEQ ID NO: 12. In some embodiments, VH3 comprises an amino acid sequence as shown in SEQ ID NO: 105.

In some such embodiments, the first polypeptide comprises an amino acid sequence as shown in SEQ ID NO: 25, the second polypeptide comprises an amino acid sequence as shown in SEQ ID NO: 26, the third polypeptide comprises an amino acid sequence as shown in SEQ ID NO: 27, and the fourth polypeptide comprises an amino acid sequence as shown in SEQ ID NO: 28. In some specific embodiments, the polypeptide complex further comprises a first Fc polypeptide and a second Fc polypeptide and comprises a first polypeptide having an amino acid sequence as shown in SEQ ID NO: 25, a second polypeptide having an amino acid sequence as shown in SEQ ID NO: 76, a third polypeptide having an amino acid sequence as shown in SEQ ID NO: 77, and a fourth polypeptide having an amino acid sequence as shown in SEQ ID NO: 28.

In some embodiments, the first antigen targeted by the polypeptide complex is GPRC5D, the second antigen is CD3, and the third antigen is GPRC5D. In some specific embodiments, the first antigen targeted by the first antigen binding domain in the DICAD is GPRC5D, where VL1 comprises mutations P100C and Q37K, and VH1 comprises mutations G44C and Q39D. In some embodiments, VL1 comprises an amino acid sequence as shown in SEQ ID NO: 103. In some embodiments, VH1 comprises an amino acid sequence as shown in SEQ ID NO: 104.

In some such embodiments, the second antigen targeted by the second antigen binding domain in the DICAD is CD3, where VL2 comprises mutation Q40K, VH2 comprises mutation Q39D, and the numbering is based on Kabat numbering. In some embodiments, VL2 comprises an amino acid sequence as shown in SEQ ID NO: 106. In some embodiments, VH2 comprises an amino acid sequence as shown in SEQ ID NO: 107.

In some such embodiments, the third antigen targeted by the third antigen binding domain is GPRC5D, and the third antigen binding domain comprises a Fab domain. In some such embodiments, VL3 in the third antigen binding domain comprises an amino acid sequence as shown in SEQ ID NO: 12. In some embodiments, VH3 comprises an amino acid sequence as shown in SEQ ID NO: 105. In some such embodiments, the first polypeptide comprises an amino acid sequence as shown in SEQ ID NO: 29, the second polypeptide comprises an amino acid sequence as shown in SEQ ID NO: 30, the third polypeptide comprises an amino acid sequence as shown in SEQ ID NO: 31, and the fourth polypeptide comprises an amino acid sequence as shown in SEQ ID NO: 32. In some specific embodiments, the polypeptide complex further comprises a first Fc polypeptide and a second Fc polypeptide, and comprises a first polypeptide having an amino acid sequence as shown in SEQ ID NO: 29, a second polypeptide having an amino acid sequence as shown in SEQ ID NO: 78, a third polypeptide having an amino acid sequence as shown in SEQ ID NO: 79 and a fourth polypeptide having an amino acid sequence as shown in SEQ ID NO: 32.

### Polypeptide complex comprising at least two Fab domains: structure C

In some embodiments, the polypeptide complex comprises a first antigen binding domain and a second antigen binding domain. The first antigen binding domain comprises a first Fab domain and the second antigen binding domain comprises a second Fab domain.

In some embodiments, the first antigen binding domain comprises a first Fab domain, comprising a first polypeptide and a second polypeptide. The first polypeptide comprises, in a N-terminal-to-C-terminal direction, a first heavy chain variable domain VH1 binding to a first antigen and a first CH1 domain CH1a. The second polypeptide comprises, in a N-terminal-to-C-terminal direction, a first light chain variable domain VL1 binding to the first antigen and a first CL domain CLa. In some embodiments, the second antigen binding domain comprises a second Fab domain, comprising a third polypeptide and a fourth polypeptide. The third polypeptide comprises, in a N-terminal-to-C-terminal direction, a second heavy chain variable domain VH2 binding to a second antigen and a second CH1 domain CH1b. The fourth polypeptide comprises, in a N-terminal-to-C-terminal direction, a second light chain variable domain VL2 binding to the second antigen and a second CL domain CLb.

In some embodiments, the polypeptide complex of the present application further comprises an Fc polypeptide, which comprises a first Fc polypeptide and a second Fc polypeptide. In some embodiments, the first Fc polypeptide and the Fc polypeptide are the same or different. In some embodiments, the first Fc polypeptide and the second Fc polypeptide can be bound to form a dimer. In some embodiments, the second polypeptide of the polypeptide complex further comprises a first Fc polypeptide at the N- terminus. In some embodiments, the polypeptide complex further comprises a third polypeptide, and the N terminus of the third polypeptide comprises a second Fc polypeptide.

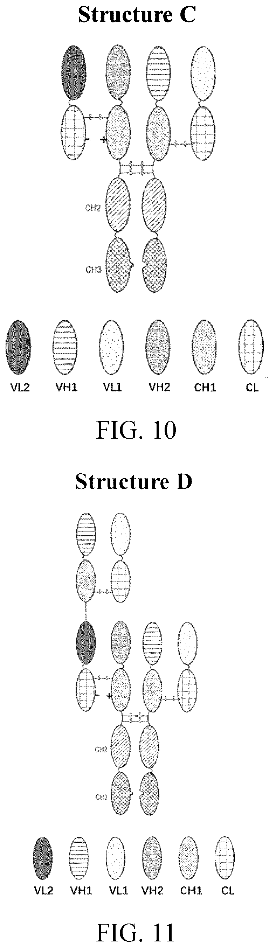

In some specific embodiments, the polypeptide complex has a structure as shown in Fig. 10.

In some embodiments, VL1 and VH1 are bound to form the first antigen binding domain, and VL2 and VH2 associate to form the second antigen binding domain.

In some embodiments, CH1a and CLa are paired, CH1b and CLb are paired, and the binding of CH1a and CLa and the binding of CH1b and CLb are configured to avoid mispairing between CH1a and CLb and/or mispairing between CH1b and CLa.

### (a) Introduction of disulfide bond between CH1/CL region

In some embodiments, at least one of the pairing of CH1b and CLb and the pairing of CH1a and CLa has at least one non-natural disulfide bond, which hinders mispairing between CH1b and CLa and/or mispairing between CH1a and CLb.

In some embodiments, a first CH1/CL pair and a second CH1/CL pair are selected from the pairing of CH1b and CLb and the pairing of CH1a and CLa, where the first CH1/CL pair is formed by a first disulfide bond. In some embodiments, the first disulfide bond is non-natural. In some embodiments, the originally naturally occurring disulfide bond in the first CH1/CL pair is deleted or destroyed (for example, by mutating cysteine residues forming a natural disulfide bond).

In some embodiments, the second CH1/CL pair is bound by a second disulfide bond, and the second disulfide bond is located at a different position from the first disulfide bond. In some embodiments, the second disulfide bond formed in the second CH1/CL pair is a natural disulfide bond or a non-natural disulfide bond.

In some embodiments, the first disulfide bond is formed by two cysteine residues introduced at positions (according to EU numbering) in the heavy chain-light chain selected from the group consisting of a) heavy chain position 134 and light chain position 116, b) heavy chain position 141 and light chain position 116, c) heavy chain position 128 and light chain position 118, d) heavy chain position 126 and light chain position 121, e) heavy chain position 127 and light chain position 121, f) heavy chain position 126 and light chain position 124, g) heavy chain position 170 and light chain position 162, h) heavy chain position 171 and light chain position 162, and i) heavy chain position 173 and light chain position 162.

In some embodiments, the first disulfide bond is formed by two cysteine residues introduced at positions (according to EU numbering) in the heavy chain-light chain selected from the group consisting of j) heavy chain position 133 and light chain position 209, k) heavy chain position 131 and light chain position 119, l) heavy chain position 133 and light chain position 207, m) heavy chain position 170 and light chain position 176, n) heavy chain position 173 and light chain position 160, o) heavy chain position 133 and light chain position 117, and p) heavy chain position 129 and light chain position 121.

In some embodiments, the first disulfide bond is formed by two cysteine residues introduced at positions (according to EU numbering) in the heavy chain-light chain selected from the group consisting of a) heavy chain position 126 and light chain position 121, b) heavy chain position 173 and light chain position 160, and c) heavy chain position 128 and light chain position 118.

In some embodiments, the natural disulfide bond is formed between a position selected from heavy chain positions 131, 219, and 220 and light chain position 214, where the numbering is based on EU numbering. In some embodiments, the natural disulfide bond is formed between heavy chain position 220 and light chain position 214, where the numbering is based on EU numbering.

In some embodiments, the first CH1/CL pair comprises modified CH1 and CL, in which position 126 in the CH1 is substituted into a cysteine residue, and position 220 in the CH1 is substituted into a non-cysteine residue; and position 121 in the CL is substituted into a cysteine residue, and position 214 in the CL is substituted into a non-cysteine residue, where the numbering is based on EU numbering.

### (b) Replacement of charged amino acids in CH1/CL region

In some embodiments, the pairing of CH1b/CLb has one or more introduced amino acid mutations and forms at least one introduced charged amino acid residue that hinders mispairing between CH1b and CLa and/or mispairing between CH1a and CLb.

In some embodiments, a first CH1/CL pair and a second CH1/CL pair are selected from the pairing of CH1b and CLb and the pairing of CH1a and CLa, where the first CH1/CL pair comprises at least one mutation from an uncharged amino acid residue to a charged amino acid residue, and/or at least one mutation from a charged amino acid residue to an uncharged amino acid residue, such that the first CH1/CL pair comprises a first pair of oppositely charged residues, and the first pair of oppositely charged residues promotes the pairing of the first CH1/CL pair. In some embodiments, the first CH1/CL pair may comprise a combination of mutations that provide a first pair of oppositely charged amino acid residues facilitating the pairing of the first CH1/CL pair.

In other words, a pair of residues with opposite charges can be introduced into the first CH1/CL pair to promote homologous pairing between the CH1 domain and the CL domain in the first CH1/CL pair. For example, a charged amino acid residue can be introduced to replace an uncharged amino acid residue at a certain position in CH1 (or CL), so that the introduced charged amino acid residue will form electrostatic interaction with another oppositely charged amino acid residue that exists or will be introduced into CL (or CH1), thus facilitating the pairing of the first CH1/CL pair. In another example, an existing charged amino acid residue at a certain position in CH1 (or CL) can be replaced by an oppositely charged amino acid residue, so that the charged amino acid residue after replacement will form electrostatic interaction with another oppositely charged amino acid residue that exists or will be introduced in CL (or CH1), thus facilitating the pairing of the first CH1/CL pair. In some embodiments, an existing charged amino acid residue in CH1 or CL can be replaced by an uncharged amino acid residue to reduce the potential interference of the electrostatic interaction between the first CH1/CL pair.

In some embodiments, the second CH1/CL pair comprises at least one mutation from an uncharged amino acid residue to a charged amino acid residue, and/or at least one mutation from a charged amino acid residue to an oppositely charged amino acid residue, such that the second CH1/CL pair comprises a second pair of oppositely charged amino acid residues that favors the pairing of the second CH1/CL pair, and optionally the first pair of oppositely charged amino acid residues and the second pair of oppositely charged amino acid residues hinder the pairing of CH1a and CLb or the pairing of CH1b and CLa.

In some embodiments, the first pair of oppositely charged amino acid residues and the second pair of oppositely charged amino acid residues are configured such that CH1a and CLb are both positively charged or negatively charged, and/or CH1b and CLa are both positively charged or negatively charged.

In some embodiments, the first pair of oppositely charged residues and/or the second pair of oppositely charged residues is introduced at a set of heavy chain-light chain (EU) positions selected from the group consisting of : a) heavy chain position 183 and light chain position 176, b) heavy chain position 183 and light chain position 133, c) heavy chain position 147 and light chain position 176, d) heavy chain position 141 and light chain position 116, e) heavy chain position 126 and light chain position 121, and f) heavy chain position 218 and light chain position 122.

In some embodiments, the first pair of oppositely charged amino acid residues and/or the second pair of oppositely charged amino acid residues is/are introduced at positions (according to EU numbering) in the heavy chain-light chain selected from the group consisting of: g) heavy chain position 147 and light chain position 131, h) heavy chain position 168 and light chain position 174, and i) heavy chain positions 147 and 168 and light chain positions 131 and 174.

In some embodiments, the pair of oppositely charged amino acid residues comprises a positively charged amino acid residue and a negatively charged amino acid residue, where the positively charged amino acid residue is selected from lysine (K), histidine (H) and arginine (R), and/or the negatively charged amino acid residue is selected from aspartate (D) and glutamate (E).

In some embodiments, lysine at position 147 in CH1b is replaced by a negatively charged amino acid residue; serine at position 176 in CLb is replaced by a positively charged amino acid residue; serine at position 183 in CH1a is replaced by a positively charged amino acid residue; and serine at position 176 in CLa is replaced by a negatively charged amino acid residue, where the numbering is based on EU numbering. In some embodiments, CH1b comprises mutation K147D; CLb comprises mutation S176K; CH1a comprises mutation S183K; and CLa comprises mutation S176D.

In some embodiments, lysine at position 147 in CH1a is replaced by a negatively charged amino acid residue; serine at position 176 in CLa is replaced by a positively charged amino acid residue; serine at position 183 in CH1b is replaced by a positively charged amino acid residue; and serine at position 176 in CLb is replaced by a negatively charged amino acid residue, where the numbering is based on EU numbering. In some embodiments, CH1a comprises mutation K147D; CLa comprises mutation S176K; CH1b comprises mutation S183K; and CLb comprises mutation S176D.

In some embodiments, the first CH1/CL pair comprises a combination of at least one non-natural disulfide bond and non-natural electrostatic interaction.

In some embodiments, the first CH1/CL pair is the pairing of CH1b and CLb. In some embodiments, the amino acid residue at position 173 in CH1b is replaced by cysteine, the amino acid residue at position 183 is replaced by a positively charged amino acid residue, and the amino acid residue at position 220 is replaced by a non-cysteine amino acid residue; and the amino acid residue at position 160 in CLb is replaced by cysteine, the amino acid residue at position 176 is replaced by a negatively charged amino acid residue, and the amino acid residue at position 214 is replaced by a non-cysteine amino acid residue, where the numbering is based on EU numbering. In some embodiments, CH1b comprises mutations V173C, S183K and C220S, and CLb comprises mutations Q160C, S176D and C214S.

In some embodiments, the first CH1/CL pair is the pairing of CH1b and CLb. In some embodiments, the amino acid residue at position 173 in CH1b is replaced by cysteine, the amino acid residue at position 183 is replaced by a negatively charged amino acid residue, and the amino acid residue at position 220 is replaced by a non-cysteine amino acid residue; and the amino acid residue at position 160 in CLb is replaced by cysteine, the amino acid residue at position 176 is replaced by a positively charged amino acid residue, and the amino acid residue at position 214 is replaced by a non-cysteine amino acid residue, where the numbering is based on EU numbering.

In some embodiments, the first CH1/CL pair comprises CH1a and CLa. In some embodiments, the amino acid residue at position 173 in CH1a is replaced by cysteine, the amino acid residue at position 183 is replaced by a positively charged amino acid residue, and the amino acid residue at position 220 is replaced by a non-cysteine amino acid residue; and the amino acid residue at position 160 in CLa is replaced by cysteine, the amino acid residue at position 176 is replaced by a negatively charged amino acid residue, and the amino acid residue at position 214 is replaced by a non-cysteine amino acid residue, where the numbering is based on EU numbering. In some embodiments, CH1a comprises mutations V173C, S183K and C220S, and CLa comprises mutations Q160C, S176D and C214S.

In some embodiments, the amino acid residue at position 173 in CH1a is replaced by cysteine, the amino acid residue at position 183 is replaced by a negatively charged amino acid residue, and the amino acid residue at position 220 is replaced by a non-cysteine amino acid residue; and the amino acid residue at position 160 in CLa is replaced by cysteine, the amino acid residue at position 176 is replaced by a positively charged amino acid residue, and the amino acid residue at position 214 is replaced by a non-cysteine amino acid residue, where the numbering is based on EU numbering.

In some embodiments, CH1 in the first CH1/CL pair comprises mutations A141K, V173C and C220S, and CL comprises mutations F116D, Q160C and C214S (the numbering is based on EU numbering).

In some embodiments, the first antigen targeted by the polypeptide complex is GPRC5D, and the second antigen is CD3. In some embodiments, the second CH1 domain CH1b in the third polypeptide comprises mutations A141K, V173C, and C220S, and the second CL domain CLb in the fourth polypeptide comprises mutations F116D, Q160C and C214S, where the numbering is based on EU numbering. In some embodiments, the second CH1 domain CH1b in the third polypeptide comprises an amino acid sequence as shown in SEQ ID NO: 112, and the second CL domain CLb in the fourth polypeptide comprises an amino acid sequence as shown in SEQ ID NO: 111.

In some embodiments, the first polypeptide comprises amino acid sequence as shown in SEQ ID NO: 39, the second polypeptide comprises an amino acid sequence as shown in SEQ ID NO: 40, the third polypeptide comprises an amino acid sequence as shown in SEQ ID NO: 38, and the fourth polypeptide comprises an amino acid sequence as shown in SEQ ID NO: 37.

In some specific embodiments, the polypeptide complex further comprises a first Fc polypeptide and a second Fc polypeptide, and comprises a first polypeptide having an amino acid sequence as shown in SEQ ID NO: 83, a second polypeptide having an amino acid sequence as shown in SEQ ID NO: 40, a third polypeptide having an amino acid sequence as shown in SEQ ID NO: 82, and a fourth polypeptide having an amino acid sequence as shown in SEQ ID NO: 37.

### (c) Formation of orthogonal interaction at CH1/CL interface

In some embodiments, at least one of CH1b/CLb pair and CH1a/CLa pair has one or more introduced amino acid mutations, to form an orthogonal CH1-CL interface, thus preventing mispairing between CH1b and CLa or mispairing between CH1a and CLb.

In some embodiments, the first CH1/CL pair and the second CH1/CL pair are selected from CH1b/CLb pair and CH1a/CLa pair, where the first CH1/CL pair comprises one or more introduced amino acid mutations, to form an orthogonal CH1-CL interface.

In some embodiments, the amino acid mutation forms an orthogonal CH1-CL interface, which facilitates the pairing of CH1b and CLb, and optionally prevents the pairing of CH1a with CLb or the pairing of CH1b with CLa. In some embodiments, the amino acid mutation forms an orthogonal CH1-CL interface, which facilitates the pairing of CH1a and CLa, and optionally prevents the pairing of CH1a with CLb or the pairing of CH1b with CLa.

In some embodiments, the orthogonal CH1-CL interface comprises mutations H168A and F170G in the heavy chain and mutations L135Y and S176W in the light chain. In some embodiments, the orthogonal CH1-CL interface comprises mutations H168A and F170G in the heavy chain and mutations L135Y and S176W in the light chain.

In some embodiments, CH1a comprises mutation S183E, CLa comprises mutation V133K, CH1b comprises mutations A141I, F170S, S181M, S183A and V185A, and CLb comprises mutations F116A, L235V, S174A, S176F and T178V.

In some embodiments, CH1b comprises mutation S183E, CLb comprises mutation V133K, CH1a comprises mutation A141I, F170S, S181M, S183A and V185A, and CLa comprises mutations F116A, L235V, S174A, S176F and T178V.

In some embodiments, the first CH1/CL pair comprises one or more introduced amino acid mutations at positions (according to EU numbering) in the heavy chain-light chain selected from the group consisting of A1411, F170S, S181M, S183A and V185A in the heavy chain, and F116A, A235V, S174A, S176F and T178V in the light chain.

In some embodiments, the first CH1/CL pair comprises one or more introduced amino acid mutations at positions (according to EU numbering) in the heavy chain-light chain selected from the group consisting of A1411, F170S, S181M, S183A and V185A in the heavy chain, and F116A, A235V, S174A, S176F and T178V in the light chain; and the second CH1/CL pair comprises an introduced amino acid mutation forming a pair of oppositely charged residues, comprising S183E in CH1 and V133K in CL.

In some embodiments, the polypeptide complex of the present application further comprises an Fc polypeptide, which comprises a first Fc polypeptide and a second Fc polypeptide. In some embodiments, the first Fc polypeptide and the Fc polypeptide are the same or different. In some embodiments, the first Fc polypeptide and the second Fc polypeptide can be bound to form a dimer. In some embodiments, the second polypeptide of the polypeptide complex further comprises a first Fc polypeptide at the N- terminus. In some embodiments, the polypeptide complex further comprises a third polypeptide, and the N terminus of the third polypeptide comprises a second Fc polypeptide.

### Polypeptide complex comprising at least two Fab domains: structure D

In some embodiments, the polypeptide complex further comprises a third antigen binding domain on the basis of structure C, and optionally the third antigen binding domain is a Fab domain. In some embodiments, the C terminus of the third antigen binding domain is linked to the N terminus of the second antigen binding domain.

In some embodiments, the third antigen binding domain is identical to the first antigen binding domain, and comprises a first fragment, comprising, in a N-terminal-to-C-terminal direction, a first heavy chain variable domain VH1 bonding to a first antigen and a first CH1 domain CH1a; and a second fragment, comprising, in a N-terminal-to-C-terminal direction, a first light chain variable domain VL1 bonding to the first antigen and a first CL domain CLa, where the C terminus of the first fragment is linked to the N terminus of the fourth polypeptide.

In some embodiments, the polypeptide complex comprises a first polypeptide, a second polypeptide, a third polypeptide, a fourth polypeptide, and a fifth polypeptide. In a N-terminal-to-C-terminal direction, the first polypeptide comprises VH1-CH1a; the third polypeptide comprises VH2-CH1b; the fourth polypeptide comprises VH1-CH1a-linker-VL2-CLb; and the second polypeptide and the fifth polypeptide are identical, and both comprise VL1-CLa.

In some embodiments, the pairing of CH1b and CLb has at least one non-natural disulfide bond, which hinders mispairing between CH1b and CLa and/or mispairing between CH1a and CLb.

In some embodiments, the pairing of CH1b and CLb has one or more introduced amino acid mutations and forms at least one introduced charged amino acid residue that hinders mispairing between CH1b and CLa and/or mispairing between CH1a and CLb.

In some embodiments, CH1 in the CH1/CL pair in the second Fab domain comprises mutations A141K, V173C and C220S, and CL comprises mutations F116D, Q160C and C214S (the numbering is based on EU numbering). In some specific embodiments, CH1 in the second Fab domain comprises an amino acid sequence as shown in SEQ ID NO: 112, and CL1 comprises an amino acid sequence as shown in SEQ ID NO: 111.

In some embodiments, the polypeptide complex of the present application further comprises an Fc polypeptide, which comprises a first Fc polypeptide and a second Fc polypeptide. In some embodiments, the first Fc polypeptide and the second Fc polypeptide are the same or different. In some embodiments, the first Fc polypeptide and the second Fc polypeptide can be bound to form a dimer. In some embodiments, the second polypeptide of the polypeptide complex further comprises a first Fc polypeptide at the N- terminus. In some embodiments, the polypeptide complex further comprises a third polypeptide, and the N terminus of the third polypeptide comprises a second Fc polypeptide.

In some specific embodiments, the polypeptide complex has a structure as shown in Fig. 11.

In some embodiments, the first antigen targeted by the polypeptide complex is GPRC5D, the second antigen is CD3, and the third antigen is GPRC5D. In some embodiments, CH1 in the CH1/CL pair in the second Fab domain comprises mutations A141K, V173C and C220S, and CL comprises mutations F116D, Q160C and C214S, where the numbering is based on EU numbering. In some embodiments, CH1 in the second Fab domain comprises an amino acid sequence as shown in SEQ ID NO: 112, and CL1 comprises an amino acid sequence as shown in SEQ ID NO: 111. In some embodiments, the first polypeptide comprises an amino acid sequence as shown in SEQ ID NO: 43, the second polypeptide comprises an amino acid sequence as shown in SEQ ID NO: 44, the third polypeptide comprises an amino acid sequence as shown in SEQ ID NO: 42, and the fourth polypeptide comprises an amino acid sequence as shown in SEQ ID NO: 41. In some specific embodiments, the polypeptide complex further comprises a first Fc polypeptide and a second Fc polypeptide, and comprises a first polypeptide having an amino acid sequence as shown in SEQ ID NO: 85, a second polypeptide or a fifth polypeptide having an amino acid sequence as shown in SEQ ID NO: 44, a third polypeptide having an amino acid sequence as shown in SEQ ID NO: 84, and a fourth polypeptide having an amino acid sequence as shown in SEQ ID NO: 41.

In some embodiments, the first antigen targeted by the polypeptide complex is GPRC5D, the second antigen is CD3, and the third antigen is GPRC5D. In some embodiments, the first antigen binding domain and/or the third antigen binding domain targeting GPRC5D is/are humanized. In some embodiments, CH1 in the CH1/CL pair in the second Fab domain comprises mutations A141K, V173C and C220S, and CL comprises mutations F116D, Q160C and C214S, where the numbering is based on EU numbering. In some embodiments, CH1 in the second Fab domain comprises an amino acid sequence as shown in SEQ ID NO: 112, and CL1 comprises an amino acid sequence as shown in SEQ ID NO: 111. In some embodiments, the first polypeptide comprises an amino acid sequence as shown in SEQ ID NO: 47, the second polypeptide comprises an amino acid sequence as shown in SEQ ID NO: 48, the third polypeptide comprises an amino acid sequence as shown in SEQ ID NO: 46, and the fourth polypeptide comprises an amino acid sequence as shown in SEQ ID NO: 45. In some specific embodiments, the polypeptide complex further comprises a first Fc polypeptide and a second Fc polypeptide, and comprises a first polypeptide having an amino acid sequence as shown in SEQ ID NO: 87, a second polypeptide or a fifth polypeptide having an amino acid sequence as shown in SEQ ID NO: 48, a third polypeptide having an amino acid sequence as shown in SEQ ID NO: 86, and a fourth polypeptide having an amino acid sequence as shown in SEQ ID NO: 45.

In some specific embodiments, the polypeptide complex has a specific sequence shown in Table 7.

**Table 7. Sequences of exemplary polypeptide complexes**

| **Name of polypeptide complex** | **Chain and SEQ ID NO** | **Sequence** | **Disulfide bond, charge mutation, and Fc mutation** |
|---|---|---|---|
| 22A1 (Structure A) | First polypeptide (SEQ ID NO: 22) | VL1(GPRC5D)-VH2(CD3) | VL1 (Q37K, P100C) |
| | | QIVLSQFPAIMSASPGEKVTMTCSASSS VSFMHWYQKKSGTSPKRWIYDTTKLA SGVPARFTGSGSGTSYSLTISSMEAEDA ATYYCQQWNSHPLTFGCGTKLELKRTV AAEVQLVESGGGLVQPGGSLRLSCAAS GFTFSTYAMNWVRQAPGKGLEWVGRI RSKYNNYATYYADSVKGRFTISRDDSK NSLYLQMNSLRAEDTAVYYCVRHGNFG NSYVSWFAYWGQGTLVTVSS | |
| | Second polypeptide (SEQ ID NO: 23) | VL2(CD3)-VH1(GPRC5D) | VH1 (Q39D, G44C) |
| | | QAVVTQEPSLTVSPGGTVTLTCRSSTGA VTTSNYANWVQQKPGQAPRGLIGGTN KRAPWTPARFSGSLLGGKAALTLSGAQ AEDEADYYCALWYSNLWVFGGGTKLT VLRTVAAQVQLQQPGAELVKPGTSVKL SCKASGYPFTNYWMHWVRDRPGQCLE WIGEINPSNGRTNYNEKFKSLATLTLDK SSSTAYMHLNSLTSEDSAVYYCARGFAY WGQGTLVTVSS | |
| 22A2 (Structure B) | First polypeptide (SEQ ID NO: 25) | VL1(Her2)-VH2(CD3) | VL1 (Q38D and Q100C) |
| | | DIQMTQSPSSLSASVGDRVTITCRASQD VNTAVAWYQDKPGKAPKLLIYSASFLYS GVPSRFSGSRSGTDFTLTISSLQPEDFAT YYCQQAYTTPPTFGCGTKVEIKRTVAAE VQLVESGGGLVQPGGSLRLSCAASGFTF STYAMNWVRDAPGKGLEWVGRIRSKY NNYATYYADSVKGRFTISRDDSKNSLYL QMNSLRAEDTAVYYCVRHGNFGNSYV SWFAYWGQGTLVTVSS | |
| | | | VH2 (Q39D) |
| | Second polypeptide (SEQ ID NO: 26) | VL2(CD3)-VH1(Her2) | VL2 (Q40K) |
| | | QAVVTQEPSLTVSPGGTVTLTCRSSTGA VTTSNYANWVQKKPGQAPRGLIGGTN KRAPWTPARFSGSLLGGKAALTLSGAQ AEDEADYYCALWYSNLWVFGGGTKLT VLRTVAAEVQLVESGGGLVQPGGSLRLS CAASGFNIKDTYIHWVRKAPGKCLEW VARIYPTNGYTRYADSVKGRFTISADTS KNTAYLQMNSLRAEDTAVYYCSRWGG DGFYAMDYWGQGTLVTVSS | VH1 (Q39K and G44C) |
| | Third polypeptide (SEQ ID NO: 27) | VH3(GPRC5D)-CH1 QVQLQQPGAELVKPGTSVKLSCKASGY PFTNYWMHWVRQRPGQGLEWIGEINPS NGRTNYNEKFKSLATLTLDKSSSTAYMH LNSLTSEDSAVYYCARGFAYWGQGTLV TVSSASTKGPSVFPLAPSSKSTSGGTAA LGCLVKDYFPEPVTVSWNSGALTSGVH TFPAVLQSSGLYSLSSVVTVPSSSLGTQT YICNVNHKPSNTKVDKKVEPKSC | / |
| | Fourth polypeptide (SEQ ID NO: 28) | VL3(GPRC5D)-CL | / |
| | | QIVLSQFPAIMSASPGEKVTMTCSASSS VSFMHWYQQKSGTSPKRWIYDTTKLAS GVPARFTGSGSGTSYSLTISSMEAEDAA TYYCQQWNSHPLTFGPGTKLELKRTVA APSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC | |
| 22A3 (Structure B) | First polypeptide (SEQ ID NO: 29) | VL1(GPRC5D)-VH2(CD3) | VL1 (P100C, Q37K) |
| | | QIVLSQFPAIMSASPGEKVTMTCSASSS VSFMHWYQKKSGTSPKRWIYDTTKLA SGVPARFTGSGSGTSYSLTISSMEAEDA ATYYCQQWNSHPLTFGCGTKLELKRTV AAEVQLVESGGGLVQPGGSLRLSCAAS GFTFSTYAMNWVRQAPGKGLEWVGRI RSKYNNYATYYADSVKGRFTISRDDSK NSLYLQMNSLRAEDTAVYYCVRHGNFG NSYVSWFAYWGQGTLVTVSS | |
| | Second polypeptide (SEQ ID NO: 30) | VL2(CD3)-VH1(GPRC5D) | VH1 (Q39D, G44C) |
| | | QAVVTQEPSLTVSPGGTVTLTCRSSTGA VTTSNYANWVQQKPGQAPRGLIGGTN KRAPWTPARFSGSLLGGKAALTLSGAQ AEDEADYYCALWYSNLWVFGGGTKLT VLRTVAAQVQLQQPGAELVKPGTSVKL SCKASGYPFTNYWMHWVRDRPGQCLE WIGEINPSNGRTNYNEKFKSLATLTLDK SSSTAYMHLNSLTSEDSAVYYCARGFAY WGQGTLVTVSS | |
| | Third polypeptide (SEQ ID NO: 31) | VH3(GPRC5D)-CH1 | / |
| | | QVQLQQPGAELVKPGTSVKLSCKASGY PFTNYWMHWVRQRPGQGLEWIGEINPS NGRTNYNEKFKSLATLTLDKSSSTAYMH LNSLTSEDSAVYYCARGFAYWGQGTLV TVSSASTKGPSVFPLAPSSKSTSGGTAA LGCLVKDYFPEPVTVSWNSGALTSGVH TFPAVLQSSGLYSLSSVVTVPSSSLGTQT YICNVNHKPSNTKVDKKVEPKSC | |
| | Fourth polypeptide (SEQ ID NO: 32) | VL3(GPRC5D)-CL | / |
| | | QIVLSQFPAIMSASPGEKVTMTCSASSS VSFMHWYQQKSGTSPKRWIYDTTKLAS GVPARFTGSGSGTSYSLTISSMEAEDAA TYYCQQWNSHPLTFGPGTKLELKRTVA APSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC | |
| 22A4 (Structure B) | First polypeptide (SEQ ID NO: 33) | VL1(GPRC5D)-VH2(GPRC5D) | VL1 (P100C, Q37K) |
| | | QIVLSQFPAIMSASPGEKVTMTCSASSS VSFMHWYQKKSGTSPKRWIYDTTKLA SGVPARFTGSGSGTSYSLTISSMEAEDA ATYYCQQWNSHPLTFGCGTKLELKRTV AAQVQLQQPGAELVKPGTSVKLSCKAS GYPFTNYWMHWVRQRPGQGLEWIGEI NPSNGRTNYNEKFKSLATLTLDKSSSTA YMHLNSLTSEDSAVYYCARGFAYWGQ GTLVTVSS | |
| | Second polypeptide (SEQ ID NO: 34) | VL2(GPRC5D)-VH1(GPRC5D) | VH1 (Q39D, G44C) |
| | | QIVLSQFPAIMSASPGEKVTMTCSASSS VSFMHWYQQKSGTSPKRWIYDTTKLAS GVPARFTGSGSGTSYSLTISSMEAEDAA TYYCQQWNSHPLTFGPGTKLELKRTVA AQVQLQQPGAELVKPGTSVKLSCKASG YPFTNYWMHWVRDRPGQCLEWIGEIN PSNGRTNYNEKFKSLATLTLDKSSSTAY MHLNSLTSEDSAVYYCARGFAYWGQGT LVTVSS | |
| | Third polypeptide (SEQ ID NO: 35) | VH3(CD3)-CH1 | / |
| | | EVQLVESGGGLVQPGGSLRLSCAASGFT FSTYAMNWVRQAPGKGLEWVGRIRSK YNNYATYYADSVKGRFTISRDDSKNSLY LQMNSLRAEDTAVYYCVRHGNFGNSY VSWFAYWGQGTLVTVSSASTKGPSVFP LAPSSKSTSGGTAALGCLVKDYFPEPVT VSWNSGALTSGVHTFPAVLQSSGLYSLS SVVTVPSSSLGTQTYICNVNHKPSNTKV DKKVEPKSC | |
| | Fourth polypeptide (SEQ ID NO: 36) | VL3(CD3)-CL | / |
| | | QAVVTQEPSLTVSPGGTVTLTCRSSTGA VTTSNYANWVQQKPGQAPRGLIGGTN KRAPWTPARFSGSLLGGKAALTLSGAQ AEDEADYYCALWYSNLWVFGGGTKLT VLRTVAAPSVFIFPPSDEQLKSGTASVVC LLNNFYPREAKVQWKVDNALQSGNSQ ESVTEQDSKDSTYSLSSTLTLSKADYEK HKVYACEVTHQGLSSPVTKSFNRGEC | |
| 22A5 (Structure C) | Fourth polypeptide (SEQ ID NO: 37) | VL2(CD3)-CLb | VL2 (Q40K) |
| | | QAVVTQEPSLTVSPGGTVTLTCRSSTGA VTTSNYANWVQKKPGQAPRGLIGGTN KRAPWTPARFSGSLLGGKAALTLSGAQ AEDEADYYCALWYSNLWVFGGGTKLT VLRTVAAPSVDIFPPSDEQLKSGTASVV CLLNNFYPREAKVQWKVDNALQSGNS CESVTEQDSKDSTYSLSSTLTLSKADYE KHKVYACEVTHQGLSSPVTKSFNRGES | CLb (F116D, Q160C and C214S) |
| | Third polypeptide (SEQ ID NO: 38) | VH2(CD3)-CH1b | VH2 (Q39D) |
| | | EVQLVESGGGLVQPGGSLRLSCAASGFT FSTYAMNWVRDAPGKGLEWVGRIRSK YNNYATYYADSVKGRFTISRDDSKNSLY LQMNSLRAEDTAVYYCVRHGNFGNSY VSWFAYWGQGTLVTVSSASTKGPSVFP LAPSSKSTSGGTAKLGCLVKDYFPEPVT VSWNSGALTSGVHTFPACLQSSGLYSLS SVVTVPSSSLGTQTYICNVNHKPSNTKV DKKVEPKSS | CH1b (A141K, V173C and C220S) |
| | First polypeptide (SEQ ID NO: 39) | VH1(GPRC5D)-CH1a | / |
| | | QVQLQQPGAELVKPGTSVKLSCKASGY PFTNYWMHWVRQRPGQGLEWIGEINPS NGRTNYNEKFKSLATLTLDKSSSTAYMH LNSLTSEDSAVYYCARGFAYWGQGTLV TVSSASTKGPSVFPLAPSSKSTSGGTAA LGCLVKDYFPEPVTVSWNSGALTSGVH TFPAVLQSSGLYSLSSVVTVPSSSLGTQT YICNVNHKPSNTKVDKKVEPKSC | |
| | Second polypeptide (SEQ ID NO: 40) | VL1(GPRC5D)-CLa | / |
| | | QIVLSQFPAIMSASPGEKVTMTCSASSS VSFMHWYQQKSGTSPKRWIYDTTKLAS GVPARFTGSGSGTSYSLTISSMEAEDAA TYYCQQWNSHPLTFGPGTKLELKRTVA APSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC | |
| 22A6 (Structure D) | Fourth polypeptide (SEQ ID NO: 41) | VH3(GPRC5D)-CH1a-linker-VL2(CD3)-CLb | VL2 (Q40K) |
| | | | CLb (F116D, Q160C and C214S) |
| | | QVQLQQPGAELVKPGTSVKLSCKASGY PFTNYWMHWVRQRPGQGLEWIGEINPS NGRTNYNEKFKSLATLTLDKSSSTAYMH LNSLTSEDSAVYYCARGFAYWGQGTLV TVSSASTKGPSVFPLAPSSKSTSGGTAA LGCLVKDYFPEPVTVSWNSGALTSGVH | |
| | Third polypeptide (SEQ ID NO: 42) | VH2(CD3)-CH1b | VH2 (Q39D) |
| | | EVQLVESGGGLVQPGGSLRLSCAASGFT FSTYAMNWVRDAPGKGLEWVGRIRSK YNNYATYYADSVKGRFTISRDDSKNSLY LQMNSLRAEDTAVYYCVRHGNFGNSY VSWFAYWGQGTLVTVSSASTKGPSVFP LAPSSKSTSGGTAKLGCLVKDYFPEPVT VSWNSGALTSGVHTFPACLQSSGLYSLS SVVTVPSSSLGTQTYICNVNHKPSNTKV DKKVEPKSS | CH1b (A141K, V173C and C220S) |
| | First polypeptide (SEQ ID NO: 43) | VH1(GPRC5D)-CH1a | / |
| | | QVQLQQPGAELVKPGTSVKLSCKASGY PFTNYWMHWVRQRPGQGLEWIGEINPS NGRTNYNEKFKSLATLTLDKSSSTAYMH LNSLTSEDSAVYYCARGFAYWGQGTLV TVSSASTKGPSVFPLAPSSKSTSGGTAA LGCLVKDYFPEPVTVSWNSGALTSGVH TFPAVLQSSGLYSLSSVVTVPSSSLGTQT YICNVNHKPSNTKVDKKVEPKSC | |
| | Second/fifth polypeptide (SEQ ID NO: 44) | VL1/VL3(GPRC5D)-CLa | / |
| | | QIVLSQFPAIMSASPGEKVTMTCSASSS VSFMHWYQQKSGTSPKRWIYDTTKLAS GVPARFTGSGSGTSYSLTISSMEAEDAA TYYCQQWNSHPLTFGPGTKLELKRTVA APSVFIFPPSDEQLKSGTASVVCLLNNFY PREAKVQWKVDNALQSGNSQESVTEQ DSKDSTYSLSSTLTLSKADYEKHKVYA CEVTHQGLSSPVTKSFNRGEC | |
| 22A8 (Structure D) | Fourth polypeptide (SEQ ID NO: 45) | VH3 (GPRC5D, humanized)-CH1a-linker-VL2(CD3)-CLb | CLb (F116D, Q160C and C214S) |
| | | QVQLVQSGAEVKKPGSSVKVSCRASGY PFTNYWMHWVRQAPGQGLEWIGEINP SNGRTNYNEKFKSRATLTLDKLTSTAYM ELSSLTSEDTALYYCARGFAYWGQGTLV TVSSASTKGPSVFPLAPSSKSTSGGTAA LGCLVKDYFPEPVTVSWNSGALTSGVH | |
| | | TFPAVLQSSGLYSLSSVVTVPSSSLGTQT YICNVNHKPSNTKVDKKVEPKSCDGGG GSGGGGSQAVVTQEPSLTVSPGGTVTLT CRSSTGAVTTSNYANWVQQKPGQAPRG LIGGTNKRAPWTPARFSGSLLGGKAALT LSGAQAEDEADYYCALWYSNLWVFGG GTKLTVLRTVAAPSVDIFPPSDEQLKSGT ASVVCLLNNFYPREAKVQWKVDNALQ SGNSCESVTEQDSKDSTYSLSSTLTLSK ADYEKHKVYACEVTHQGLSSPVTKSFN RGES | |
| | Third polypeptide (SEQ ID NO: 46) | VH2(CD3)-CH1b | CH1b (A141K, V173C and C220S) |
| | | EVQLVESGGGLVQPGGSLRLSCAASGFT FSTYAMNWVRQAPGKGLEWVGRIRSK YNNYATYYADSVKGRFTISRDDSKNSLY LQMNSLRAEDTAVYYCVRHGNFGTSY VSWFAYWGQGTLVTVSSASTKGPSVFP LAPSSKSTSGGTAKLGCLVKDYFPEPVT VSWNSGALTSGVHTFPACLQSSGLYSLS SVVTVPSSSLGTQTYICNVNHKPSNTKV DKKVEPKSS | |
| | First polypeptide (SEQ ID NO: 47) | VH1 (GPRC5D, humanized)-CH1a | / |
| | | QVQLVQSGAEVKKPGSSVKVSCRASGY PFTNYWMHWVRQAPGQGLEWIGEINP SNGRTNYNEKFKSRATLTLDKLTSTAYM ELSSLTSEDTALYYCARGFAYWGQGTLV TVSSASTKGPSVFPLAPSSKSTSGGTAA LGCLVKDYFPEPVTVSWNSGALTSGVH TFPAVLQSSGLYSLSSVVTVPSSSLGTQT YICNVNHKPSNTKVDKKVEPKSC | |
| | Second/fifth polypeptide( SEQ ID NO: 48) | VL1/VL3 (GPRC5D, humanized)-CLa | / |
| | | EIVLTQSPGTLSLSPGERATLSCSASSSVS FMHWYQQKPGQAPRRWIYDTTKLASGI PDRFSGSGSGTDYTLTISRLEPEDFAVYY CQQWNSHPLTFGQGTTVEIKRTVAAPSV FIFPPSDEQLKSGTASVVCLLNNFYPRE AKVQWKVDNALQSGNSQESVTEQDSK DSTYSLSSTLTLSKADYEKHKVYACEVT HQGLSSPVTKSFNRGEC | |

### Fc variants

In some embodiments, the first Fc polypeptide and/or the second Fc polypeptide is/are derived from IgG1, IgG2, IgG3 or IgG4.

In some embodiments, the polypeptide complex comprises one or more amino acid substitutions at the interface in the Fc region to promote and/or facilitate the dimerization. In some embodiments, the first Fc polypeptide and the second Fc polypeptide have different amino acid sequences and are at least configured to promote the heterodimerization of the first Fc polypeptide and the second Fc polypeptide. For example, a protuberance can be introduced into the first Fc polypeptide and a cavity can be introduced into the second Fc polypeptide, where the protuberance can be positioned in the cavity to promote the interaction between the first Fc polypeptide and the second Fc polypeptide to form a heterodimer or complex. Methods for producing antibodies with these modifications are known in the art, for example, as described in US Patent No. 5,731,168.

In some embodiments, in the polypeptide complex provided in the present application, one of the first Fc polypeptide and the second Fc polypeptide comprises a first Fc mutation, and the other one comprises a second Fc mutation. The first Fc mutation and the second Fc mutation comprise a) a combination of T366W or S354C with Y349C, T366S, L368A or Y407V; b) a combination of D399K or E356K with K392D or K409D; c) a combination of E356K, E357K or D399K with K370E, K409D or K439E; d) a combination of S364H or F405A with Y349T or T394F; e) a combination of S364H or T394F with Y394T or F405A; f) a combination of K370D or K409D with E357K or D399K; or g) a combination of L351D or L368E with L351K or T366K, where the amino acid position is based on EU numbering.

In some embodiments, the first Fc polypeptide comprises an amino acid sequence as shown in SEQ ID NO: 65 or SEQ ID NO: 67, and the second Fc polypeptide comprises an amino acid sequence as shown in SEQ ID NO: 66.

The polypeptide complex provided herein may be a monoclonal antibody, a polyclonal antibody, a humanized antibody, a chimeric antibody, a recombinant antibody, a labeled antibody, a divalent antibody or an anti-idiotypic antibody. The recombinant antibody is an antibody produced in vitro by a recombinant method rather than in animals.

### Antibody variants

The polypeptide complex provided herein also cover various antibody variants thereof.

In some embodiments, the antibody variants comprise one or more modifications or substitutions in one or more CDR sequences provided in Tables 1-2 and 5-6 above, one or more variable region sequences provided in Tables 3-6 above (but not in any CDR sequence) and/or constant regions (such as Fc regions). Such variants retain the binding specificity of their parent antibody to the corresponding antigens (for example, GPRC5D, CD3 or Her2), and have one or more desired characteristics endowed by one or more modifications or one or more substitutions. For example, the antibody variants may have increased antigen binding affinity, improved glycosylation pattern, reduced glycosylation risk, reduced deamination, reduced or depleted effector function(s), improved FcRn receptor binding, increased pharmacokinetic half-life, pH sensitivity and/or compatibility with binding *(e.g.,* one or more introduced cysteine residues).

Methods known in the art, such as "alanine scanning mutagenesis" (see, for example, Cunningham and Wells (1989) Science, 244:1081-1085), can be used to screen the parent antibody sequence to identify suitable or preferred residues for modification or substitution. Briefly, target residues (for example, charged residues such as Arg, Asp, His, Lys and Glu) can be recognized, and the target residues can be replaced by neutral or negatively charged amino acids (for example, alanine or polyalanine), to produce modified antibodies that are screened for characteristics of interest. If a substitution at a specific amino acid position shows a functional change of interest, the position can be identified as a potential residue for modification or substitution. The potential residues can be further evaluated by substitution with different types of residues (e.g. cysteine residues, and positively charged residues, etc.).

### Affinity variants

The affinity variants may comprise modifications or substitutions in one or more CDR sequences provided in Tables 1-2 and 5-6 above, one or more FR sequences provided herein, or a heavy chain or light chain variable region sequence provided in Tables 3-6 above. The FR sequences can be easily identified by those skilled in the art based on the CDR sequences in Tables 1-2 and 5-6 and the variable region sequences in Tables 3-6 above, because it is well known in the art that the CDR region is flanked by two FR regions in the variable region. The affinity variants retain the specific binding affinity of the parent antibody to the corresponding antigens (for example, GPRC5D, CD3 or Her2), or even have an improved specific binding affinity to the corresponding antigens (for example, GPRC5D, CD3 or Her2) superior to that of the parent antibody. In some embodiments, at least one (or all) substitution in the CDR sequence, FR sequence or variable region sequence comprises a conservative substitution.

It should be understood by those skilled in the art that in the CDR sequences and variable region sequences provided in Tables 1-6 above, one or more amino acid residues can be substituted, and the resulting polypeptide complex still retains the binding affinity or binding ability to the corresponding antigens (for example, GPRC5D, CD3 or Her2), or even has an improved binding affinity or ability. Various methods known in the art can be used to achieve this goal. For example, a library of antibody variants (such as Fab or scFv variants) can be produced and expressed by the phage display technology, and then screened for the binding affinity to the corresponding antigens (for example, GPRC5D, CD3 or Her2). For another example, computer software can be used to practically simulate the binding of an antibody to a corresponding antigen (for example, GPRC5D, CD3 or Her2), and to identify amino acid residues on the antibody forming a binding interface. Substitution of such residues can be avoided to prevent the decrease of the binding affinity, or such residues can be used as the target of substitution to achieve stronger binding.

In some embodiments, the humanized polypeptide complexes provided herein comprise one or more substitutions of amino acid residues in one or more CDR sequences and/or one or more FR sequences. In some embodiments, the affinity variants comprise not more than 20, 15, 10, 9, 8, 7, 6, 5, 4, 3, 2 or 1 substitution in total in the CDR sequence and/or FR sequence.

In some embodiments, the GPRC5D binding domain in the polypeptide complex comprises 1, 2 or 3 CDR sequences having at least 80% *(e.g.,* at least 85%, 88%, 90%, 91%, 92%, 93%, 94%, 95%, 96%, 97%, 98%, or 99%) sequence identity to (those) sequences listed in Table 1 or Table 2 above, and retains a GPRC5D binding affinity comparable to or even higher than that of the parent antibody. In some embodiments, the Her2 binding domain in the polypeptide complex comprises 1, 2 or 3 CDR sequences having at least 80% *(e.g.,* at least 85%, 88%, 90%, 91%, 92%, 93%, 94%, 95%, 96%, 97%, 98%, or 99%) sequence identity to (those) sequences listed in Table 5 above, and retains a Her2 binding affinity comparable to or even higher than that of the parent antibody. In some embodiments, the CD3 binding domain in the polypeptide complex comprises 1, 2 or 3 CDR sequences having at least 80% *(e.g.,* at least 85%, 88%, 90%, 91%, 92%, 93%, 94%, 95%, 96%, 97%, 98%, or 99%) sequence identity to (those) sequences listed in Table 6 above, and retains a CD3 binding affinity comparable to or even higher than that of the parent antibody.

In some embodiments, the GPRC5D binding domain in the polypeptide complex comprises one or more variable region sequences having at least 80% *(e.g.,* at least 85%, 88%, 90%, 91%, 92%, 93%, 94%, 95%, 96%, 97%, 98%, or 99%) sequence identity to (those) sequences listed in Tables 3-4 above, and retains a GPRC5D binding affinity comparable to or even higher than that of the parent antibody. In some embodiments, the variable region sequence listed in Table 3 above has 1 to 10 amino acid substitutions, insertions or deletions in total. In some embodiments, the substitution, insertion or deletion occurs in regions outside the CDR *(e.g.,* in FR). In some embodiments, the Her2 binding domain in the polypeptide complex comprises one or more variable region sequences having at least 80% (*e.g.,* at least 85%, 88%, 90%, 91%, 92%, 93%, 94%, 95%, 96%, 97%, 98%, or 99%) sequence identity to (those) sequences listed in Table 5 above, and retains a Her2 binding affinity comparable to or even higher than that of the parent antibody. In some embodiments, the variable region sequence listed in Table 5 above has 1 to 10 amino acid substitutions, insertions or deletions in total. In some embodiments, the substitution, insertion or deletion occurs in regions outside the CDR (*e.g.,* in FR). In some embodiments, the CD3 binding domain in the polypeptide complex comprises one or more variable region sequences having at least 80% (*e.g.,* at least 85%, 88%, 90%, 91%, 92%, 93%, 94%, 95%, 96%, 97%, 98%, or 99%) sequence identity to (those) sequences listed in Table 6 above, and retains a CD3 binding affinity comparable to or even higher than that of the parent antibody. In some embodiments, the variable region sequence listed in Table 6 above has 1 to 10 amino acid substitutions, insertions or deletions in total. In some embodiments, the substitution, insertion or deletion occurs in regions outside the CDR (*e.g.,* in FR).

### Glycosylation variants

The polypeptide complex provided herein also covers glycosylation variants, which can be obtained to increase or decrease the extent of glycosylation of the antibody or antigen binding fragment or polypeptide complex.

The polypeptide complex may comprise one or more modifications to introduce or remove the glycosylation sites. The glycosylation site is an amino acid residue having a side chain that can be linked to a carbohydrate moiety (such as an oligosaccharide structure). Glycosylation of the antibody is usually N-linked or O-linked. N-linked refers to the linkage of a carbohydrate moiety to the side chain of an asparagine residue (for example, the asparagine residue in a tripeptide sequence such as asparagine-X-serine and asparagine-X-threonine, where X is any amino acid except proline). O-linked glycosylation refers to the linkage of one of N-acetylgalactosamine, galactose or xylose to a hydroxyamino acid, most commonly to serine or threonine. The removal of a natural glycosylation site can be conveniently achieved, for example, by changing the amino acid sequence so that one of the tripeptide sequences described above (for N-linked glycosylation site) or the serine or threonine residue present in the sequence (for O-linked glycosylation site) is replaced. In a similar manner, a new glycosylation site can be produced by introducing such a tripeptide sequence or a serine or threonine residue.

In some embodiments, the polypeptide complex provided herein comprise mutations at N297 (e.g., N297A, N297Q or N297G) to remove the glycosylation site.

### Cysteine engineered variants

The polypeptide complex provided herein also cover a cysteine engineered variant, which comprises one or more introduced free cysteine residues.

The free cysteine residue is a cysteine residue that is not part of a disulfide bridge. The cysteine engineered variant can be bound to for example, a cytotoxic and/or imaging compound a label or a radioisotope at the engineered cysteine site, for example, by a maleimide or haloacetyl radical. Methods for engineering an antibody or an antigen binding fragment thereof to introduce a free cysteine residue are known in the art, see, for example, WO2006/034488.

### Fc variants

The polypeptide complex provided herein also covers an Fc variant, which comprise one or more modifications or substitutions of amino acid residues in the Fc region and/or hinge region, for example, to provide a changed effector function, such as ADCC and CDC. Methods of changing the ADCC activity by antibody engineering are described in the art, see, for example, Shields RL. et al., J Biol Chem. 2001. 276(9): 6591-604; Idusogie EE. et al., J Immunol. 2000.164(8):4178-84; Steurer W. et al., J Immunol. 1995, 155(3): 1165-74; Idusogie EE. et al., J Immunol. 2001, 166(4): 2571-5; Lazar GA. et al., Proc. Natl. Acad. USA, 2006, 103(11): 4005-4010; and Ryan MC. et al., Mol. Cancer Ther., 2007, 6: 3009-3018; Richards JO,. et al., Mol. Cancer Ther. 2008, 7(8): 2517-27; Shields R. L. et al., J. Biol. Chem, 2002, 277: 26733-26740; Shinkawa T. et al., J. Biol. Chem, 2003, 278: 3466-3473.

The CDC activity of the antibody provided herein can also be changed, for example, by improving or weakening the C1q binding and/or CDC (see, for example, WO99/51642; Duncan and Winter, Nature 322: 738-40 (1988); US Patent No. 5,648,260; US Patent No. 5,624,821); and for other examples of Fc region variants, see WO94/29351. One or more amino acids selected from amino acid residues 329, 331 and 322 in the Fc region can be substituted with different amino acid residues to change the Clq binding and/or reduce or eliminate the complement-dependent cytotoxicity (CDC) (see US Patent No. 6,194,551 to Idusogie et al.*).* One or more amino acid substitutions can also be introduced to change the antibody's ability to fix the complement (see PCT Publication No. WO 94/29351 to Bodmer et al.).

In some embodiments, the polypeptide complex provided herein has a reduced effector function and comprise one or more amino acid substitutions in IgG1 at positions selected from the group consisting of 234, 235, 237 and 238, 268, 297, 309, 330 and 331. In some embodiments, the polypeptide complex provided herein has an IgG1 isotypes and comprises one or more amino acid substitutions selected from the group consisting of N297A, N297Q, N297G, L235E, L234A, L235A, L234F, P331S and any combination thereof. In some embodiments, the polypeptide complex provided herein has an IgG2 isotypes, and comprises one or more amino acid substitutions selected from the group consisting of H268Q, V309L, A330S, P331S, V234A, G237A, P238S, H268A and any combination thereof (e.g. H268Q/V309L/A330S/P331S, V234A/G237A/P238S/H268A/V309L/A330S/P331S). In some embodiments, the polypeptide complex provided herein has an IgG4 isotypes and comprises one or more amino acid substitutions selected from the group consisting of N297A, N297Q, N297G, L235E, L234A, L235A, and any combination thereof. In some embodiments, the polypeptide complex provided herein has an IgG2/IgG4 cross-isootype. Examples of the IgG2/IgG4 cross-isotype are described in Rother RP et al., Nat Biotechnol, 25:1256-1264 (2007).

In some embodiments, the polypeptide complex provided herein has an IgG1 isotype and comprises one or more amino acid substitutions at one or more of sites 234, 235 and 331. In some embodiments, the polypeptide complex provided herein has an IgG1 isotype and comprise triple mutations L234F/L235E/P331S in the Fc region.

In some embodiments, the polypeptide complex provided herein has increased ADCC and/or increased affinity to Fcγ receptor, and comprises one or more amino acid substitutions at one or more of positions 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439 (see WO 00/42072 to Presta). Specific mutations at positions 256, 290, 298, 333, 334 and 339 have been shown to improve the binding to FcγRIII. In addition, the following combination of mutations are shown to improve the binding to FcyRIII: T256A/S298A, S298A/E333A, S298A/K224A, and S298A/E333A/K334A.

In some embodiments, the polypeptide complex comprises one or more amino acid substitutions that improve the pH-dependent binding to neonatal Fc receptor (FcRn). Such variants have an extended pharmacokinetic half-life because they bind to FcRn at an acidic pH to prevent them from being degraded in lysosomes, and then translocated and released from cells. Methods of engineering the polypeptide complexes to improve their binding affinity to FcRn are well known in the art, see, for example Vaughn, D. et al., Structure, 6(1): 63-73, 1998; Kontermann, R. et al., Antibody Engineering, Vol. 1, Chapter 27: Engineering of the Fc region for improved PK, Springer, 2010; Yeung, Y. et al., Cancer Research, 70: 3269-3277 (2010); and Hinton, P. et al., J Immunol., 176:346-356 (2006).

### Conjugates

In some embodiments, the polypeptide complex further comprises a conjugate moiety. The conjugate moiety may be linked to the polypeptide complex. The conjugate moiety is a moiety that can be linked to the polypeptide complex. It is considered that various conjugate moieties can be linked to the polypeptide complex provided herein (see, for example, "Conjugate Vaccines", Contributions to microbiology and immunology, J.M. Cruse and R.E.Lewis, Jr (ed.), Cage Press (New York) (1989)). These conjugation moieties can be linked to the polypeptide complex by covalent binding, affinity binding, intercalation, coordination, recombination, association, blending or addition, and other methods.

In some embodiments, the polypeptide complex disclosed herein can be engineered to contain specific sites other than the epitope conjugate moiety that can be used to bind to one or more conjugate moieties. For example, such sites may contain one or more reactive amino acid residues, such as, for example, cysteine or histidine residues, to facilitate the covalent attachment to the conjugate moiety.

In some embodiments, the antibody can be linked to the conjugate moiety indirectly or through another conjugate moiety. For example, the polypeptide complex can bind to biotin and then indirectly bind to a second conjugate that binds to avidin. The conjugate may be a clearance-modifying agent, a toxin (such as a chemotherapeutic agent), a detectable label (such as a radioisotope, lanthanide, a luminescent label, a fluorescent label or an enzyme-substrate label) or a purification moiety.

The "toxin" can be any agent that is harmful to cells or can destroy or kill cells. Examples of toxins comprise, but are not limited to, taxol, cytochalasin B, gramicidin D, ethidium bromide, emetine, mitomycin, etoposide, tenoposide, vincristine, MMAE, MMAF, DM1, vinblastine, colchicin, doxorubicin, daunorubicin, dihydroxy anthracin dione, mitoxantrone, mithramycin, actinomycin, 1-dehydrotestosterone, glucocorticoid, procaine, tetracaine, lidocaine, propranolol, puromycin and anologs thereof, antimetabolites (such as methotrexate, 6- mercaptopurine, 6-thioguanine, cytarabine, and 5-fluorouracil decarbazine), alkylating agents (for example, mechlorethamine, thioepa chlorambucil, melphalan, carmustine (BSNU) and lomustine (CCNU), cyclophosphamide, busulfan, dibromomannitol, streptozotocin, mitomycin C, cis-diamminedichloridoplatinum (II) (DDP), and cisplatin), anthracycline (such as daunomycin) and doxorubicin), antibiotics (such as dactinomycin (formly known as actinomycin)), bleomycin, mithramycin and anthramycin (AMC) ), antimitotic agents (such as vincristine and vinblastine), topoisomerase inhibitors, and tubulin-binding agents.

Examples of detectable labels may comprise fluorescent labels (*e.g.,* fluorescein, rhodamine, dansyl, phycoerythrin or Texas Red), enzyme-substrate labels (*e.g.,* horseradish peroxidase, alkaline phosphatase, luciferase, glucoamylase, lysozyme, glucose oxidase or β-D-galactosidase), radioactive isotopes (e.g., ¹²³I, ¹²⁴I, ¹²⁵I, ¹³¹I, ³⁵S, ³H, ¹¹¹In, ¹¹²In, ¹⁴C, ⁶⁴Cu, ⁶⁷Cu, ⁸⁶Y, ⁸⁸Y, ⁹⁰Y, ¹⁷⁷Lu, ²¹¹At, ¹⁸⁶Re, ¹⁸⁸Re, ¹⁵³Sm, ²¹²Bi, ³²P, and other lanthanides), luminescent labels, chromogenic moieties, digoxigenin, biotin/avidin, DNA molecules or gold labels for detection.

In some embodiments, the conjugate moiety can be a clearance modulator that helps to increase the half-life of the antibody. Illustrative examples comprise water-soluble polymers such as PEG, carboxymethyl cellulose, dextran, polyvinyl alcohol, polyvinylpyrrolidone, ethylene glycol/propylene glycol copolymers, and the like. The polymer may have any molecular weight and can be branched or non-branched. The number of polymer molecules linked to the antibody may vary, and if more than one polymer molecule is linked, they can be the same or different molecules.

In some embodiments, the conjugate moiety can be a purification moiety, such as magnetic beads.

In some embodiments, the polypeptide complex provided herein is used as the base of the conjugate.

### Polynucleotide and recombination method

The present application provides an isolated polynucleotide encoding the polypeptide complex. As used herein, the term "nucleic acid" or "polynucleotide" refers to a single-stranded or double-stranded deoxyribonucleic acid (DNA) or ribonucleic acid (RNA) and polymers thereof. In some embodiments, the isolated polynucleotide comprises one or more nucleotide sequences as shown in SEQ ID NOs: 11, 12, 13 and 14, and/or a homologous sequence with at least 80% (for example, at least 85%, 88%, 90%, 92%, 93%, 94%, 95%, 96%, 97%, 98% or 99%) sequence identity, and/or a variant thereof having only degenerate substitution; and encodes the variable regions of the exemplary antibodies provided herein. Unless otherwise indicated, a specific polynucleotide sequence also implicitly covers a conservatively modified variant thereof (for example, with degenerate codon substitution), an allele, an ortholog, SNP and a complementary sequence, as well as an explicitly indicated sequence. Specifically, degenerate codon substitution can be achieved by producing a sequence in which the third position of one or more selected (or all) codons is replaced by a mixed base and/or a deoxyinosine residue (see Batzer et al., Nucleic Acids Res., 19:5081 (1991); Ohtsuka et al., J. Biol. Chem., 260:2605-2608 (1985); and Rossolini et al., Mol. Cell. Probes, 8:91-98 (1994)).

DNA encoding a monoclonal antibody can be easily isolated and sequenced by using a conventional procedure (for example, by using an oligonucleotide probe that can specifically bind to a genes encoding the heavy chain and light chain of the antibody). The coding DNA can also be obtained by synthesis.

The isolated polynucleotide encoding the polypeptide complex (e.g., comprising the sequence shown in Table 3) can be inserted into a vector by a recombinant technique known in the art, for further cloning (DNA amplification) or expression. Many vectors can be used. The vector component generally comprises, but is not limited to, one or more of a signal sequence, a replication origin, one or more marker genes, an enhancer element, a promoter (such as SV40, CMV, and EF-1α) and a transcription termination sequence.

The present application provides an expression vector comprising the isolated polynucleotide provided herein. In some embodiments, the polynucleotide provided herein encodes the polypeptide complex, at least one promoter (e.g., SV40, CMV, and EF-1α) operably linked to a nucleic acid sequence, and at least one selection marker. Examples of vectors comprise, but are not limited to, retrovirus (comprising lentivirus), adenovirus, adeno-associated virus, herpes virus (such as herpes simplex virus), poxvirus, baculovirus, papillomavirus, papovavirus (such as SV40), λ phage and M13 phage, and plasmids pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, pEF-Bos, and others.

A vector comprising a polynucleotide sequence encoding the polypeptide complex can be introduced into a host cell for cloning or gene expression. Suitable host cells for cloning or expressing DNA in the vector herein are prokaryotes, yeasts or higher eukaryotes as described above. Suitable prokaryotes for this purpose comprise eubacteria, such as Gram-negative or Gram-positive organisms, such as *Enterobacteriaceae,* for example, *Escherichia,* like E. coli; *Enterobacter; Erwinia; Klebsiella; Proteus; Salmonella,* for example, *Salmonella typhimurium; Serratia,* for example, *Serratia marcescans;* and *Shigella* and *Bacilli,* for example, *B. subtilis* and *B. licheniformis; Pseudomonas,* for example, *P. aeruginosa;* and *Streptomyces.*

In addition to prokaryotes, eukaryotic microorganisms such as filamentous fungi or yeast are suitable cloning or expression hosts for antibody coding vectors. Saccharomyces cerevisiae or common baking yeast is the most commonly used lower eukaryotic host microorganisms. However, many other genera, species and strains are commonly used and useful herein, for example, *Schizosaccharomyces pombe; Kluyveromyces,* for example, *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans* and *K. marxianus; yarrowia* (EP 402,226); *Pichia pastoris* (EP *183,070); Candida; Trichoderma reesia* (EP 244,234); *Neurospora crassa; Schwanniomyces,* for example, *Schwanniomyces occidentalis;* and filamentous fungi, for example, *Neurospora, Penicillium, Tolypocladium* and *Aspergillus* hosts, such as *A. nidulans* and *A. niger.*

Suitable host cells expressing the glycosylated polypeptide complex provided herein are derived from multicellular organisms. Examples of invertebrate cells comprise plant and insect cells. A variety of baculovirus strains and variants, as well as corresponding allowable insect host cells from the following hosts are identified: *Spodoptera frugiperda* (caterpillar), *Aedes aegypti)* (mosquito), *Aedes albopictus* (mosquito), *Drosophila melanogaster* (fruit flies) and *Bombyx mori.* A variety of virus strains for transfection are publicly available, such as L-1 variant *of Autographa californica* NPV and Bm-5 virus strain of Bombyx mori NPV, and such viruses can be used as viruses herein according to the present invention, especially for transfection of Spodoptera frugiperda cells. Plant cell cultures of cotton, corn, potato, soybean, Petunia, tomato and tobacco can also be used as hosts.

However, vertebrate cells receive greatest interest, and the culture and proliferation of vertebrate cells (tissue culture) has become a routine procedure. Examples of useful mammalian host cell lines are the monkey kidney cell line CV1 (COS-7, ATCC CRL 1651) transformed by SV40; human embryonic kidney cell line (293 or 293 cells subcloned to grow in suspension culture); Graham et al., J. Gen Virol., 36:59 (1977)); baby hamster kidney cells (BHK, ATCC CCL 10); Chinese hamster ovary cells/-DHFR (CHO, Urlaub et al., Proc Natl Acad Sci USA, 77:4216 (1980)); mouse support cells (TM4, Mather, Biol. Reprod., 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human hepatocytes (Hep G2, HB 8065); mouse breast tumor cells (MMT 060562, ATCC CCL51); TRI cells (Mather et al., Annals N.Y. Acad. Sci., 383:44-68 (1982)); MRC 5 cells; FS4 cells; and human hepatic tumor cell line (Hep G2). In some preferred embodiments, the host cells are mammalian culture cell lines, such as CHO, BHK, NS0, 293 and their derivatives.

The host cells are transformed with the above expression or cloning vector for producing the polypeptide complex, and cultured in a conventional nutrient medium modified as needed to induce the promoter, select a transformant or amplify a gene encoding the desired sequence. In another embodiment, the antibody can be produced by homologous recombination known in the art. In some embodiments, the host cell can produce the polypeptide complex provided herein.

The host cell used to produce the polypeptide complex provided herein can be cultured in various media. Commercial media, such as Ham's F10 (Sigma), minimum essential medium (MEM) (Sigma), RPMI-1640 (Sigma) and Dulbecco's Modified Eagle's Medium (DMEM, Sigma) are suitable for culturing the host cells. In addition, any medium described in Ham et al., Meth. Enz., 58:44(1979); Barnes et al., Anal. Biochem.,102:255 (1980); US Patent Nos. 4,767,704, 4,657,866, 4,927,762, 4,560,655 or 5,122,469; WO90/03430; WO 87/00195; or US Reissue Patent No. 30,985 can be used as the culture medium for the host cells. Any of these media can be supplemented with a hormone and/or other growth factors (such as insulin, transferrin or epidermal growth factor), a salt (such as sodium chloride, a calcium salt, a magnesium salt and a phosphate), a buffer (such as HEPES), a nucleotide (such as adenosine and thymidine), an antibiotic (such as GENTAMYCIN^{™}), a trace element (defined as inorganic compounds that usually exist at a final concentration in the micromolar range), and glucose or an equivalent energy source as required. Any other necessary supplements may also be comprised in appropriate concentrations known to those skilled in the art. The culture conditions, such as temperature, and pH, etc., are those previously used for the expression of host cells, and will be obvious to those of ordinary skill in the art.

When the recombinant technology is used, the antibody can be produced in the periplasmic space in the cells, or secreted directly into the culture medium. If the antibody is produced in the cells, then the host cells or the particle debris of lyzed fragments are removed for example by centrifugation or ultrafiltration in a first step. Carter et al., Bio/Technology, 10: 163-167 (1992) describes a procedure for isolating an antibody secreted into the periplasmic space of Escherichia coli. Briefly, in the presence of sodium acetate (pH 3.5), EDTA and benzyl sulfonyl fluoride (PMSF), the cell paste is thawed in about 30 minutes. The cell debris can be removed by centrifugation. When an antibody is secreted into the culture medium, the supernatant from such expression systems is generally concentrated by using a commercially available protein concentration filter, such as Pellicon ultrafiltration unit from Amicon or Millipore. A protease inhibitor, such as PMSF, may be comprised in any of the aforementioned steps to inhibit the proteolysis, and antibiotics may be comprised to prevent the growth of foreign pollutants.

The polypeptide complex prepared from cells can be purified by, for example, hydroxyapatite chromatography, gel electrophoresis, dialysis, DEAE-cellulose ion exchange chromatography, precipitation with ammonium sulfate, salt precipitation and affinity chromatography, where the affinity chromatography is the preferred purification technique.

In some embodiments, protein A immobilized on the solid phase is used for immunoaffinity purification of the polypeptide complex. The suitability of protein A as an affinity ligand depends on the species and isotypes of any immunoglobulin Fc domain present in the antibody. Protein A can be used to purify antibodies based on human γ1, γ2 or γ4 heavy chain (Lindmark et al., J. Immunol. Meth., 62:1-13 (1983)). Protein G is recommended for all mouse isotypes and human γ3 (Guss et al., EMBO J., 5:1567 1575 (1986)). The matrix to which the affinity ligand is attached is most commonly agarose, but other matrices can also be used. Mechanically stable matrices, such as controlled-pore glass or poly(styrene-divinyl benzene) allow for faster flow rate and shorter treatment time than agarose. Bakerbond ABX^{™} resin (Phillipsburg, N.J., J. T. Baker) can be used for purification when the antibody comprises a CH3 domain. Other techniques for protein purification can also be used, such as fractionation on ion exchange column, ethanol precipitation, reversed-phase HPLC, chromatography on silica gel, chromatography on heparin SEPHAROSE^{™} chromatography on anion or cation exchange resin (such as poly-aspartate column), chromatofocusing, SDS-PAGE, and precipitation with ammonium sulfate, depending on the antibody to be recovered.

After any preliminary purification step, the mixture containing the antibody of interest and contaminants can be subjected to low-pH hydrophobic interaction chromatography using an elution buffer with a pH between about 2.5 and 4.5, preferably at a low salt concentration (e.g., about 0-0.25M salt).

### Pharmaceutical composition

The present application further provides a pharmaceutical composition comprising the polypeptide complex or the polynucleotide of the present application, and one or more pharmaceutically acceptable carriers.

The pharmaceutically acceptable carrier for use in the pharmaceutical composition disclosed herein may comprise, for example, a pharmaceutically acceptable liquid, a gel or a solid carrier, an aqueous vehicle, a non-aqueous vehicle, an antimicrobial agent, an isotonic agent, a buffer, an antioxidant, an anesthetic, a suspending/dispersing agent, a sequestering/chelating agent, a diluent, an adjuvant, an excipient, a non-toxic aid, and other components known in the art, or various combinations thereof.

Suitable components may comprise, for example, antioxidants, fillers, binders, disintegrants, buffers, preservatives, lubricants, flavoring agents, thickeners, colorants, emulsifiers or stabilizers such as sugars and cyclodextrins. Suitable antioxidant may comprise, for example, methionine, ascorbic acid, EDTA, sodium thiosulfate, platinum, catalase, citric acid, cysteine, thioglycerol, thioglycolic acid, thiosorbitol, butylated hydroxanisol, butylated hydroxytoluene and/or propyl gallate. As disclosed herein, the composition comprising the polypeptide complex and the conjugate as provided herein comprises one or more antioxidants, such as methionine, to reduce the oxidation of the polypeptide complex. This reduction in oxidation prevents or reduces the loss of binding affinity, thereby improving the antibody stability and maximizing the storage time. Therefore, in some embodiments, a composition comprising one or more polypeptide complexes as disclosed herein and one or more antioxidants such as methionine is provided. A method for preventing the oxidation, extending the storage time, and/or improving the efficacy of the polypeptide complex as provided herein by by mixing the polypeptide complex with one or more antioxidants such as methionine is also provided.

For further explanation, the pharmaceutically acceptable carrier may comprise, for example, an aqueous vehicle such as sodium chloride injection, Ringer's injection, isotonic dextrose injection, sterile water injection, or dextrose and lactate in Ringer's injection; a non-aqueous vehicle, such as a plant-derived non-volatile oil, cottonseed oil, corn oil, sesame oil or peanut oil; an antimicrobial agent that inhibits the concentration of bacteria or fungi; an isotonic agent, such as sodium chloride or dextrose; a buffer, such as a phosphate or citrate buffer; an antioxidant, such as sodium bisulfate; a local anesthetic, such as procaine hydrochloride; a suspending and dispersing agent, such as sodium carboxymethyl cellulose, hydroxypropyl methylcellulose or polyvinylpyrrolidone; an emulsifier, such as polysorbate 80 (TWEEN-80); a chelating agent, such as ethylenediamine tetraacetic acid (EDTA) or ethylene glycol tetraacetic acid (EGTA), ethanol, polyethylene glycol, propylene glycol, sodium hydroxide, hydrochloric acid, citric acid or lactic acid. The antimicrobial agent used as a carrier can be added to the pharmaceutical composition in a multi-dose container. The antimicrobial agent comprises phenol or cresol, a mercuric agent, benzyl alcohol, chlorobutanol, methyl and propyl p-hydroxybenzoate, thiomersal, benzalkonium chloride and benzethonium chloride. Suitable excipients may comprise, for example, water, physiological saline, dextrose, glycerol or ethanol. Suitable nontoxic aids may comprise, for example, a wetting agent or an emulsifier, a pH buffer, a stabilizer, a solubility enhancer or agents such as sodium acetate, sorbitan monolaurate, triethanolamine oleate or cyclodextrin.

The pharmaceutical composition can be in the form of a liquid solution, a suspension, an emulsion, pills, capsules, tablets, a sustained release preparation or a powder. An oral preparation can contain a standard carrier, such as pharmaceutical-grade mannitol, lactose, starch, magnesium stearate, polyvinylpyrrolidone, saccharin sodium, cellulose, and magnesium carbonate, etc.

In some embodiments, the pharmaceutical composition is prepared into an injectable composition. The injectable pharmaceutical composition can be prepared into any conventional form, such as, for example, a liquid solution, a suspension, an emulsion or a solid form suitable for producing a liquid solution, a suspension or an emulsion. Preparations for injection may contain sterile and/or pyrogen-free solutions that can be used for injection immediately; sterile dry soluble products that are prepared to combine with a solvent only before use, such as freeze-dried powder, comprising subcutaneous tablets; sterile suspension ready for injection; sterile dry insoluble products that are prepared to combine with a vehicle only before use; and sterile and/or pyrogen-free emulsions. The solution can be aqueous or nonaqueous.

In some embodiments, a unit dose of a parenteral preparation is packed in an ampoule, a vial or a syringe with a needle. All preparations for parenteral administration should be sterile and pyrogen-free, as known and practiced in the art.

In some embodiments, the sterile freeze-dried powder is prepared by dissolving the polypeptide complex as disclosed herein in a suitable solvent. The solvent may contain an excipient that improves the stability of the powder or a reconstitution solution prepared from the powder or other pharmacological components. Excipients that can be used comprise, but are not limited to, water, dextrose, sorbitol, fructose, corn syrup, xylitol, glycerol, glucose, sucrose or other suitable reagents. The solvent may contain a buffer, such as a nitrate, sodium phosphate or potassium phosphate, or other such buffers known to those skilled in the art. In one embodiment, the buffer is at about a neutral pH. Then the solution is sterilized and filtered under the standard conditions known to those skilled in the art, and then freeze-dried to obtain the required preparation. In one embodiment, the resulting solution is dispensed into vials for lyophilization. Each vial can contain a single dose or multiple doses of the polypeptide complex or its composition. It is acceptable that the vial is overfilled with a small amount (for example, about 10%) excess of the required single dose or a group of doses, so as to facilitate accurate sampling and accurate administration. The freeze-dried powder can be stored under appropriate conditions, such as at about 4°C to room temperature.

The freeze-dried powder reconstituted with water for injection provides a formulation for parenteral administration. In one embodiment, sterile and/or pyrogen-free water or other suitable liquid carriers are added to the lyophilized powder for reconstitution. The exact amount depends on the chosen therapy given and can be determined empirically.

### Method of use

The present application also provides a method for treating a GPRC5D-related disease or condition in a subject, which comprises administering, to the subject, a therapeutically effective amount of a polypeptide complex provided herein or a pharmaceutical composition provided herein.

In some embodiments, the GPRC5D-related disease or condition is characterized by the expression or overexpression of GPRC5D. The overexpression of GPRC5D has been confirmed in several autoimmune diseases, comprising myeloma.

In some embodiments, the GPRC5D-related disease or condition comprises, but is not limited to, cancers and other hyperproliferative diseases, immune diseases and inflammation.

The present application also provides a method for treating a Her2-related disease or condition in a subject, which comprises administering, to the subject, a therapeutically effective amount of a polypeptide complex provided herein or a pharmaceutical composition provided herein.

In some embodiments, the Her2-related disease or condition is characterized by the expression or overexpression of Her2. The overexpression of Her2 has been confirmed in several cancers, comprising breast cancer and lung cancer.

In some embodiments, the cancers and other hyperproliferative diseases comprise benign or malignant tumors, leukemia and malignant lymphoid tumors. Examples comprise neuron, glial cell, astrocyte, hypothalamus, glandular cell, macrophage, epithelial cell, endothelial cell, and interstitial malignant tumors, according to cell types with cancers or hyperproliferative diseases. Depending on the organ/site suffering from the cancer or hyperproliferative disease, examples comprise: head, neck, eyes, mouth, throat, esophagus, chest, skin, bone, lung, colon, rectum, colon, stomach, spleen, kidney, skeletal muscle, subcutaneous tissue, metastatic melanoma, endometrium, prostate, breast, ovary, testis, thyroid, blood, lymph node, kidney, liver, pancreas, brain or central nervous system etc.

In some embodiments, the immune diseases and/or inflammations comprise alopecia areata, ankylosing spondylitis, antiphospholipid syndrome, autoimmune Addison's disease, adrenal autoimmune diseases, autoimmune hemolytic anemia, autoimmune hepatitis, autoimmune oophoritis and orchitis, Sjogren's syndrome, psoriasis, atherosclerosis, diabetes and other retinopathy, retrolental fibroplasia, age-related macular degeneration, neovascular glaucoma, hemangioma, thyroid hyperplasia (comprising Graves' disease), corneal and other tissue transplantation, chronic inflammation, sepsis, rheumatoid arthritis, peritonitis, Crohn's disease, reperfusion injury, sepsis, endotoxic shock, cystic fibrosis, endocarditis, psoriasis, arthritis (such as psoriatic arthritis), anaphylactic shock, organ ischemia, reperfusion injury, spinal cord injury and allograft rejection, autoimmune thrombocytopenia, Behcet's disease, bullous pemphigoid, cardiomyopathy, dermatitis herpetiformis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, discoid lupus erythematosus, primary mixed cryoglobulinemia, fibromyalgia, fibromyositis, glomerulonephritis, Guillain-Barre syndrome, Hashimoto's thyroiditis, idiopathic pulmonary fibrosis, idiopathic thrombocytopenic purpura (ITP), IgA neuropathy, juvenile arthritis, lichen planus, lupus erythematosus, Meniere's disease, mixed connective tissue disease, multiple sclerosis, type 1 or immune-mediated diabetes, myasthenia gravis, pemphigus vulgaris, pernicious anemia, polyarthritis nodosa, hypertrichosis, multiple syndrome, polymyalgia rheumatica, polymyositis and dermatomyositis, primary agammaglobulinemia, primary biliary cirrhosis, psoriasis, psoriatic arthritis, Raynaud's phenomenon, Reiter's syndrome, rheumatoid arthritis, nodular disease, scleroderma, Sjogren's syndrome, stiff-person syndrome, systemic lupus erythematosus, lupus erythematosus, Takayasu's arteritis, temporal arteritis/giant cell arteritis, ulcerative colitis, uveitis, vasculitis, such as dermatitis herpetiformis-associated vasculitis, leukodermia and Wegener's granulomatosis. The inflammatory diseases may further comprise, but are not limited to, asthma, encephalitis, inflammatory bowel disease, chronic obstructive pulmonary disease (COPD), allergic diseases, septic shock, pulmonary fibrosis, undifferentiated spondyloarthritis, undifferentiated arthropathy, arthritis, inflammatory osteolysis, and chronic inflammation caused by chronic viral or bacterial infection.

In one embodiment, the GPRC5D-related disease or condition is cancers, especially multiple myeloma. In one embodiment, the GPRC5D-related disease or condition is an autoimmune disease, such as systemic lupus erythematosus and/or rheumatoid arthritis.

In some embodiments, the GPRC5D-related disease or condition is an GPRC5D expressing cancer. As used herein, the "GPRC5D expressing cancer" refers to any cancer or tumor that expresses GPRC5D on the surface of cancer cells. In some embodiments, the expression level of GPRC5D on cancer cells expressing GPRC5D is significantly higher than the expression level of GPRC5D on normal cells.

In some embodiments, the subject is identified as having cancer cells expressing GPRC5D. The presence and/or expression level of GPRC5D on cancer cells can be determined by various methods known in the art. A biological sample containing or suspected of containing cancer cells can be obtained from a subject. In some embodiments, the biological sample may be derived from cancer cells or cancer tissues. In some embodiments, the biological sample may be further treated to, for example, separate an analyte, such as a nucleic acid or protein. The presence and/or expression level of GPRC5D can be determined by, for example, quantitative fluorescence cell counting, immunohistochemistry (IHC), or nucleic acid-based methods. For example, a biological sample from a subject may be exposed to the polypeptide complex that binds to and detects the expressed GPRC5D protein. Alternatively, methods such as qPCR, reverse transcriptase PCR, microarray, SAGE, and FISH, etc. can also be used to detect GPRC5D at the nucleic acid expression level.

In one embodiment, the GPRC5D-related disease or condition comprises, but is not limited to, breast cancer, multiple myeloma, Waldenstrom's macroglobulinemia, endometrial cancer, ovarian cancer, lung cancer, gastric cancer, prostate cancer, renal cancer, liver cancer, pancreatic cancer, colorectal cancer, esophageal cancer, bladder cancer, cervical cancer, hematological cancer, lymphoma or malignant melanoma that express GPRC5D.

The therapeutically effective amount of the polypeptide complex or pharmaceutical composition as provided herein depends on various factors known in the art, such as the weight, age, past medical history, current medication, health status and possibility of cross-reaction, allergy, sensitivity and adverse side effects of the subjects, as well as the route of administration and the degree of disease development. As indicated by these and other situations or requirements, those skilled in the art (such as doctors or veterinarians) can reduce or increase the dosage proportionally.

In some embodiments, the polypeptide complex as provided herein can be administered at a therapeutically effective dose of about 0.01 mg/kg to about 100 mg/kg. In some embodiments, the dosage administered can be changed during the treatment. For example, in some embodiments, the initial dosage may be higher than the subsequent dosage administered. In some embodiments, depending on the response of the subject, the administered dosage may be changed during the treatment.

The dosage regimen can be adjusted to provide the optimal desired response (e.g., therapeutic response). For example, a single dose may be administered, or several divided doses may be administered over time.

The polypeptide complex disclosed herein can be administered through any route known in the art, such as, for example, parenteral (such as subcutaneous, intraperitoneal, intravenous (comprising intravenous infusion), intramuscular or intradermal injection) or non-parenteral (such as oral, nasal, intraocular, sublingual, rectal or topical) route.

In some embodiments, the antibody or antigen binding fragment thereof disclosed herein can be administered alone or in combination with one or more additional therapeutic means or agents. For example, the antibody or antigen binding fragment disclosed herein can be administered in combination with a second therapeutic agent *(e.g.,* chemotherapeutic agent, anti-cancer agent, radiotherapy, immunotherapy, anti-angiogenic agent, targeting therapy, cell therapy, gene therapy, hormone therapy, and palliative therapy), surgery for cancer treatment (e.g., tumor resection), or one or more treatments for complications caused by chemotherapy.

As used herein, the term "immunotherapy" refers to a type of therapy that stimulates the immune system to combat the diseases such as cancers or strengthens the immune system in a general way. The immunotherapy comprises passive immunotherapy. The passive immunotherapy is carried out by delivering drugs (e.g., effector cells) with definite tumor immunoreactivity, which can directly or indirectly mediate the anti-tumor effect without relying on the complete immune system in the host (e.g., antibody therapy or CAR-T cell therapy). The immunotherapy may further comprise active immunotherapy. The treatment relies on the in-vivo stimulation of the endogenous immune system in the host by administering an immune response modulator to fight against diseased cells.

In some of these embodiments, the polypeptide complex as disclosed herein administered in combination with one or more additional therapeutic agents can be administered simultaneously with the one or more additional therapeutic agents, and in some of these embodiments, the polypeptide complex and the additional therapeutic agent(s) can be administered as part of the same pharmaceutical composition. However, the polypeptide complex administered "in combination" with another therapeutic agent need not be administered with the agent simultaneously or in the same composition. The polypeptide complex administered before or after another agent is considered to be administered "in combination" with the agent, as the phrase is used herein, even if the polypeptide complex and the second agent are administered by different routes. When possible, according to the schedule listed in the product information form of an additional therapeutic agent or according to the Physicians'Desk Reference 2003 (Physicians'Desk Reference, Edition 57; Medical Economics Company; ISBN: 1563634457; Edition 57 (Nov. 2002)) or a protocol well known in the art, the additional therapeutic agent is administered in combination with the polypeptide complex disclosed herein.

In some embodiments, the present application provides a method for detecting the presence or level of GPRC5D in a sample, which comprises contacting the sample with the polypeptide complex provided herein.

In some embodiments, the present application provides a detection or therapeutic kit, which comprises the polypeptide complex provided herein and optionally instructions for use with a detectable moiety. The kit is suitable for detecting GPRC5D or for the treatment of GPRC5D-related diseases or conditions.

In some embodiments, the present application also provides use of the polypeptide complex provided herein in manufacturing a medicament for treating a GPRC5D-related disease or condition in a subject and for manufacturing a diagnostic reagent for diagnosing a GPRC5D-related disease or condition.

The following examples are provided to better illustrate the claimed invention and should not be construed as limiting the scope of the invention. The particular compositions, materials and methods described below totally or partly fall within the scope of the present invention. These specific compositions, materials and methods are not intended to limit the present invention, but used to illustrate specific embodiments that fall within the scope of the present invention. Those skilled in the art can develop equivalent compositions, materials and methods with no need of fulfilling the inventiveness, without departing from the scope of the invention. It should be understood that many changes can be made to the procedures described herein within the scope of the present invention. The present inventors anticipates that such variations are comprised in the scope of the present invention.

### Examples:

### Example 1: Preparation of GPRC5D monoclonal antibody

In this example, a tumor cell line expressing GPRC5D was used to immunize mice to prepare a monoclonal antibody.

### 1.1 Construction of 293T-GPRC5D and CHOS-GPRCSD cell lines

The human GPRC5D (hGPRC5D) was over-expressed in HEK293 cells (ATCC) and CHOS cells (Invitrogen) by lentivirus infection (MOI=3-10, 5 µg/ml polybrene). 72 hrs after cell infection, a corresponding antibiotic was added, and the cells were continuously cultured for 2-4 weeks. The cells were expanded and frozen to obtain 2 overexpression cell lines, HEK293-hGPRC5D and CHOS-hGPRC5D, which were used for subsequent immunological experiments.

### 1.2 Construction of control antibody GC5B596

The HC and LC plasmids of GC5B596 antibody were constructed with the following sequences, and then transferred into CHO cells and cultured. The supernatant was harvested and purified by affinity chromatography to obtain the control antibody GC5B596.

**Table 8**

| Antibody | Sequence (the CDR region is underlined) |
|---|---|
| GC5B596 | Heavy chain _HC |
| | QVQLVQSGAEVKKPGASVKVSCKASGYSFTGYTMNWVRQAPGQG LEWMGLINPYNSDTNYAQKLQGRVTMTTDTSTSTAYMELRSLRSDD TAVYYCARVALRVALDYWGQGTLVTVSSASTKGPSVFPLAPCSRSTS ESTAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVSN KGLPSSIEKTISKAKGQPREPQVYTLPPSQEEMTKNQVSLTCLVKGFY PSDIAVEWESNGQPENNYKTTPPVLDSDGSFFLYSRLTVDKSRWQEG NVFSCSVMHEALHNHYTQKSLSLSLGK (SEQ ID NO: 88) |
| | Light chain _LC |
| | DIQMTQSPSSLSASVGDRVTITCKASQNVATHVGWYQQKPGKAPKR LIYSASYRYSGVPSRFSGSGSGTEFTLTISNLQPEDFATYYCQQYNRY PYTFGQGTKLEIKRTVAAPSVFIFPPSDEQLKSGTASVVCLLNNFYPR EAKVQWKVDNALQSGNSQESVTEQDSKDSTYSLSSTLTLSKADYEK HKVYACEVTHQGLSSPVTKSFNRGEC (SEQ ID NO: 89) |

### 1.3 Mouse immunization/hybridoma fusion

To obtain an anti-human GPRC5D antibody, Balb/c mice (Beijing Weitong Lihua Laboratory Animal Technology Co., Ltd., strain code 216) were immunized with the constructed HEK293-GPRC5D cells overexpressing human GPRC5D. Complete Freund's adjuvant CFA (InvivoGen, article number vac-cfa-60) was used as an adjuvant in the primary immunization, and IFA (InvivoGen, article number vac-ifa-60) was used as an adjuvant in the subsequent immunization. The immunization route was subcutaneous multipoint. After multiple immunization, the spleen cells of immunized mice were fused with mouse myeloma cells SP2/0 by the polyethylene glycol method, and cultured in an HAT selective medium to obtain hybridoma cells that could express the antibody and proliferate indefinitely in vitro. The hybridoma cells were plated and cultured in a 96-well cell culture plate.

### 1.4 Cloning and screening of hybridoma

The antibody secreted by the hybridoma cells in the 96-well cell culture plate was tested for its binding ability to GPRC5D at a cell level. The cells highly expressing GPRC5D were cultured in a DMEM medium containing 10% FBS. The cells were digested with TrypLE, centrifuged and resuspended in a DPBS solution (FACS buffer, 4°C) containing 2% BSA. Then the cells were added to a 96-well plate with a U-shaped bottom in an amount of 5 × 10⁵ cells/50 µl and placed in a low-adsorption 96-well plate with a round bottom. 50 µl of mouse hybridoma supernatant was added and incubated at 4°C for 1 hr. After centrifugation, the supernatant was removed. The cells were washed twice with a FACS buffer, and the secondary antibody (DyLight488 goat anti-human IgG, Abcam, ab97003) was added to each well and incubated at 4°C for 0.5 hr. After centrifugation, the supernatant was removed. The cells were washed twice with a FACS buffer, and the FACS buffer was added to each well to resuspend the cells. The fluorescence of the cells in the test plate was measured by flow cytometer (BD, model CantoII) to determine the binding of the hybridoma supernatant and the cells. Meanwhile, HEK293, the background cell used for constructing the GPRC5D overexpression cells, was used for the same binding analysis. Using the criteria of positive binding to HEK293-hGPRC5D and negative binding to HEK293 cell, the GPRC5D binding positive clones were picked up and subcloned for 2-3 times.

### 1.5 Hybridoma sequencing/construction of recombinant expression vector

A ch-72C7 clone was obtained by screening. The picked hybridoma clone was sequenced according to the standard hybridoma sequencing method to obtain the heavy chain and light chain variable regions (VH and VL) of the picked clones. VH and VL were synthesized by whole gene synthesis and human IgG1 and kappa constant region were linked. The heavy chain and light chain sequences were ligated into the pcDNA3.4 vector, expressed transiently in the 293 system and purified by Protein A/G. The obtained chimeric recombinant antibody was ultrafiltered, and the buffer was replaced with a PBS solution. The sequencing results of ch-72C7 clone are shown in Table 9.

**Table 9**

| Clone | Sequence (the CDR region is underlined) |
|---|---|
| ch-72C7 | Heavy chain variable region_VH |
| | QIVLSQFPAIMSASPGEKVTMTCSASSSVSFMHWYQQKSGTSPKRW IYDTTKLASGVPARFTGSGSGTSYSLTISSMEAEDAATYYCQQWNS HPLTFGPGTKLELK(SEQ ID NO: 13) |
| | Light chain variable region _VL |
| | QVQLQQPGAELVKPGTSVKLSCKASGYPFTNYWMHWVRQRPGQ GLEWIGEINPSNGRTNYNEKFKSLATLTLDKSSSTAYMHLNSLTSED SAVYYCARGFAYWGQGTLVTVSA(SEQ ID NO: 12) |

### Example 2: FACS test for antigen binding

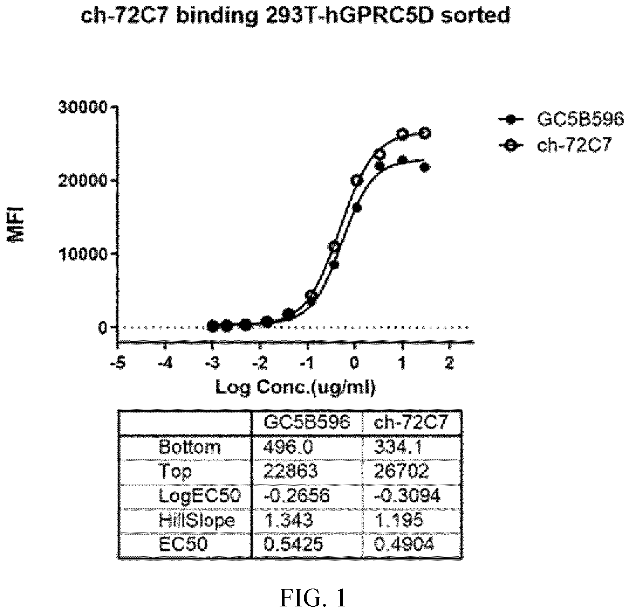

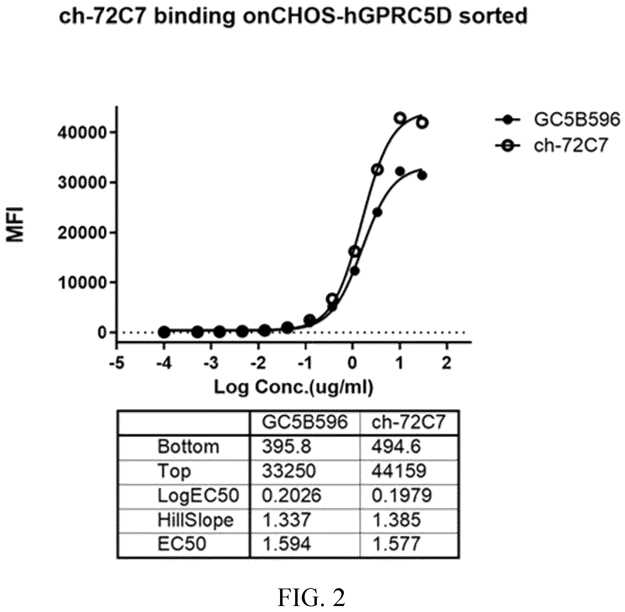

After centrifugation, HEK293-hCD22GPRC5D and CHOS-hGPRC5D cells expressing hGPRC5D were resuspended in a DPBS solution (FACS buffer, 4°C) containing 2% BSA, added to a 96-well plate with a U-shaped bottom in an amount of 5 × 10⁵ cells/100µl/well, added with antibody diluted over concentration gradient, and incubated at 4°C for 1 hr. After centrifugation, the supernatant was discarded. 100 µl of anti-human IgG Fc-APC secondary antibody was added to each well and incubated at 4°C for 1 hr. Then the cells were washed once with a FACS buffer, and resuspended in 200 µl FACS buffer. The fluorescence signal was read on BD CantoII. The results show that ch-72C7 antibody binds to HEK293-hGPRC5D (Fig. 1) and CHOS-hGPRC5D (Fig. 2).

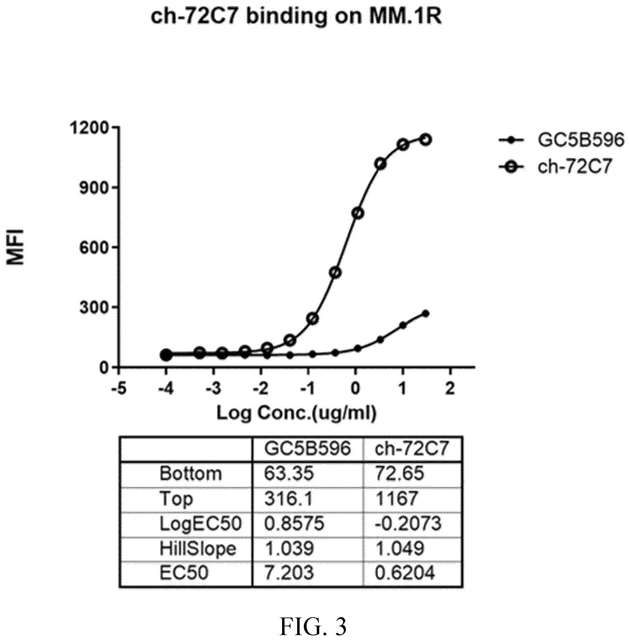

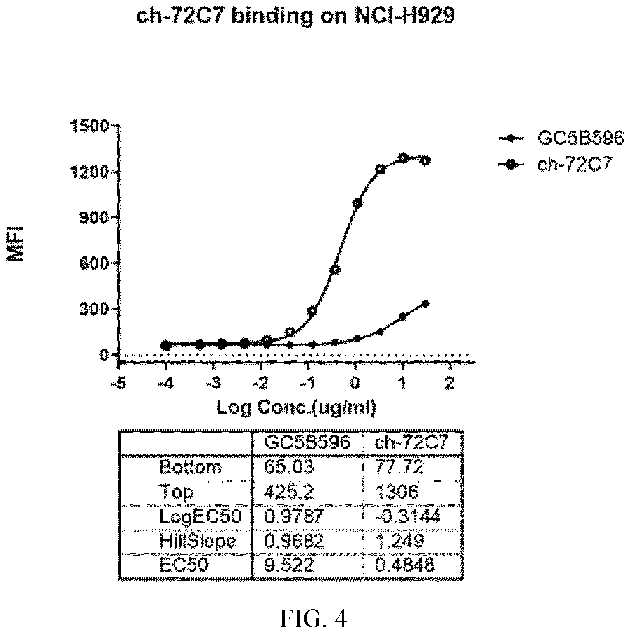

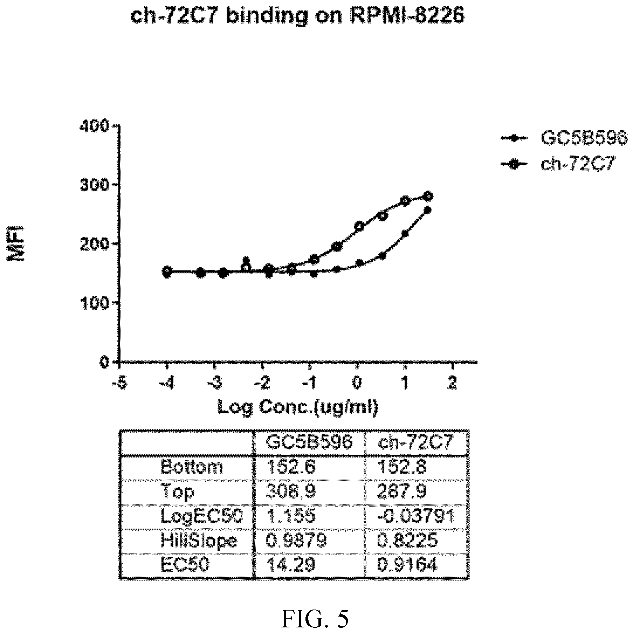

MM.1R cells, NCI-H929 cells and RPMI-8226 cells (highly expressing GPRC5D; ATCC, CL-188) were cultured in RPMI 1640 medium containing 10% FBS. After the cells were digested with TrypLE, the digested cells were centrifuged and resuspended in a DPBS solution containing 2% BSA (FACS buffer, 4°C), added into a 96-well plate with a U-shaped bottom in an amount of 5 × 10⁵ cells/100 µl/well, added with antibody diluted over concentration gradient, and incubated at 4°C for 1 hr. After centrifugation, the supernatant was discarded. 100 µl of anti-human IgG Fc-APC secondary antibody was added to each well and incubated at 4°C for 1 hr. Then the cells were washed once with a FACS buffer, and resuspended in 200 µl FACS buffer. The fluorescence signal was read on BD CantoII. The results show that the antibody ch-72C7 binds to MM.1R cells (Fig. 3), NCI-H929 cells (Fig. 4) and RPMI-8226 cells (Fig. 5).

**Table 10**

| Antibody | MM.1R cell binding EC50 (µg/ml) | NCI-H929 cell binding EC50 (µg/ml) | RPMI-8226 cell binding EC50 (µg/ml) |
|---|---|---|---|
| ch-72C7 | 0.620 | 0.485 | 0.916 |
| GC5B596 | 7.203 | 9.522 | 14.290 |

### Example 3: Evaluation of ADCC effect of antibodies

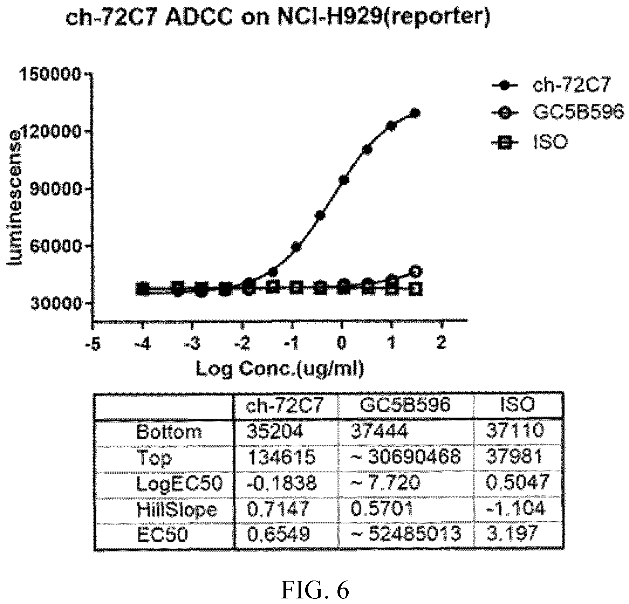

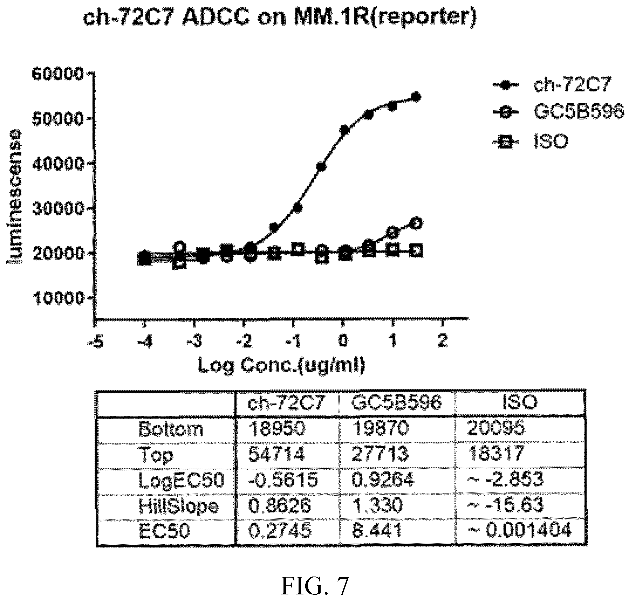

The target cells were tumor cells naturally expressing GPRC5D (NCI-H929, Nanjing Cobioer; MM.1R, Nanjing Cobioer), the effector cells were Jurkat-NFAT-Luc-CD16 cell line stably transfected with CD16 receptor and NFAT reaction element constructed by in-house, and the experiment was carried out in a 96-well flat-bottomed cell plate (Corning 3903). The antibody diluted over gradient was added to the target cells and incubated at 37°C for 30 min. 60,000 effector cells were added to every 10,000 target cells, and the reaction was carried out at 37°C for 6 hrs. After the reaction, One-Glo^{™} reagent (Promega, E6110) was added for fluorescent development, and the luminescence reading of the cell plate was measured on Tecan Spark10 plate reader. GraphPad was used for data analysis, the horizontal axis represents the logarithm of the antibody concentration, and the vertical axis represents the luminescence reading of the corresponding well. The EC50 of the antibody-dependent cytotoxicity of the anti-GPRC5D antibody was fitted from the curve. The results show that the blank control Isotype (ISO) has no cytotoxic effect on NCI-H929 cells and MM.1R cells. Both the ch-72C7 antibody and the CG5B596 antibody have cytotoxic effects on tumor cells NCI-H929 (Fig. 6) and RPMI-8226 (Fig. 7) that naturally express GPRC5D, and compared with the CG5B596 antibody, the ch-72C7 antibody shows better ADCC effect.

### Example 4: Design and expression of humanized antibodies

The ch-72C7 antibody was compared with IMGT database, a human framework sequence with the highest homology to VH/VL was selected, and CDR graft was carried out. Computational chemical simulation was carried out to maintain its binding with antigen. The humanized antibodies designed have been shown in Table 11.

**Table 11**

| Humanized antibody | Sequence (the CDR region is underlined) |
|---|---|
| 22Mono5JO4 | Heavy chain variable region_VH |
| | QVQLVQSGAEVKKPGSSVKVSCRASGYPFTNYWMHWVRQAPGQ GLEWIGEINPSNGRTNYNEKFKSRATLTLDKLTSTAYMELSSLTSED TALYYCARGFAYWGQGTLVTVSS (SEQ ID NO: 15) |
| | Light chain variable region_VL EIVLTQSPGTLSLSPGERATLSCSASSSVSFMHWYQQKPGQAPRR WIYDTTKLASGIPDRFSGSGSGTDYTLTISRLEPEDFAVYYCQQWN SHPLTFGQGTTVEIK (SEQ ID NO: 14) |
| 22Mono3L7F | Heavy chain variable region_VH |
| | EVTLKESGPVLVKPTETLTLTCTASGYPFTNYWMHWVRQPPGKA LEWIGEINPSNGRTNYNEKFKSRATLSLDKSKSQAVLTMTNMDPV DTATYYCARGFAYWGQGTLVTVSS (SEQ ID NO: 17) |
| | Light chain variable region_VL |
| | EIVLTQSPATLSLSPGERATLSCSASSSVSFMHWYQQKPGQAPRRW IYDTTKLASGIPARFSGSGSGTDYTLTISSLEPEDFAVYYCQQWNSH PLTFGQGTKLEIK (SEQ ID NO: 16) |
| 22Mono4DN4 | Heavy chain variable region_VH |
| | ELVQSGAEVKKPGSSVKVSCKASGYPFTNYWMHWVRQAPGQGL EWIGEINPSNGRTNYNEKFKSRATLTLDKSTSTAYMELSSLRSEDT AVYYCARGFAYWGQGTLVTVSS (SEQ ID NO: 19) |
| | Light chain variable region_VL |
| | EIVLTQSPATLSLSPGERATLSCSASSSVSFMHWYQQKPGQAPRRW IYDTTKLASGVPARFSGSGSGTDYTLTISSLEPEDFAVYYCQQWNS HPLTFGQGTKVEIK (SEQ ID NO: 18) |
| 22Mono5UQY | Heavy chain variable region_VH |
| | QVQLQESGPGLVKPSETLSLTCTASGYPFTNYWMHWVRQPPGKG LEWIGEINPSNGRTNYNEKFKSRATLSLDKSKNQASLNLDSVSAA DTAIYYCARGFAYWGKGSTVTVSS (SEQ ID NO: 21) |
| | Light chain variable region_VL |
| | AIQLTQSPSSLSASVGDRVTITCSASSSVSFMHWYQQKPGKAPKR WIYDTTKLASGVPSRFSGSGSGTDYTFTISSLQPEDIATYYCQQWN SHPLTFGPGTKVDIK (SEQ ID NO: 20) |

The VH and VL regions of the above antibody were linked to the human IgG1 Fc region and kappa constant region, and the heavy chain and light chain sequences of the antibody were inserted into the pcDNA3.4 vector. The vector was transiently expressed in HEK293 cells, and the antibody was purified by protein A or G.

Moreover, VH and VL of ch-72C7 antibody were substituted for VH and VL of human IgG1 antibody, respectively, to form chimeric antibody 22mono, which was used as a control to evaluate the humanization result of each antibody.

**Table 12**

| Chimeric antibody | Sequence (the CDR region is underlined) |
|---|---|
| 22Mono | Heavy chain _HC QVQLQQPGAELVKPGTSVKLSCKASGYPFTNYWMHWVRQRPGQGLE WIGEINPSNGRTNYNEKFKSLATLTLDKSSSTAYMHLNSLTSEDSAVYYC ARGFAYWGQGTLVTVSSASTKGPSVFPLAPSSKSTSGGTAALGCLVKDY FPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQP REPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKS LSLSP (SEQ ID NO: 90) |
| | Light chain_LC |
| | QIVLSQFPAIMSASPGEKVTMTCSASSSVSFMHWYQQKSGTSPKRWIYD TTKLASGVPARFTGSGSGTSYSLTISSMEAEDAATYYCQQWNSHPLTFG PGTKLELKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGEC (SEQ ID NO: 91) |

### Example 5: Affinity test of humanized GPRC5D antibody

The recombinant GPRC5D antigen (Human GPRC5D protein-Flag-His tag (51.8KD) ACRO Cat:GPD-H52D3) was immobilized on a chip and detected by Octet^{®} R8 biomolecular interaction analysis system. The results are shown in Table 13.

**Table 13**

| Loading Sample ID | KD (M) | kₒₙ(1/Ms) | k_{dis}(1/s) |
|---|---|---|---|
| 22Mono5JO4 | 7.87E-10 | 1.51E+05 | 1.19E-04 |
| 22monoO3L7 | 2.56E-08 | 7.80E+04 | 2.00E-03 |
| 22mono4DN4 | 1.69E-08 | 6.73E+04 | 1.14E-03 |
| 22mono5UQY | 5.67E-09 | 8.62E+04 | 4.89E-04 |

Based on the above data, VH and VL of 22Mono5JO4 were used as the results of humanization of the antibody and used for the subsequent construction of diabodies.

### Example 6: Construction of multi-specific antibody

Using the above-mentioned GPRC5D antibody and adopting four different antibody structures (Figs. 8 to 11), a series of CD3 × GPRC5D bispecific antibodies or Her2 × CD3 × GPRC5D trispecific antibodies were constructed. The results are shown in Table 14.

**Table 14**

| **Name of polypeptide complex** | **Chain and SEQ ID NO** | **N-terminal-to-C-terminal Sequence** | **Disulfide bond and charge mutation** | **Mutations of Fc** |
|---|---|---|---|---|
| 22A1 (Structure A) | First polypeptide (SEQ ID NO: 22) | VL1(GPRC5D)-VH2(CD3) | VL1 (Q37K, P100C) | / |
| | Second polypeptide (SEQ ID NO: 75) | VL2(CD3)-VH1(GPRC5D)-1st Fc | VH1 (Q39D, G44C) | T366W |
| | Third polypeptide (SEQ ID NO: 24) | 2nd Fc | / | Y407V, T366S, L368A |
| 22A2 (Structure B) | First polypeptide (SEQ ID NO: 25) | VL1(Her2)-VH2(CD3) | VL1 (Q38D and Q100C) VH2 (Q39D) | / |
| | Second polypeptide (SEQ ID NO: 76) | VL2(CD3)-VH1(Her2)-1st Fc | VL2 (Q40K) VH1 (Q39K and G44C) | T366W |
| | Third polypeptide (SEQ ID NO: 77) | VH3(GPRC5D)-CH1-2nd Fc | / | Y407V, T366S, L368A |
| | Fourth polypeptide (SEQ ID NO: 28) | VL3(GPRC5D)-CL | / | / |
| 22A3 (Structure B) | First polypeptide (SEQ ID NO: 29) | VL1(GPRC5D)-VH2(CD3) | VL1 (P100C, Q37K) | / |
| | Second polypeptide (SEQ ID NO: 78) | VL2(CD3)-VH1(GPRC5D)-1st Fc | VH1 (Q39D, G44C) | T366W |
| | Third polypeptide (SEQ ID NO: 79) | VH3(GPRC5D)-CH1-2nd Fc | / | Y407V, T366S, L368A |
| | Fourth polypeptide (SEQ ID NO: 32) | VL3(GPRC5D)-CL | / | / |
| 22A4 (Structure B) | First polypeptide (SEQ ID NO: 33) | VL1(GPRC5D)-VH2(GPRC5D) | VL1 (P100C, Q37K) | / |
| | Second polypeptide (SEQ ID NO: 80) | VL2(GPRC5D)-VH1(GPRC5D)-1st Fc | VH1 (Q39D, G44C) | T366W |
| | Third polypeptide (SEQ ID NO: 81) | VH3(CD3)-CH1-2nd Fc | / | Y407V, T366S, L368A |
| | Fourth polypeptide (SEQ ID NO: 36) | VL3(CD3)-CL | / | / |
| 22A5 (Structure C) | First polypeptide (SEQ ID NO: 83) | VH1(GPRC5D)-CH1a -1st Fc | / | Y407V, T366S, L368A |
| | Second polypeptide (SEQ ID NO: 40) | VL1(GPRC5D)-CLa | / | / |
| | Third polypeptide (SEQ ID NO: 82) | VH2(CD3)-CH1b -2nd Fc | VH2 (Q39D) CH1b (A141K, V173C and C220S) | T366W |
| | Fourth polypeptide (SEQ ID NO: 37) | VL2(CD3)-CLb | VL2 (Q40K) CLb (F116D, Q160C and C214S) | / |
| 22A6 (Structure D) | First polypeptide (SEQ ID NO: 85) | VH1(GPRC5D)-CH1a -1st Fc | / | Y407V, T366S, L368A |
| | Second polypeptide (SEQ ID NO: 44) | VL1/VL3(GPRC5D)-CLa | / | / |
| | Third polypeptide (SEQ ID NO: 84) | VH2(CD3)-CH1b-2nd Fc | VH2(Q39D) CH1b(A139K, V177C and C233S) | T366W |
| | Fourth polypeptide (SEQ ID NO: 41) | VH3 (GPRC5D)-CH1a-linker-VL2(CD3)-CLb | VH1 VL2 (Q40K) CLb (F116D, Q160C and C214S) | / |
| 22A8 (Structure D) | First polypeptide (SEQ ID NO: 87) | VH1 (GPRC5D, humanized)-CH1a-2nd Fc | / | Y407V, T366S, L368A |
| | Second polypeptide (SEQ ID NO: 48) | VL1/VL3 (GPRC5D, humanized)-CLa | / | / |
| | Third polypeptide (SEQ ID NO: 86) | VH2(CD3)-CH1b-1st Fc | CH1b (A139K, V177C and C233S) | T366W |
| | Fourth polypeptide (SEQ ID NO: 45) | VH3 (GPRC5D, humanized)-CH1a-linker-VL2(CD3)-CLb | CLb (F116D, Q160C and C214S) | / |

### Example 7: Detection of binding affinity of bispecific antibodies to monkey or human GPRC5D

The objective of this experiment was to compare the binding ability of the constructed bispecific antibodies to monkey GPRC5D or human GPRC5D by inoculating a defined amount of HEK293T cells overexpressing human GPRC5D (HEK293T-hGPRC5D) or monkey GPRC5D (HEK293T-cynoGPRC5D), followed by the addition of gradient dilutions of the test antibodies.

### Experimental method:

HEK293T-hGPRC5D or HEK293T-cynoGPRC5D cells in logarithmic growth phase were inoculated into a 96-well culture plate, and the antibodies at different concentrations (200, 66.7, 22.22, 7.41, 2.47, 0.82, 0.27, 0.09, 0.03, and 0.01nM) was added. 2 duplicate wells were set for each concentration and a negative control (PBS) well was also set. The cells were incubated at 25°C for about 60 minutes and then developed and detected. The raw data of the test was the difference between the detection wavelength and the reference wavelength. A dose-response curve was fitted using statistical software, based on OD value and the logarithmic value of the concentration, from which the EC50 was obtained.

**Table 15**

| **Parameter** | **Human** | | | **Monkey** | | |
|---|---|---|---|---|---|---|
| | **22A8** | **22B1** | **10B1** | **22A8** | **22B1** | **10B1** |
| Bottom | 6.483 | 3.572 | 1.005 | 0.7424 | 1.003 | 1.014 |
| Top | 331.6 | 232.8 | / | 92.87 | / | / |
| EC50 (nM) | 3.862 | 23.1 | >200 | 10.07 | >200 | >200 |

### Experimental results:

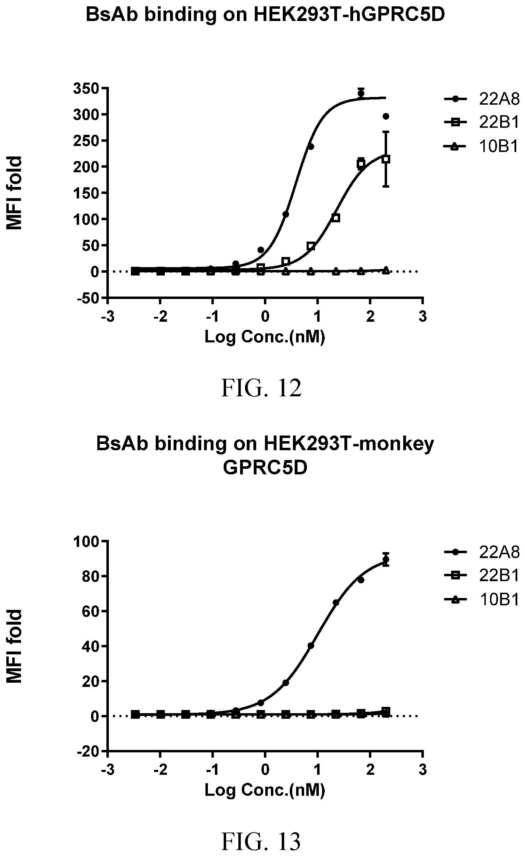

The experimental results are shown in Figs. 12 and 13. The binding affinity of 22A8 to monkey GPRC5D or human GPRC5D was comparable, with an EC50 of 10.07 nM and 3.8862 nM, respectively. The binding affinity of the positive control antibody 22B1 to human GPRC5D (EC50 is 23.1 nM) was significantly higher than the binding affinity to monkey GPRC5D (EC50 is > 200 nm). The negative antibody 10B1 has no binding to monkey GPRC5D protein or human GPRC5D protein.

### Example 8: In vitro cytotoxicity assay of bispecific antibodies

### 8.1. Detection of inhibitory effect of bispecific antibodies on the proliferation of NCI-H929 cells highly expressing GPRC5D

The objective of this experiment was to compare the inhibitory effect of PBMC on NCI-H929 cells mediated by the constructed bispecific antibodies.

### Experimental method:

H929 cells in logarithmic growth phase were prepared, and some cells were taken as a negative control of CFSE, and the rest cells were stained with 1uM CFSE. E:T cells (PBMC:H929) were inoculated into a 96-well culture plate at a ratio of 20:1, and the antibody at different concentrations (4500, 1500, 500, 166.67, 55.56, 18.52, 6.17, 2.06, 0.69, and 0.23 pM) was added with 2 replicate wells for each concentration, and a negative control (PBS) well was also set. After the cells were incubated at 37°C for 24 hrs, they were stained with PI and detected by FACS. The FACS data file was analyzed by Thermo Attune NxT software, and EC50 was analyzed by Graphpad Prism 6.0.

### Experimental results:

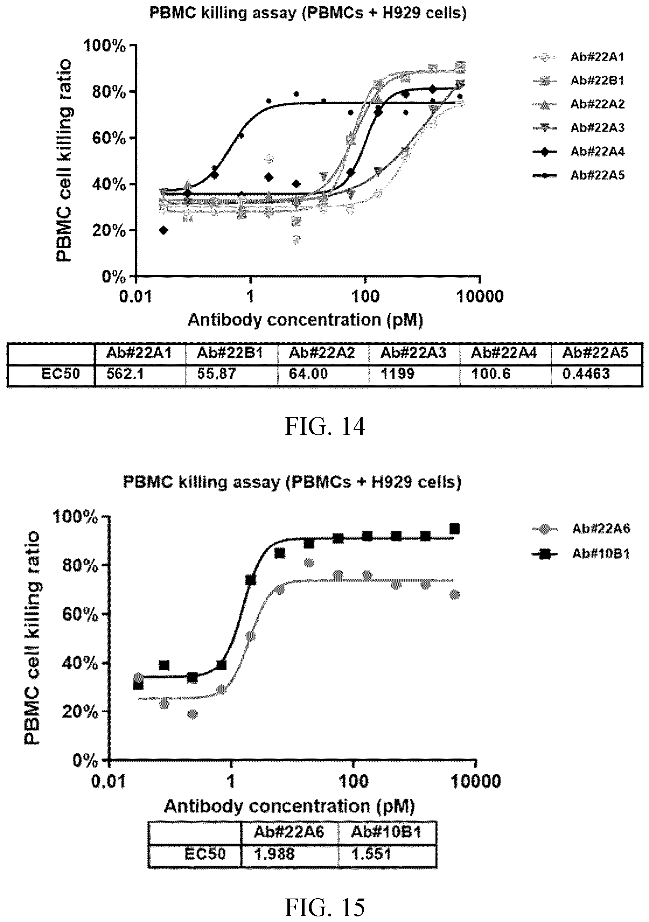

The 22 serial antibodies (22A1-22A8) and the positive antibody 22B1 at various concentrations were added into a co-culture system of H929 and PBMC (pre-stained with CFSE) respectively, and the ratio of CFSE/PI positive cells to CFSE positive cells was detected by flow cytometry, to evaluate the cytotoxicity on H929 cells of PBMCs mediated by the antibodies. As shown in Figs. 14-16, the antibodies 22A1 to 22A5 and 22B1 could mediate the cytotoxicity of PBMCs on H929 in a dose-dependent manner.

### 8.2. Detection of inhibitory effect of 22A8 and 22B1 antibodies on the proliferation of MM1S cells highly expressing GPRC5D

The objective of this experiment was to compare the inhibitory effects of PBMC mediated by 22A8 and 22B1 on the proliferation of MM1 S cells.

### Experimental method:

MM1S cells in logarithmic phase were prepared, and some cells were taken as a negative control of CFSE, and the rest cells were stained with 1uM CFSE. E:T cells (PBMC:MM1S) were inoculated into a 96-well culture plate at a ratio of 20:1, and the antibody at different concentrations (4500, 1500, 500, 166.67, 55.56, 18.52, 6.17, 2.06, 0.69, and 0.23 pM) was added with 2 replicate wells for each concentration, and a negative control (PBS) well was also set. After the cells were incubated at 37°C for 24 hrs, they were stained with PI and detected by FACS.

### Experimental results:

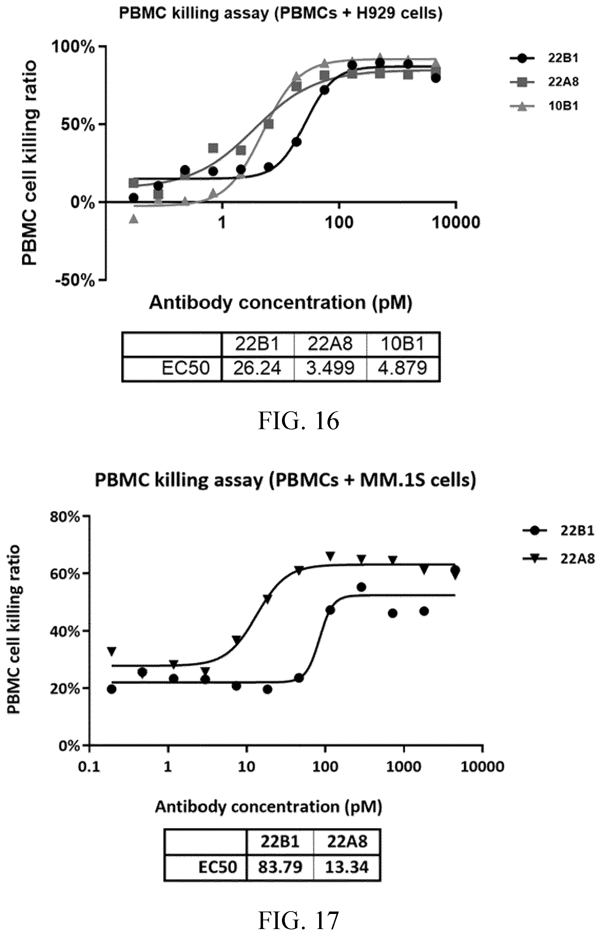

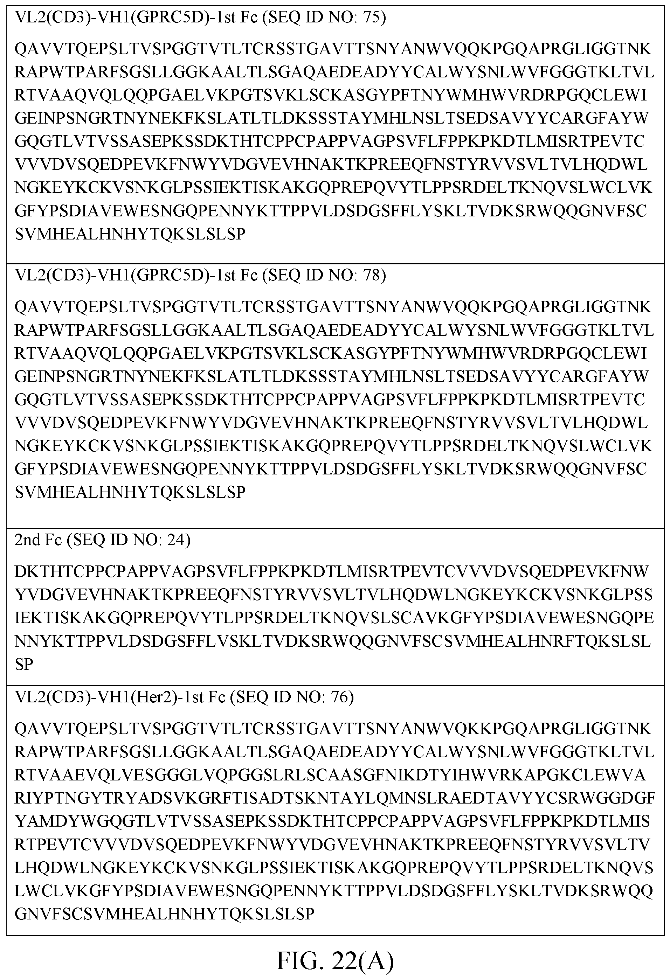

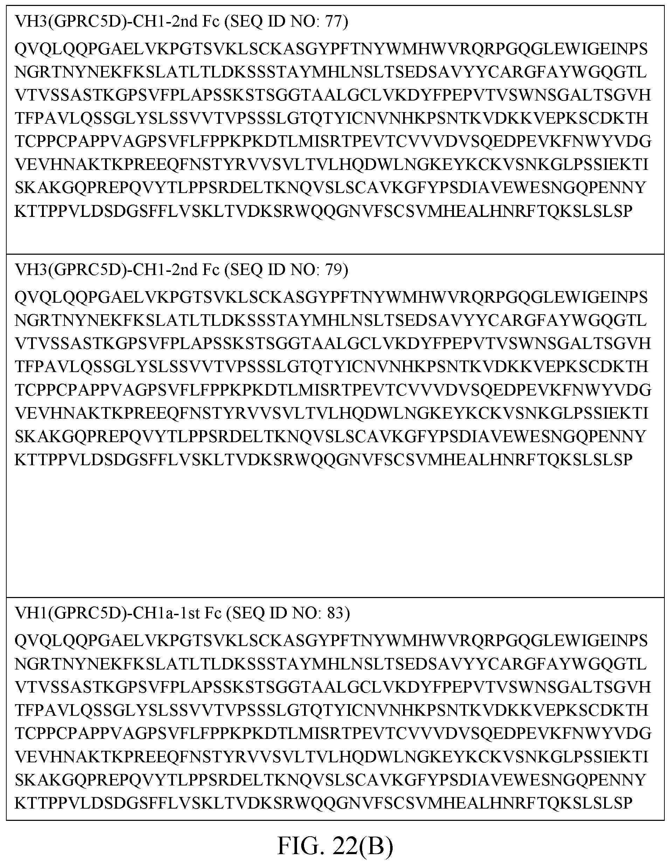

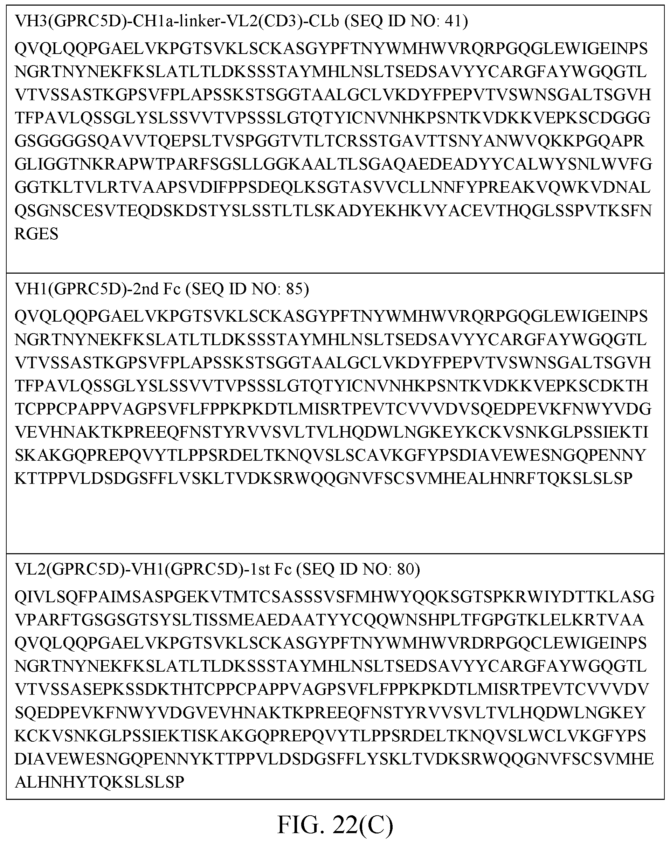

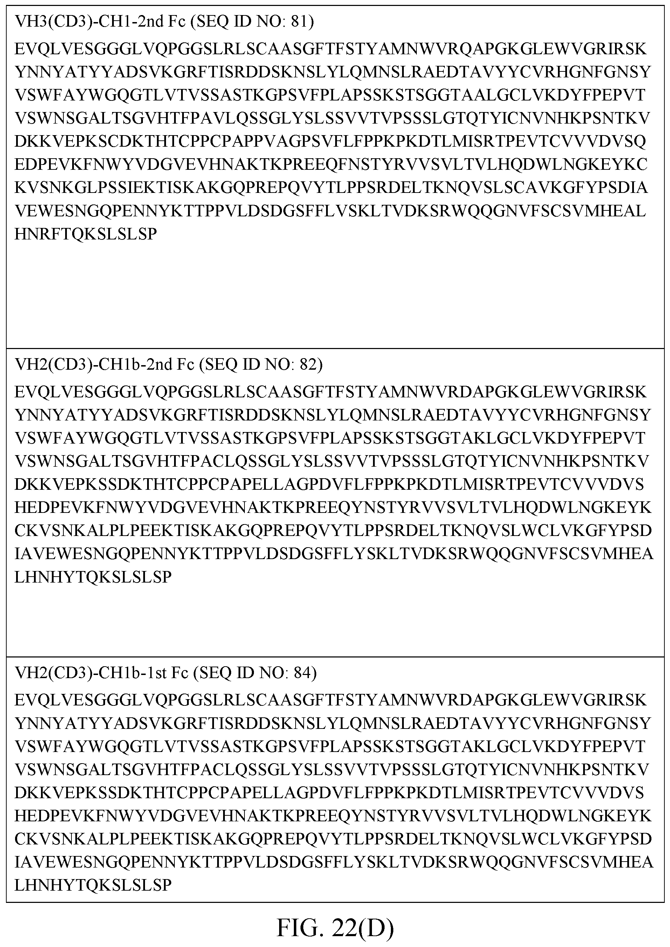

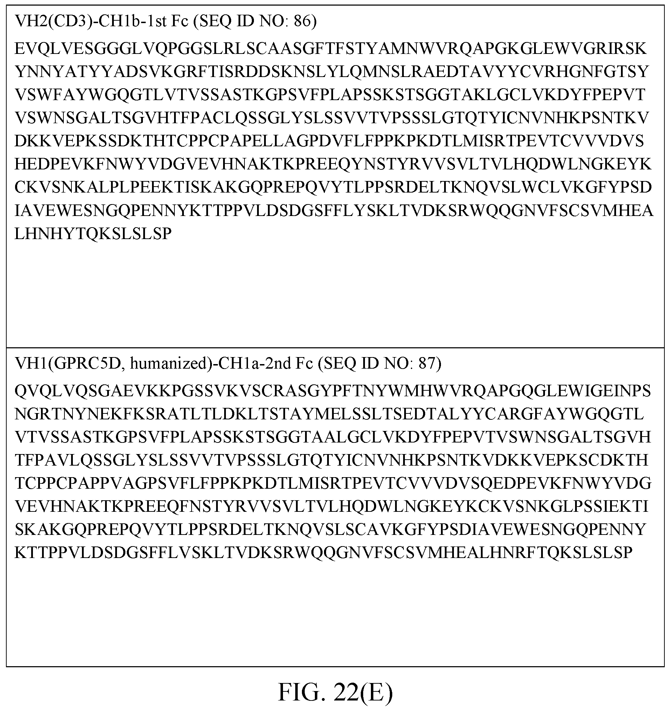

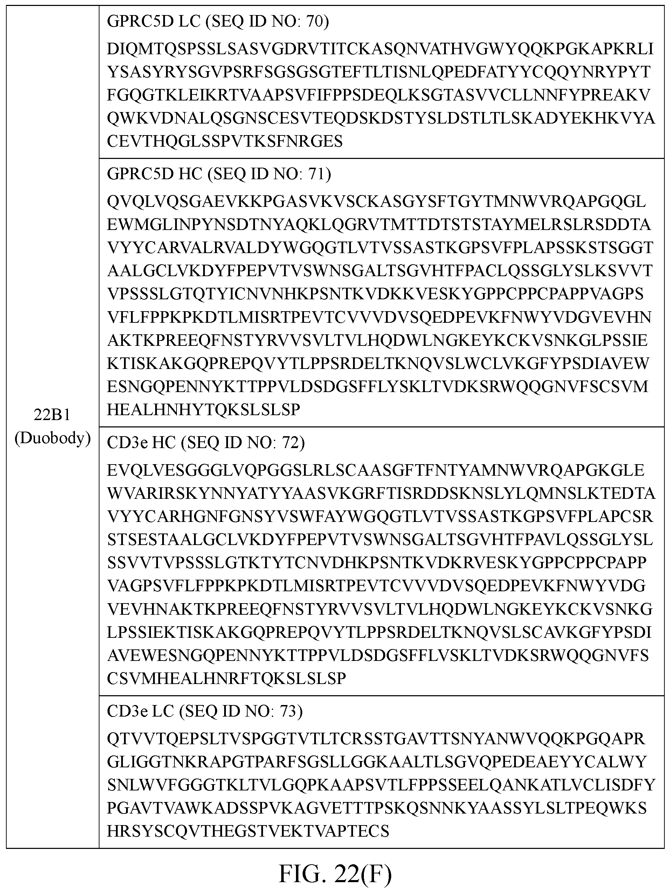

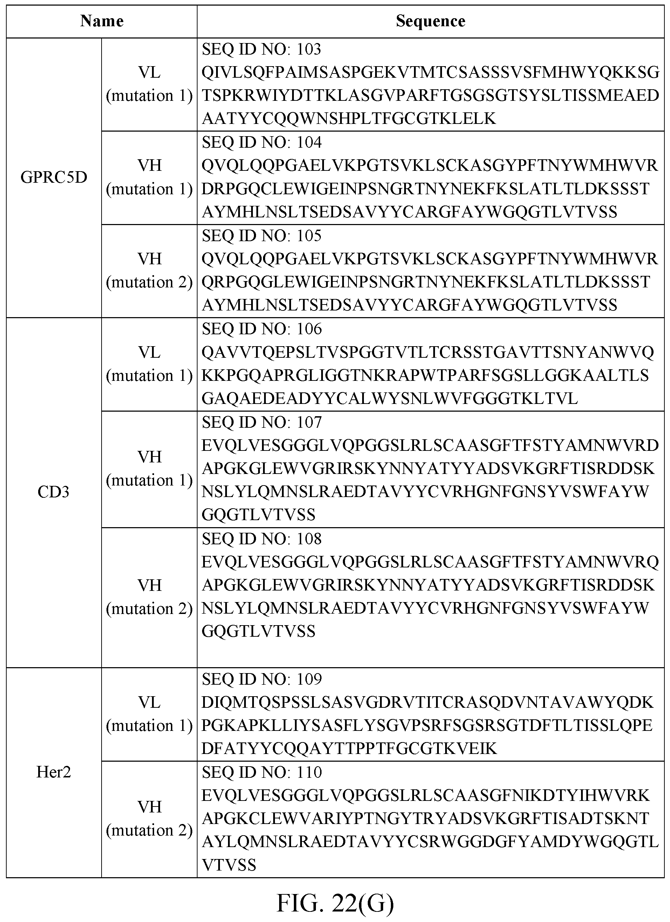

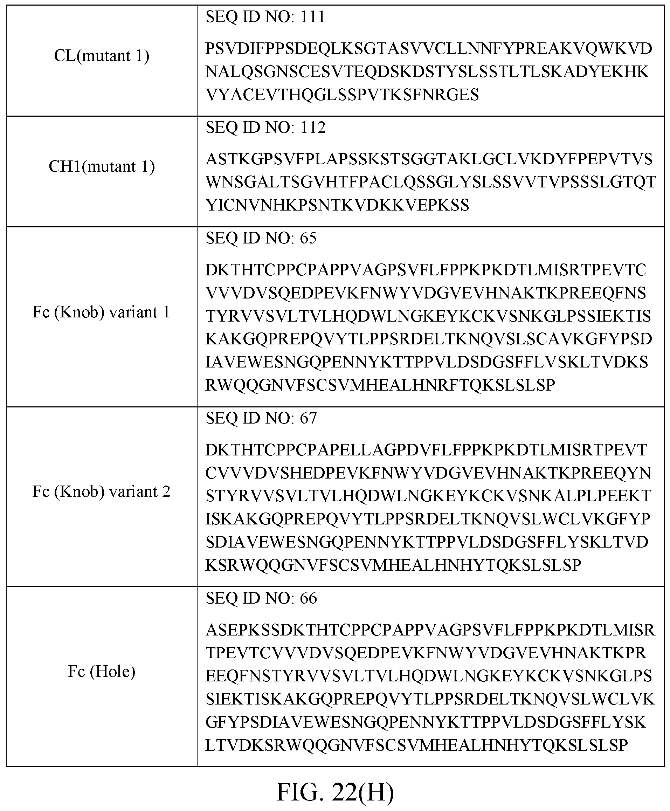

22A8, 22A16 and the positive antibody 22B1 of various concentrations were added into a co-culture system of MM1S and PBMC (pre-stained with CFSE) respectively, and the ratio of CFSE/PI positive cells to CFSE positive cells was detected by flow cytometry, to evaluate the killing effect on MM1S cells of PBMCs mediated by the antibodies. As shown in Fig. 17, 22A8 and 22B1 could mediate the cytotoxicity of PBMCs on MM1S in a dose-dependent manner, with an EC50 of 13.34 and 83.79 pM, respectively.

### 8.3. Detection of inhibitory effect of 22A8 and 22B1 antibodies on the proliferation of GPRCSD low-expressing cell line RPMI 8226 or KMS-12-BM

The objective of this experiment was to compare the inhibitory effects of PBMC mediated by 22A8 and 22B1 on the proliferation of RPMI8226 or KMS-12-BM cells.

### Experimental method:

RPMI 8226 or KMS-12-BM cells in logarithmic growth phase were prepared, and some cells were taken as a negative control of CFSE, and the rest cells were stained with 1uM CFSE. E:T cells (PBMC:MM1S) were inoculated into a 96-well culture plate at a ratio of 20:1, and added with the antibody of different concentrations (4500, 1500, 500, 166.67, 55.56, 18.52, 6.17, 2.06, 0.69, and 0.23 pM) with 2 replicate wells for each concentration. A negative control (PBS) well was also set. After the cells were incubated at 37°C for 24 hrs, they were stained with PI and detected by FACS. The FACS data file was analyzed by Thermo Attune NxT software, and EC50 was analyzed by Graphpad Prism 6.0.

### Experimental results:

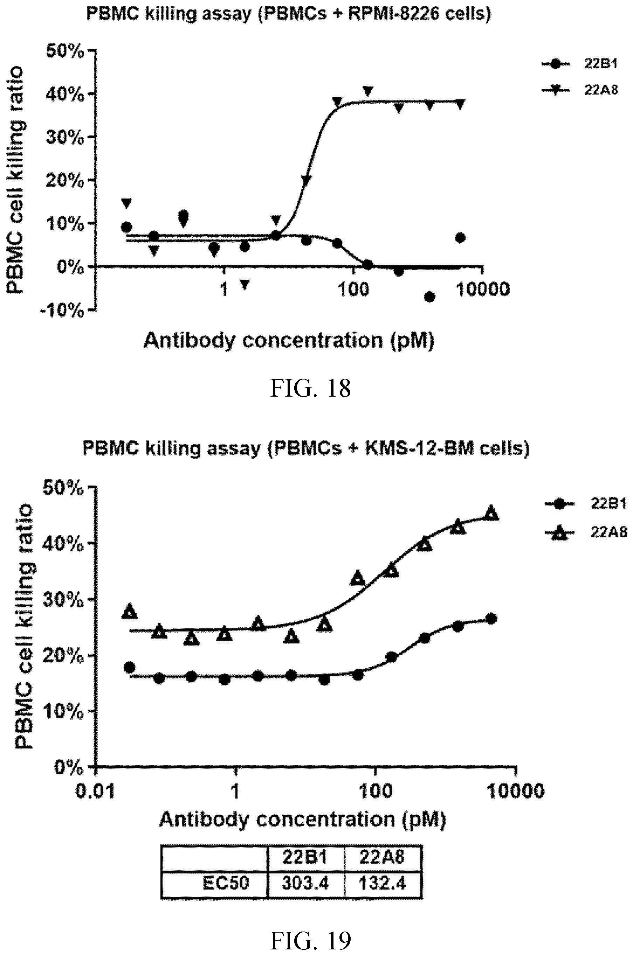

22A8 and the positive antibody 22B1 of various concentrations were added into a co-culture system of RPMI 8226 or KMS-12-BM and PBMC (pre-stained with CFSE), respectively, and the ratio of CFSE/PI positive cells to CFSE positive cells was detected by flow cytometry to evaluate the cytotoxicity on MM1S cells of PBMCs mediated by the antibodies. The data showed that the EC50 of 22A8 and 22B1 on RPMI 8266 was 19.91pM and NA, respectively (Fig. 18); and the EC50 of 22A8 and 22B1 on KMS-12-BM was 132.4 and 303.4 pM respectively (Fig. 19).

### Example 9: Binding affinity assay of humanized CD3 × GPRC5D bispecific antibody (22A8)

The recombinant CD3e antigen (Human CD3 epsilon Protein, His Tag, ACRO Cat: CDE-H5223) and recombinant GPRC5D antigen (Human GPRC5D protein-Flag-His tag (51.8KD) ACRO Cat: GPD-H52D3) were immobilized on a chip and detected by Octet^{®} R8 biomolecular interaction analysis system. The results have been shown in Table 16.

**Table 16**

| Loading Sample ID | Antigen | KD (M) | kₒₙ(1/Ms) | k_{dis} (1/s) |
|---|---|---|---|---|
| 22A8 | GPRC5D | 9.472E-09 | 6.291E04 | 5.959E-04 |
| 22A8 | CD3 | 2.999E-08 | 1.272E05 | 3.815E-03 |

The results showed that the humanized CD3 × GPRC5D bispecific antibody (22A8) had strong binding affinity to CPRG5D and CD3 antigen, respectively.

### Example 9: In vivo cytotoxicity assay of bispecific antibody

### 9.1 Animal model of human myeloma NCI-H929

### Experimental method:

5 × 10⁶ NCI-H929 cells/animal were subcutaneously inoculated into 6-week-old female NPG mice. 5 × 10⁶ PBMC cells/animal were injected intraperitoneally (i.p.) on the day (day 0) of inoculation of NCI-H929 cells. When the average tumor volume grew to about 113 mm³, the mice were grouped according to the tumor volume, and 22A2 of 1mg/kg and 22A8 of mg/kg were injected intravenously (i.v.), twice a week (BIW). The injection volume was 0.1 mL/10 g of weight.

The effect of drugs on tumor growth was investigated, with the tumor proliferation rate (T/C%) or tumor inhibition rate (TGI%) as a specific indicator.

The tumor diameter was measured with a vernier caliper three times a week.

The tumor volume was calculated by a formula: tumor volume TV (mm³) = 1/2 × (a × b²), where a stands for a major axis and b stands for a minor axis.

Calculation formula of tumor proliferation rate: T/C%= T mTV / C mTV × 100%, where TmTV: the average the the treatment group; and CmTV: the average of the control group.

Calculation formula of tumor inhibition rate: TGI% = (1-T/C) × 100%, where T/C% is the tumor proliferation rate, and the percentage tumor volume (or weight) of the treatment group relative to the control group at a defined time.

If the tumor volume is smaller than the initial volume, it is defined as partial regression (PR). If the tumor disappears completely, it is defined as complete regression (CR).

### Experimental results:

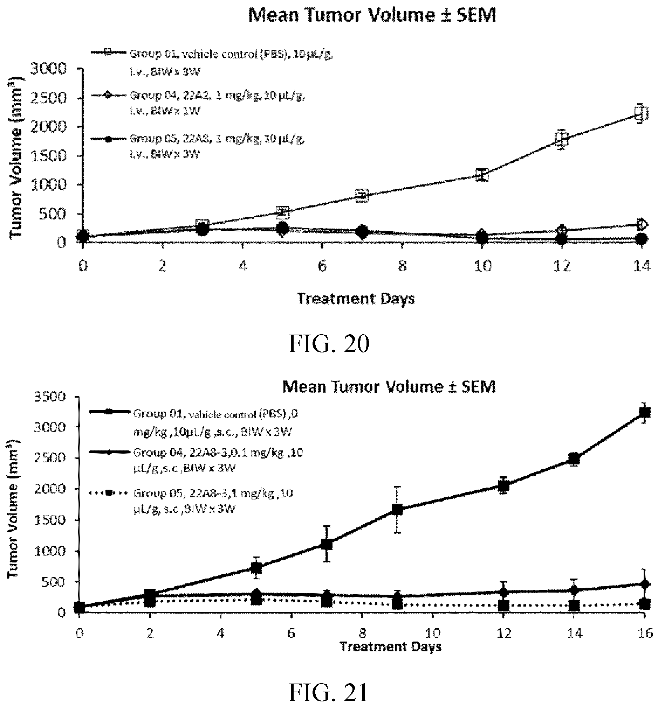

The results are shown in Fig. 20 and Table 17. The results showed that 22A2 and 22A8 (1 mg/kg, i.v., twice a week, 5 administrations in total) had a significant inhibitory effect on the growth of tumor in mice developed by subcutaneous transplantation of human myeloma cells NCI-H929 after immune reconstitution with human PBMC. At the end point (D17) of the test, TGI was respectively 84.84% and 97.71%, and the tumor in 5 out of 6 mice in the 22A8 group was completely regressed. The tumor-bearing mice were able to tolerate the above drugs well, and no obvious weight loss and other symptoms were noted during administration.

**Table 17**

| **Group** | **Administration** | **Dose (mg/kg)** | **Day 17** | | | | **Day 0** | **Day 17** | |
|---|---|---|---|---|---|---|---|---|---|
| | | | **Tumor volume (mean ± SEM)** | **T/C (%)** | **TGI (%)** | ***P* value** | **Average weight g** (mean±SEM) | **Average weight g** (mean±SEM) | **Variation of weight (%)** |
| 1 | Vehicle control (PBS) | 0 | 3032.59 ± 259.46 | - | - | - | 25.83 ± 0.77 | 27.7 ± 0.54 | 7.51% |
| 2 | 22A2 | 1 | 459.68 ± 82.07 | 15.16 | 84.84 | <0.001 | 27.17 ± 0.7 | 28.2 ± 0.57 | 3.88% |
| 3 | 22A8 | 1 | 69.48 ± 69.48 | 2.29 | 97.71 | <0.001 | 26.32 ± 0.48 | 26.63 ± 0.3 | 1.29% |

### 9.2 Animal model of human myeloma NCI-H929

### Experimental method:

5 × 10⁶ NCI-H929 cells/animal were subcutaneously inoculated into 6-week-old female NPG mice. 5 × 10⁶ PBMC cells/animal were injected intraperitoneally (i.p.) on the day (day 0) of inoculation of NCI-H929 cells. When the average tumor volume grew to about 97 mm³, the mice were grouped according to the tumor volume, and 22A8 of 0.1 mg/kg and 1 mg/kg were injected intravenously (i.v.), twice a week (BIW). The injection volume was 0.1 mL/10 g of weight.

The effect of drugs on tumor growth was investigated, with the tumor proliferation rate (T/C%) or tumor inhibition rate (TGI%) as a specific indicator.

The tumor diameter was measured with a vernier caliper three times a week.

The tumor volume was calculated by a formula: tumor volume TV (mm³) = 1/2 × (a × b²);
wherein a stands for a major axis and b stands for a minor axis.

Calculation formula of tumor proliferation rate: T/C%= T mTV / C mTV × 100%;
wherein TmTV: the average the the treatment group; and CmTV: the average of the control group.

Calculation formula of tumor inhibition rate: TGI% = (1-T/C) × 100%,
Wherein, T/C% is the tumor proliferation rate, and the percentage tumor volume (or weight) of the treatment group relative to the control group at a defined time.

If the tumor volume is smaller than the initial volume, it is defined as partial regression (PR). If the tumor disappears completely, it is defined as complete regression (CR).

### Experimental results:

The results are shown in Fig. 21 and Table 18. The results showed that 22A8 (0.1mg/kg or 1 mg/kg, i.v., twice a week, 5 administrations in total) had a significant inhibitory effect on the growth of tumor in mice developed by subcutaneous transplantation of human myeloma cells NCI-H929 after immune reconstitution with human PBMC. TGI of 22A8 was respectively 85.61% and 95.65%. Partial tumor regression was observed in 1 out of 5 mice in the 0.1 mg/kg 22A8 group and in 2 out of 5 mice in the 1 mg/kg 22A8 group. Except for in the 0.1 mg/kg 22A8 group, in which the tumor-bearing mice exhibited slight weight loss (the weight was decreased by 3.6% compared with that at D16 before administration), the tumor-bearing mice in the remaining group was able to tolerate the above drugs well, and no obvious weight loss and other symptoms were noted during administration.

**Table 18**

| **Group** | **Administration** | **Dose (mg/kg)** | **Day 16** | | | | **Day 0** | **Day 16** | |
|---|---|---|---|---|---|---|---|---|---|
| | | | **Tumor volume (mean ± SEM)** | **T/C (%)** | **TGI (%)** | ***P* Value** | **Average weight g (mean±SEM)** | **Average weight g (mean±SEM)** | **Variation of weight (%)** |
| 1 | Vehicle control (PBS) | 0 | 3236.87 ± 166.9 | - | - | - | 25.96 ± 0.86 | 29.4 ± 0.69 | 14.77% |
| 2 | 22A8 | 0.1 | 465.66 ± 239.92 | 14.39 | 85.61 | 0.050 | 28.46 ± 0.47 | 27.4 ± 0.56 | 3.60% |
| 3 | 22A8 | 1 | 140.95 ± 63.65 | 4.35 | 95.65 | 0.008 | 27.88 ± 0.93 | 27.88 ± 0.57 | 0.30% |

### 9.3. PK study of escalating doses administered subcutaneously or intravenously in cynomolgus macaques

### Experimental method:

4 female cynomolgus macaques, numbered #1, #2, #5 and #6, were used. Animals #1 and #2 were given the test agent 22A8 subcutaneously, and animals #5 and #6 were given the test agent 22A8 intravenously. The administration was completed within 90 min min. The administration dosage to animal #1 was 0.3 mg/kg, 1 mg/kg, and 3 mg/kg, the administration volume was 1mL/kg, and the agent was administered at days 0, 3 and 6, for a total of 3 times. The administration dosage to animal #2 was 1 mg/kg, 3 mg/kg, and 10 mg/kg, the administration volume was 1 mL/kg or 1.65 mL/kg, and the agent was administered at days 0, 3 and 6, for a total of 3 times. The administration dosage to animal #5 was 0.1 mg/kg, 0.3 mg/kg, and 1 mg/kg, the administration volume was 5 mL/kg, and the agent was administered at days 0, 3 and 6, for a total of 3 times. The administration dosage to animal #6 was 0.3 mg/kg, 1 mg/kg, and 3 mg/kg, the administration volume was 5 mL/kg, and the agent was administered at days 0, 3 and 6, for a total of 3 times. Among the above 4 animals, #1 and #5 were administered at the same time, and #2 and #6 were administered at the same time. Specific designs are shown in Table 19. During the test, blood samples were collected at various time points, and clinical observation, clinical pathology, serum cytokines, immunophenotype detection and blood drug concentration detection were performed to calculate the PK parameters.

**Table 19**

| **Group** | **Test agent** | **Dosing occasion** | **Route of administration** | **Administration dosage (mg/kg)** | **Administration concentration (mg/mL)** | **Administration volume (mL/kg)** |
|---|---|---|---|---|---|---|
| 1# | 22A8 | 1^{st} | s.c | 0.3 | 0.3 | 1 |
| | | 2^{nd} | | 1 | 1 | 1 |
| | | 3^{rd} | | 3 | 3 | 1 |
| 2# | 22A8 | 1^{st} | | 1 | 1 | 1 |
| | | 2^{nd} | | 3 | 3 | 1 |
| | | 3^{rd} | | 10 | 6.06 | 1.65 |
| 5# | 22A8 | 1^{st} | i.v. | 0.1 | 0.02 | 5 |
| | | 2^{nd} | | 0.3 | 0.06 | 5 |
| | | 3^{rd} | | 1 | 0.2 | 5 |
| 6# | 22A8 | 1^{st} | | 0.3 | 0.06 | 5 |
| | | 2^{nd} | | 1 | 0.2 | 5 |
| | | 3^{rd} | | **3** | **0.6** | **5** |

### Experimental results:

During the experiment, no obvious abnormality was observed in clinic for all animals, and no weight loss was noted.

The results show that 22A8 is well absorbed in animals. Cmax and AUC increased with the dose proportionally in the case of subcutaneous injection. For animal #1 (0.3/1/3mg/kg, Q3D × 3, s.c.), the half-life HL was 26-64 hrs. For animal #2 (1/3/10 mg/kg, Q3D × 3, s.c.), the half-life HL was 36-74 hrs. For animal #5 (0.1/0.3/1 mg/kg, Q3D × 3, i.v.), the half-life HL was 23-38 hrs. For animal #6 (0.3/1/3 mg/kg, Q3D × 3, i.v.), the half-life HL was 30-47 hrs.

Although the present application has been particularly shown and described with reference to specific embodiments (some of which are preferred embodiments), it will be understood by those skilled in the art that various changes in form and details can be made without departing from the spirit and scope of the present application disclosed herein.

## Claims

1. A multi-specific polypeptide complex, comprising a first antigen binding domain and a second antigen binding domain, wherein at least one of the first antigen binding domain and the second antigen binding domain binds to GPRC5D and comprises a GPRC5D binding domain; the GPRC5D binding domain comprises three heavy chain complementarity determining regions HCDR1, HCDR2, and HCDR3, and three light chain complementarity determining regions LCDR1, LCDR2, and LCDR3; and the heavy chain complementarity determining regions are identical to three heavy chain complementarity determining regions comprised in a heavy chain variable region (V_{H}) as shown in SEQ ID NO: 13, and the light chain complementarity determining regions are identical to three light chain complementarity determining regions comprised in a light chain variable region (V_{L}) as shown in SEQ ID NO: 12.

2. The polypeptide complex according to claim 1, wherein in the GPRC5D binding domain,
a) the HCDR1 comprises an amino acid sequence selected from the group consisting of SEQ ID NO: 4 or a variant thereof having no more than 3, 2 or 1 amino acid substitution, or SEQ ID NO: 9 or a variant thereof having no more than 3, 2 or 1 amino acid substitution;
b) the HCDR2 comprises an amino acid sequence selected from the group consisting of SEQ ID NO: 5 or a variant thereof having no more than 3, 2 or 1 amino acid substitution, or SEQ ID NO: 10 or a variant thereof having no more than 3, 2 or 1 amino acid substitution;
c) the HCDR3 comprises an amino acid sequence selected from the group consisting of SEQ ID NO: 6 or a variant thereof having no more than 3, 2 or 1 amino acid substitution, or SEQ ID NO: 11 or a variant thereof having no more than 3, 2 or 1 amino acid substitution;
d) the LCDR1 comprises an amino acid sequence selected from the group consisting of SEQ ID NO: 1 or a variant thereof having no more than 3, 2 or 1 amino acid substitution, or SEQ ID NO: 7 or a variant thereof having no more than 3, 2 or 1 amino acid substitution;
e) the LCDR2 comprises an amino acid sequence selected from the group consisting of SEQ ID NO: 2 or a variant thereof having no more than 3, 2 or 1 amino acid substitution, or SEQ ID NO: 8 or a variant thereof having no more than 3, 2 or 1 amino acid substitution; and
f) the LCDR3 comprises an amino acid sequence selected from the group consisting of SEQ ID NO: 3 or a variant thereof having no more than 3, 2 or 1 amino acid substitution.

3. The polypeptide complex according to any one of preceding claims, wherein in the GPRC5D binding domain,
a) the HCDR1 comprises the amino acid sequence as shown in SEQ ID NO: 4 or a variant thereof having no more than 3, 2 or 1 amino acid substitution, the HCDR2 comprises the amino acid sequence as shown in SEQ ID NO: 5 or a variant thereof having no more than 3, 2 or 1 amino acid substitution, the HCDR3 comprises the amino acid sequence as shown in SEQ ID NO: 6 or a variant thereof having no more than 3, 2 or 1 amino acid substitution, the LCDR1 comprises the amino acid sequence as shown in SEQ ID NO: 1 or a variant thereof having no more than 3, 2 or 1 amino acid substitution, the LCDR2 comprises the amino acid sequence as shown in SEQ ID NO: 2 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution; or
b) the HCDR1 comprises the amino acid sequence as shown in SEQ ID NO: 9 or a variant thereof having no more than 3, 2 or 1 amino acid substitution, the HCDR2 comprises the amino acid sequence as shown in SEQ ID NO: 10 or a variant thereof having no more than 3, 2 or 1 amino acid substitution, the HCDR3 comprises the amino acid sequence as shown in SEQ ID NO: 11 or a variant thereof having no more than 3, 2 or 1 amino acid substitution, the LCDR1 comprises the amino acid sequence as shown in SEQ ID NO: 7 or a variant thereof having no more than 3, 2 or 1 amino acid substitution, the LCDR2 comprises the amino acid sequence as shown in SEQ ID NO: 8 or a variant thereof having no more than 3, 2 or 1 amino acid substitution, and the LCDR3 comprises the amino acid sequence as shown in SEQ ID NO: 3 or a variant thereof having no more than 3, 2 or 1 amino acid substitution.

4. The polypeptide complex according to any one of preceding claims, wherein in the GPRC5D binding domain, the heavy chain variable region comprises the amino acid sequence as shown in SEQ ID NO: 13 or a variant thereof having no more than 3, 2 or 1 amino acid substitution, and the light chain variable region comprises the amino acid sequence as shown in SEQ ID NO: 12 or a variant thereof having no more than 3, 2 or 1 amino acid substitution.

5. The polypeptide complex according to any one of preceding claims, wherein the GPRC5D binding domain is humanized.

6. The polypeptide complex according to any one of preceding claims, wherein the GPRC5D binding domain comprises a heavy chain variable region (V_{H}) and/or a light chain variable region (V_{L}); and
a) the heavy chain variable region comprises an amino acid sequence selected from the group consisting of SEQ ID NO: 15, SEQ ID NO: 17, SEQ ID NO: 19, and SEQ ID NO: 21, or a variant thereof having no more than 3, 2 or 1 amino acid substitution; and
b) the light chain variable region comprises an amino acid sequence selected from the group consisting of SEQ ID NO: 14, SEQ ID NO: 16, SEQ ID NO: 18, and SEQ ID NO: 20, or a variant thereof having no more than 3, 2 or 1 amino acid substitution.

7. The polypeptide complex according to any one of preceding claims, wherein the GPRC5D binding domain comprises a heavy chain variable region (V_{H}) and/or a light chain variable region (V_{L}); and
a) the heavy chain variable region comprises the amino acid sequence as shown in SEQ ID NO: 15 or a variant thereof having no more than 3, 2 or 1 amino acid substitution, and the light chain variable region comprises the amino acid sequence as shown in SEQ ID NO: 14 or a variant thereof having no more than 3, 2 or 1 amino acid substitution;
b) the heavy chain variable region comprises the amino acid sequence as shown in SEQ ID NO: 17 or a variant thereof having no more than 3, 2 or 1 amino acid substitution, and the light chain variable region comprises the amino acid sequence as shown in SEQ ID NO: 16 or a variant thereof having no more than 3, 2 or 1 amino acid substitution;
c) the heavy chain variable region comprises the amino acid sequence as shown in SEQ ID NO: 19 or a variant thereof having no more than 3, 2 or 1 amino acid substitution, and the light chain variable region comprises the amino acid sequence as shown in SEQ ID NO: 18 or a variant thereof having no more than 3, 2 or 1 amino acid substitution; or
d) the heavy chain variable region comprises the amino acid sequence as shown in SEQ ID NO: 21 or a variant thereof having no more than 3, 2 or 1 amino acid substitution, and the light chain variable region comprises the amino acid sequence as shown in SEQ ID NO: 20 or a variant thereof having no more than 3, 2 or 1 amino acid substitution,
wherein the amino acid substitution does not occur in the CDR region.

8. The polypeptide complex according to any one of preceding claims, further comprising an immunoglobulin constant region, optionally a human immunoglobulin constant region, or optionally a human IgG constant region.

9. The polypeptide complex according to any one of preceding claims, wherein the other one of the first antigen binding domain and the second antigen binding domain binds to an antigen other than GPRC5D.

10. The polypeptide complex according to claim 9, wherein the antigen other than GPRC5D is an immunostimulatory antigen, optionally wherein the immunostimulatory antigen is CD3.

11. The polypeptide complex according to claim 9, wherein the other one of the first antigen binding domain and the second antigen binding domain comprises a CD3 binding domain.

12. The polypeptide complex according to claim 11, wherein the CD3 binding domain comprises three heavy chain complementarity determining regions HCDR1, HCDR2 and HCDR3, and three light chain complementarity determining regions LCDR1, LCDR2 and LCDR3; the HCDR1 comprises the amino acid sequence as shown in SEQ ID NO: 49 or a variant thereof having no more than 3, 2 or 1 amino acid substitution, the HCDR2 comprises the amino acid sequence as shown in SEQ ID NO: 50 or a variant thereof having no more than 3, 2 or 1 amino acid substitution, the HCDR3 comprises the amino acid sequence as shown in SEQ ID NO: 51 or a variant thereof having no more than 3, 2 or 1 amino acid substitution, the LCDR1 comprises the amino acid sequence as shown in SEQ ID NO: 52 or a variant thereof having no more than 3, 2 or 1 amino acid substitution, the LCDR2 comprises the amino acid sequence as shown in SEQ ID NO: 53 or a variant thereof having no more than 3, 2 or 1 amino acid substitution, and the LCDR3 comprises the amino acid sequence as shown in SEQ ID NO: 54 or a variant thereof having no more than 3, 2 or 1 amino acid substitution; or
the HCDR1 comprises the amino acid sequence as shown in SEQ ID NO: 92 or a variant thereof having no more than 3, 2 or 1 amino acid substitution, the HCDR2 comprises the amino acid sequence as shown in SEQ ID NO: 93 or a variant thereof having no more than 3, 2 or 1 amino acid substitution, the HCDR3 comprises the amino acid sequence as shown in SEQ ID NO: 94 or a variant thereof having no more than 3, 2 or 1 amino acid substitution, the LCDR1 comprises the amino acid sequence as shown in SEQ ID NO: 95 or a variant thereof having no more than 3, 2 or 1 amino acid substitution, the LCDR2 comprises the amino acid sequence as shown in SEQ ID NO: 96 or a variant thereof having no more than 3, 2 or 1 amino acid substitution, and the LCDR3 comprises the amino acid sequence as shown in SEQ ID NO: 54 or a variant thereof having no more than 3, 2 or 1 amino acid substitution.

13. The polypeptide complex according to claim 12, wherein the CD3 binding domain comprises a heavy chain variable region and a light chain variable region; and the heavy chain variable region comprises an amino acid sequence as shown in SEQ ID NO: 55 or a variant thereof having no more than 3, 2 or 1 amino acid substitution, and the light chain variable region comprises an amino acid sequence as shown in SEQ ID NO: 56 or a variant thereof having no more than 3, 2 or 1 amino acid substitution.

14. The polypeptide complex according to any one of claims 1 to 13, wherein the first antigen binding domain and the second antigen binding domain form a DICAD domain comprising:
(i) a first polypeptide, comprising, in a N-terminal-to-C-terminal direction, a first light chain variable domain VL1 binding to a first antigen and a second heavy chain variable domain VH2 binding to a second antigen, wherein VL1 and VH2 are linked directly or by a first linker; and
(ii) a second polypeptide, comprising, in a N-terminal-to-C-terminal direction, a second light chain variable domain VL2 binding to the second antigen and a first heavy chain variable domain VH1 binding to the first antigen, wherein VL2 and VH1 are linked directly or by a second linker,
wherein
VL1 and VH1 associate to form the first antigen binding domain,
VL2 and VH2 associate to form the second antigen binding domain, and
VL1 and VH1 are covalently linked by a disulfide bond.

15. The polypeptide complex according to claim 14, wherein the first linker and/or the second linker each independently comprise(s) 5 to 9 amino acid residues.

16. The polypeptide complex according to claim 14 or 15, wherein VL1 has a first cysteine substitution in the FR, VH1 has a second cysteine substitution in the FR, and the first cysteine and the second cysteine form a disulfide bond.

17. The polypeptide complex according to claim 16, wherein the first cysteine and second cysteine are selected from the group consisting of:
100C in VL1 and 44C in VH1;
43C in VL1 and 105C in VH1;
49C in VL1 and 100bC in VH1;
50C in VL1 and 100C in VH1; and
46C in VL1 and 101C in VH1;
wherein the numbering is based on Kabat numbering.

18. The polypeptide complex according to claim 17, wherein the disulfide bond is formed between 100C in VL1 and 44C in VH1.

19. The polypeptide complex according to any one of claims 14 to 18, wherein VL1 and VH1 further have an electrostatic interaction between two residues with opposite charges.

20. The polypeptide complex according to any one of claims 14 to 19, wherein the two residues with opposite charges are introduced into VL1 and VH1 to replace residues at positions selected from the group consisting of:
a) Q38 in VL1 and Q39 in VH1;
b) Q40 in VL1 and Q39 in VH1, or
c) Q37 in VL1 and Q39 in VH1, wherein the numbering is based on Kabat numbering.

21. The polypeptide complex according to any one of claims 14 to 20, wherein VL2 and VH2 further have an electrostatic interaction between two residues with opposite charges.

22. The polypeptide complex according to any one of claims 14 to 21, wherein the two residues with opposite charges are introduced into VL2 and the VH2 to replace residues at positions selected from the group consisting of:
a) Q38 in VL2 and Q39 in VH2;
b) Q40 in VL2 and Q39 in VH2, or
c) Q37 in VL2 and Q39 in VH2, wherein the numbering is based on Kabat numbering.

23. The polypeptide complex according to any one of claims 19 to 22, wherein the two residues with opposite charges comprise a negatively charged amino acid residue selected from the group consisting of aspartate (D) or glutamate (E), and a positively charged amino acid residue selected from the group consisting of lysine (K) or arginine (R).

24. The polypeptide complex according to claim 23, wherein at least one of the residues in the FR of VL1 is replaced by a negatively charged amino acid, and at least one of the residues in the FR of VH1 is replaced by a positively charged amino acid; or at least one of the residues in the FR of VL1 is replaced by a positively charged amino acid, and at least one of the residues in the FR of VH1 is replaced by a negatively charged amino acid.

25. The polypeptide complex according to any one of claims 17 to 24, wherein the first antigen binding domain comprises the GPRC5D binding domain as defined in any one of claims 1 to 7, and the second antigen binding domain comprises the CD3 binding domain as defined in claim 11 or 12.

26. The polypeptide complex according to any one of claims 16 to 25, wherein the amino acid sequence of the first polypeptide comprises an amino acid sequence as shown in SEQ ID NO: 22, and the amino acid sequence of the second polypeptide comprises an amino acid sequence as shown in SEQ ID NO: 23.

27. The polypeptide complex according to any one of claims 14 to 26, wherein the second polypeptide further comprises a first Fc polypeptide at the C terminus.

28. The polypeptide complex according to any one of claims 14 to 27, further comprising a third polypeptide comprising a second Fc polypeptide at the C terminus.

29. The polypeptide complex according to any one of claims 14 to 26, further comprising a third antigen binding domain, wherein optionally, the third antigen binding domain comprises a Fab domain.

30. The polypeptide complex according to claim 29, wherein the Fab domain comprises:
(i) a third polypeptide, comprising, in a N-terminal-to-C-terminal direction, a third heavy chain variable domain VH3 binding to a third antigen and a CH1 domain; and
(ii) a fourth polypeptide, comprising, in a N-terminal-to-C-terminal direction, a third light chain variable domain VL3 binding to the third antigen and a CL domain,
wherein VL3 and VH3 associate to form the third antigen binding domain.

31. The polypeptide complex according to claim 30, wherein the Fab domain binds to GPRC5D, and comprises the GPRC5D binding domain as defined in any one of claims 1 to 7.

32. The polypeptide complex according to claim 31, wherein the first polypeptide comprises an amino acid sequence as shown in SEQ ID NO: 29, the second polypeptide comprises an amino acid sequence as shown in SEQ ID NO: 30, the third polypeptide comprises an amino acid sequence as shown in SEQ ID NO: 31, and the fourth polypeptide comprises an amino acid sequence as shown in SEQ ID NO: 32.

33. The polypeptide complex according to any one of claims 14 to 24, further comprising a third antigen binding domain, wherein optionally, the third antigen binding domain comprises a Fab domain, and the Fab domain comprises:
(iii) a third polypeptide, comprising, in a N-terminal-to-C-terminal direction, a third heavy chain variable domain VH3 binding to a third antigen and a CH1 domain; and
(iv) a fourth polypeptide, comprising, in a N-terminal-to-C-terminal direction, a third light chain variable domain VL3 binding to the third antigen and a CL domain,
wherein VL3 and VH3 associate to form the third antigen binding domain.

34. The polypeptide complex according to claim 33, wherein the first antigen, the second antigen and the third antigen are each independently selected from GPRC5D, an immunostimulatory antigen and a tumor antigen; and optionally, the immunostimulatory antigen is CD3, and the tumor antigen is Her2.

35. The polypeptide complex according to claim 34, wherein the first antigen is Her2, the second antigen is CD3, and the third antigen is GPRC5D.

36. The polypeptide complex according to claim 35, wherein the Her2 binding domain comprises three heavy chain complementarity determining region HCDR1, HCDR2 and HCDR3 in a heavy chain variable region (V_{H}), and three light chain complementarity determining regions LCDR1, LCDR2 and LCDR3 in a light chain variable region (V_{L}); and the HCDR1 comprises the amino acid sequence as shown in SEQ ID NO: 57 or a variant thereof having no more than 3, 2 or 1 amino acid substitution, the HCDR2 comprises the amino acid sequence as shown in SEQ ID NO: 58 or a variant thereof having no more than 3, 2 or 1 amino acid substitution, the HCDR3 comprises the amino acid sequence as shown in SEQ ID NO: 59 or a variant thereof having no more than 3, 2 or 1 amino acid substitution, the LCDR1 comprises the amino acid sequence as shown in SEQ ID NO: 60 or a variant thereof having no more than 3, 2 or 1 amino acid substitution, the LCDR2 comprises the amino acid sequence as shown in SEQ ID NO: 61 or a variant thereof having no more than 3, 2 or 1 amino acid substitution, and the LCDR3 comprises the amino acid sequence as shown in SEQ ID NO: 62 or a variant thereof having no more than 3, 2 or 1 amino acid substitution.

37. The polypeptide complex according to claim 36, wherein the Her2 binding domain comprises a light chain variable domain having the amino acid sequence as shown in SEQ ID NO: 64, and VH3 comprises a heavy chain variable domain having the amino acid sequence as shown in SEQ ID NO: 63.

38. The polypeptide complex according to any one of claims 35 to 37, wherein the first antigen binding domain comprises the Her2 binding domain as defined in any one of claims 39 to 40, the second antigen binding domain comprises the CD3 binding domain as defined in any one of claims 15 to 16, and the third antigen binding domain comprises the antigen binding fragment of an antibody binding to GPRC5D according to any one of claims 1 to 10.

39. The polypeptide complex according to claim 38, wherein the first polypeptide comprises the amino acid sequence as shown in SEQ ID NO: 25, the second polypeptide comprises the amino acid sequence as shown in SEQ ID NO: 26, the third polypeptide comprises the amino acid sequence as shown in SEQ ID NO: 27, and the fourth polypeptide comprises the amino acid sequence as shown in SEQ ID NO: 28.

40. The polypeptide complex according to claim 33, wherein the first antigen is GPRC5D, the second antigen is GPRC5D, and the third antigen is CD3; and the first antigen binding domain and the second antigen binding domain comprise the GPRC5D binding domain as defined in any one of claims 1 to 7, and the third antigen binding domain comprises the CD3 binding domain as defined in claim 12 or 13.

41. The polypeptide complex according to claim 40, wherein the first polypeptide comprises the amino acid sequence as shown in SEQ ID NO: 33, the second polypeptide comprises the amino acid sequence as shown in SEQ ID NO: 34, the third polypeptide comprises the amino acid sequence as shown in SEQ ID NO: 35, and the fourth polypeptide comprises the amino acid sequence as shown in SEQ ID NO: 36.

42. The polypeptide complex according to any one of claims 30 to 41, wherein the second polypeptide further comprises, in a N-terminal-to-C-terminal direction, a first Fc polypeptide, and/or the third polypeptide further comprises, in a N-terminal-to-C-terminal direction, a second Fc polypeptide; and the first Fc polypeptide and the second Fc polypeptide are bounded to form a dimer.

43. The polypeptide complex according to any one of claims 1 to 8, comprising the first antigen binding domain and the second antigen binding domain, wherein
the first antigen binding domain comprises a first Fab domain, comprising:
(i) a first polypeptide, comprising, in a N-terminal-to-C-terminal direction, a first heavy chain variable domain VH1 binding to a first antigen and a first CH1 domain CH1a, and
(ii) a second polypeptide, comprising, in a N-terminal-to-C-terminal direction, a first light chain variable domain VL1 binding to the first antigen and a first CL domain CLa; and
the second antigen binding domain comprises a second Fab domain, comprising:
(iii) a third polypeptide, comprising, in a N-terminal-to-C-terminal direction, a second heavy chain variable domain VH2 binding to a second antigen and a second CH1 domain CH1b, and
(iv) a fourth polypeptide, comprising, in a N-terminal-to-C-terminal direction, a second light chain variable domain VL2 binding to the second antigen and a second CL domain CLb, wherein VL1 and VH1 associate to form the first antigen binding domain, and VL2 and VH2 associate to form the second antigen binding domain; and
CH1a and CLa are paired, CH1b and CLb are paired, and the pairing of CH1a and CLa and the pairing of CH1b and CLb are configured to avoid mispairing between CH1a and CLb and/or mispairing between CH1b and CLa.

44. The polypeptide complex according to claim 43, wherein the first antigen binding domain comprises the GPRC5D binding domain as defined in any one of claims 1 to 7.

45. The polypeptide complex according to claim 43 or 44, wherein the second antigen binding domain comprises the CD3 binding domain as defined in 12 or 13.

46. The polypeptide complex according to any one of claims 43 to 45, wherein the pairing of CH1b and CLb has at least one non-natural disulfide bond, which hinders mispairing between CH1b and CLa and/or mispairing between CH1a and CLb.

47. The polypeptide complex according to claim 46, wherein a first CH1/CL pair and a second CH1/CL pair are respectively selected from CH1b/CLb and CH1a/CLa, the first CH1/CL pair is formed by a first disulfide bond, the first disulfide bond is non-natural, and optionally the originally naturally occurring disulfide bond in the first CH1/CL pair is deleted or destroyed.

48. The polypeptide complex according to claim 47, wherein the second CH1/CL pair is formed by a second disulfide bond, and the second disulfide bond is located at a different position from the first disulfide bond, and is optionally a naturally occurring disulfide bond.

49. The polypeptide complex according to claim 47 or 48, wherein the first disulfide bond is formed by two cysteines introduced at positions selected from the group consisting of:
a) heavy chain EU position 126 and light chain EU position 121 in the first CH1/CL pair;
b) heavy chain EU position 173 and light chain EU position 160 in the first CH1/C pair; and
c) heavy chain EU position 128 and light chain EU position 118 in the first CH1/CL pair.

50. The polypeptide complex according to any one of claims 47 to 49, wherein the naturally occurring disulfide bond is formed between heavy chain EU position 220 and light chain EU position 214.

51. The polypeptide complex according to any one of claims 47 to 50, wherein the first CH1/CL pair comprises CH1 with a mutation to a cysteine residue at EU position 126 and a mutation to a non-cysteine residue at EU position 220; and CL with a mutation to a cysteine residue at EU position 121 and a mutation to a non-cysteine residue at EU position 214.

52. The polypeptide complex according to any one of claims 47 to 51, wherein the first CH1/CL pair comprises at least one mutation from an uncharged amino acid residue to a charged amino acid residue, and/or at least one mutation from a charged amino acid residue to an oppositely charged amino acid residue, such that the first CH1/CL pair comprises a first pair of oppositely charged residues that favors the pairing of the first CH1/CL pair.

53. The polypeptide complex according to claim 52, wherein the second CH1/CL pair comprises at least one mutation from an uncharged amino acid residue to a charged amino acid residue, and/or at least one mutation from a charged amino acid residue to an oppositely charged amino acid residue, such that the second CH1/CL pair comprises a second pair of oppositely charged residues that favors the pairing of the second CH1/CL pair, and optionally the first pair of oppositely charged residues and the second pair of oppositely charged residues hinder the pairing of CH1a and CLb or the pairing of CH1b and CLa.

54. The polypeptide complex according to claim 53, wherein the first pair of oppositely charged residues and the second pair of oppositely charged residues are configured such that CH1a and CLb are both positively charged or negatively charged, and/or CH1b and CLa are both positively charged or negatively charged.

55. The polypeptide complex according to any one of claims 52 to 54, wherein the first pair of oppositely charged residues and/or the second pair of oppositely charged residues is introduced at a set of heavy chain-light chain EU positions selected from the group consisting of:
a) heavy chain EU position 183 and light chain EU position 176 in the first CH1/CL pair;
b) heavy chain EU position 183 and light chain EU position 133 in the first CH1/CL pair;
c) heavy chain EU position 147 and light chain EU position 176 in the first CH1/CL pair;
d) heavy chain EU position 141 and light chain EU position 116 in the first CH1/CL pair;
e) heavy chain EU position 126 and light chain EU position 121 in the first CH1/CL pair; and
f) heavy chain EU position 218 and light chain EU position 122 in the first CH1/CL pair.

56. The polypeptide complex according to any one of claims 52 to 55, wherein the pair of oppositely charged amino acid residues comprises one positively charged amino acid residue and one negatively charged amino acid residue, wherein the positively charged amino acid residue is selected from the group consisting of lysine (K), histidine (H) and arginine (R), and/or the negatively charged amino acid residue is selected from the group consisting of aspartate (D) and glutamate (E).

57. The polypeptide complex according to any one of claims 52 to 56, wherein the pairing of CH1b and CLb has a first non-natural disulfide bond and a first pair of oppositely charged residues that hinder mispairing of CH1b and CLa and/or mispairing of CH1a and CLb.

58. The polypeptide complex according to claim 57, wherein the first polypeptide comprises the amino acid sequence as shown in SEQ ID NO: 39, the second polypeptide comprises the amino acid sequence as shown in SEQ ID NO: 40, the third polypeptide comprises the amino acid sequence as shown in SEQ ID NO: 38, and the fourth polypeptide comprises the amino acid sequence as shown in SEQ ID NO: 37.

59. The polypeptide complex according to any one of claims 43 to 58, wherein the third polypeptide further comprises a first Fc polypeptide at the C terminus, and the first polypeptide further comprises a second Fc polypeptide at the C terminus.

60. The polypeptide complex according to any one of claims 43 to 59, further comprising a third antigen binding domain, optionally wherein the third antigen binding domain is a Fab domain.

61. The polypeptide complex according to claim 60, wherein a C terminus of the third antigen binding domain is linked to a N terminus of the second antigen binding domain.

62. The polypeptide complex according to claim 60 or 61, wherein the third antigen binding domain is identical to the first antigen binding domain and comprises:
(i) a first fragment, comprising, in a N-terminal-to-C-terminal direction, a first heavy chain variable domain VH1 binding to a first antigen and a first CH1 domain CH1a , and
(ii) a second fragment, comprising, in a N-terminal-to-C-terminal direction, a first light chain variable domain VL1 binding to the first antigen and a first CL domain CLa; and
wherein the C terminus of the first fragment is linked to the N terminus of the fourth polypeptide.

63. The polypeptide complex according to claim 62, comprising a first polypeptide, a second polypeptide, a third polypeptide, a fourth polypeptide and a fifth polypeptide, wherein in a N-terminal-to-C-terminal direction,
(i) the first polypeptide comprises VH1-CH1a;
(ii) the third polypeptide comprises VH2-CH1b;
(iii) the fourth polypeptide comprises VH1-CH1a-linker-VL2-CLb; and
(iv) the second polypeptide and the fifth polypeptide are identical, and both comprise VL1-CLa.

64. The polypeptide complex according to claim 63, wherein the pairing of CH1b and CLb has at least one non-natural disulfide bond, which hinders mispairing between CH1b and CLa and/or mispairing between CH1a and CLb.

65. The polypeptide complex according to claim 64, wherein the pairing of CH1b and CLb has one or more introduced amino acid mutations and forms at least one introduced charged amino acid residue that hinders mispairing between CH1b and CLa and/or mispairing between CH1a and CLb.

66. The polypeptide complex according to claim 65, wherein the first polypeptide comprises the amino acid sequence as shown in SEQ ID NO: 43, the second polypeptide comprises the amino acid sequence as shown in SEQ ID NO: 44, the third polypeptide comprises the amino acid sequence as shown in SEQ ID NO: 42, and the fourth polypeptide comprises the amino acid sequence as shown in SEQ ID NO: 41.

67. The polypeptide complex according to claim 65, wherein the first polypeptide comprises the amino acid sequence as shown in SEQ ID NO: 47, the second polypeptide comprises the amino acid sequence as shown in SEQ ID NO: 48, the third polypeptide comprises the amino acid sequence as shown in SEQ ID NO: 46, and the fourth polypeptide comprises the amino acid sequence as shown in SEQ ID NO: 45.

68. The polypeptide complex according to any one of claims 43 to 67, wherein the third polypeptide further comprises, in a N-terminal-to-C-terminal direction, a first Fc polypeptide, and the first polypeptide comprises, in a N-terminal-to-C-terminal direction, a second Fc polypeptide.

69. The polypeptide complex according to claim 28, 42, 59 or 68, wherein the first Fc polypeptide and/or the second Fc polypeptide is/are derived from IgG1, IgG2, IgG3 or IgG4.

70. The polypeptide complex according to claim 69, wherein the first Fc polypeptide and the second Fc polypeptide have different amino acid sequences and are at least configured to favor the heterodimerization of the first Fc polypeptide and the second Fc polypeptide.

71. The polypeptide complex according to claim 70, wherein one of the first Fc polypeptide and the second Fc polypeptide comprises a first Fc mutation, and the other one comprises a second Fc mutation, wherein the first Fc mutation and the second Fc mutation comprise the following combinations:
a) a combination of T366W or S354C with Y349C, T366S, L368A or Y407V,
b) a combination of D399K or E356K with K392D or K409D;
c) a combination of E356K, E357K or D399K with K370E, K409D or K439E;
d) a combination of S364H or F405A with Y349T or T394F;
e) a combination of S364H or T394F with Y394T or F405A;
f) a combination of K370D or K409D with E357K or D399K; or
g) a combination of L351D or L368E with L351K or T366K,
wherein the amino acid position is numbered according to EU numbering.

72. The polypeptide complex according to claim 71, wherein the first Fc polypeptide comprises the amino acid sequence as shown in SEQ ID NO: 65 or SEQ ID NO: 67, and the second Fc polypeptide comprises the amino acid sequence as shown in SEQ ID NO: 66.

73. A nucleic acid, comprising a nucleotide sequence encoding the polypeptide complex according to any one of claims 1 to 72.

74. A vector, comprising the nucleic acid according to claim 73.

75. A host cell, comprising the nucleic acid according to claim 73 or the vector according to claim 74.

76. A pharmaceutical composition, comprising the polypeptide complex according to any one of claims 1 to 72 or the nucleic acid according to claim 73, and a pharmaceutically acceptable carrier.

77. A conjugate, comprising the polypeptide complex according to any one of claims 1 to 72 and a payload conjugated to the polypeptide complex, wherein the payload is selected from the group consisting of a radioactive label, a fluorescent label, an enzyme substrate label, an affinity purification tag, a tracer molecule, an anticancer drug and a cytotoxic molecule.

78. A composition, comprising the polypeptide complex according to any one of claims 1 to 72 or the conjugate according to claim 77, and a pharmaceutically acceptable carrier.

79. A method for treating or preventing a disease, a condition, or a disorder, comprising administering a therapeutically effective amount of the polypeptide complex according to any one of claim 1 to 72, the pharmaceutical composition according to claim 76, the conjugate according to claim 77 or the composition according to claim 78 to a subject in need thereof.

80. The method according to claim 79, wherein the disease, the condition or the disorder is selected from the group consisting of cancers, autoimmune diseases, and inflammations.
